# EUROPEAN PATENT SPECIFICATION

(11) **EP 2 838 556 B1**
(45) Date of publication and mention of the grant of the patent: **01.02.2017**
(21) Application number: 13716305.1
(22) Date of filing: 16.04.2013
(51) Int. Cl.: A61K 39/35, A61K 39/36, C07K 14/415

(54) **PLANT PROFILIN POLYPEPTIDES FOR USE IN NON-SPECIFIC ALLERGY IMMUNOTHERAPY**
PFLANZENPROFILINPOLYPEPTIDE ZUR VERWENDUNG IN NICHTSPEZIFISCHEN ALLERGIEIMMUNTHERAPIEN
POLYPEPTIDES PROFILINES VÉGÉTALES POUR UTILISATION DANS L'IMMUNOTHÉRAPIE D'ALLERGIQUE NON SPÉCIFIQUE

(30) Priority: 16.04.2012 EP 12164272; 16.04.2012 US 201261624998 P
(43) Date of publication of application: 25.02.2015
(73) Proprietor: ALK-Abelló A/S, 2970 Hørsholm (DK)
(72) Inventor: LUND, Kaare, DK-2830 Virum (DK); BRIMNES, Jens, DK-2730 Herlev (DK); HENMAR, Helene, DK-2980 Kokkedal (DK); IPSEN, Hans-Henrik, DK-3400 Hillerød (DK); MÆRKEDAHL, Lise Lund, DK-3480 Fredensborg (DK); HANSEN, Gitte Nordskov, DK-3480 Fredensborg (DK); MONSALVE CLEMENTE, Rafael Ignacio, E-28220 Madrid (ES)
(74) Representative: Inspicos P/S
(86) International application number: PCT/EP2013/057886
(87) International publication number: WO 2013/156467

(56) References cited:
- WO-A1-02/070005
- WO-A1-2004/082710
- WO-A1-2012/049310
- WO-A1-2012/049312
- WO-A2-2006/058359
- SHAO-HSUAN KAO ET AL: "Sub-proteome analysis of novel IgE-binding proteins from Bermuda grass pollen", PROTEOMICS, WILEY - VCH VERLAG, WEINHEIM, DE, vol. 5, no. 14, 1 September 2005 (2005-09-01), pages 3805-3813, XP002678364, ISSN: 1615-9853, DOI: 10.1002/PMIC.200401229 [retrieved on 2005-08-24]
- ERLER ANJA ET AL: "Proteomic profiling of birch (Betula verrucosa) pollen extracts from different origins", PROTEOMICS, vol. 11, no. 8, April 2011 (2011-04), pages 1486-1498, XP002698678,
- OWAIN R MILLINGTON ET AL: "Induction of Bystander Suppression by Feeding Antigen Occurs despite Normal Clonal Expansion of the Bystander T Cell Population", THE JOURNAL OF IMMUNOLOGY, THE AMERICAN ASSOCIATION OF IMMUNOLOGISTS, US, vol. 173, no. 10, 15 November 2004 (2004-11-15), pages 6059-6064, XP002618893, ISSN: 0022-1767
- TORDESILLAS LETICIA ET AL: "A mutant of the major melon allergen, Cuc m 2, with reduced IgE binding capacity is a good candidate for specific immunotherapy.", MOLECULAR IMMUNOLOGY DEC 2011, vol. 49, no. 3, December 2011 (2011-12), pages 504-511, XP002691223, ISSN: 1872-9142
- KILDSGAARD J ET AL: "Sublingual immunotherapy in sensitized mice", ANNALS OF ALLERGY, ASTHMA & IMMUNOLOGY, ARLINGTON HEIGHTS, IL, US, vol. 98, no. 4, 1 April 2007 (2007-04-01), pages 366-372, XP026959766, ISSN: 1081-1206 [retrieved on 2007-04-01]
- SANTOS ALEXANDRA ET AL: "Profilins: Mimickers of Allergy or Relevant Allergens?", INTERNATIONAL ARCHIVES OF ALLERGY AND IMMUNOLOGY, vol. 155, no. 3, 2011, pages 191-204, XP009166664,
- SABATOS-PEYTON C A ET AL: "Antigen-specific immunotherapy of autoimmune and allergic diseases", CURRENT OPINION IN IMMUNOLOGY, ELSEVIER, OXFORD, GB, vol. 22, no. 5, 1 October 2010 (2010-10-01), pages 609-615, XP027450102, ISSN: 0952-7915, DOI: 10.1016/J.COI.2010.08.006 [retrieved on 2010-10-01]
- Song-Jung Park ET AL: "Successful Treatment of Two Dogs with Allergic Dermatitis by Anti-Allergic Peptides (MS-antigen TM )", J. Vet. Med. Sci, 1 January 2002 (2002-01-01), pages 63-65, XP055186648, Retrieved from the Internet: URL:https://www.jstage.jst.go.jp/article/j vms/64/1/64_1_63/_pdf [retrieved on 2015-04-29]

### SEQUENCE LISTING

<110> ALK-Abelló A/S
<120> PLANT PROFILIN POLYPEPTIDES FOR USE IN NON-SPECIFIC ALLERGY IMMUNOTHERAPY
<130> 19190PCT00
<160> 47
<170> PatentIn version 3.5
<210> 1
   <211> 131
   <212> PRT
   <213> Phleum pratense
<400> 1
   Met Ser Trp Gln Thr Tyr Val Asp Glu His Leu Met Cys Glu Ile Glu
   1               5                   10                  15
   Gly His His Leu Ala Ser Ala Ala Ile Leu Gly His Asp Gly Thr Val
               20                  25                  30
   Trp Ala Gln Ser Ala Asp Phe Pro Gln Phe Lys Pro Glu Glu Ile Thr
           35                  40                  45
   Gly Ile Met Lys Asp Phe Asp Glu Pro Gly His Leu Ala Pro Thr Gly
       50                  55                  60
   Met Phe Val Ala Gly Ala Lys Tyr Met Val Ile Gln Gly Glu Pro Gly
   65                  70                  75                  80
   Ala Val Ile Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Cys Asn Met Val Val Glu Arg Leu Gly Asp Tyr Leu Val Glu
           115                 120                 125
   Gln Gly Met
       130
<210> 2
   <211> 131
   <212> PRT
   <213> Phleum pratense
<400> 2
   Met Ser Trp Gln Thr Tyr Val Asp Glu His Leu Met Cys Glu Ile Glu
   1               5                   10                  15
   Gly His His Leu Ala Ser Ala Ala Ile Phe Gly His Asp Gly Thr Val
               20                  25                  30
   Trp Ala Gln Ser Ala Asp Phe Pro Gln Phe Lys Pro Glu Glu Ile Thr
           35                  40                  45
   Gly Ile Met Lys Asp Leu Asp Glu Pro Gly His Leu Ala Pro Thr Gly
       50                  55                  60
   Met Phe Val Ala Ala Ala Lys Tyr Met Val Ile Gln Gly Glu Pro Gly
   65                  70                  75                  80
   Ala Val Ile Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Cys Asn Met Val Val Glu Arg Leu Gly Asp Tyr Leu Val Glu
           115                 120                 125
   Gln Gly Met
       130
<210> 3
   <211> 131
   <212> PRT
   <213> Phleum pratense
<400> 3
   Met Ser Trp Gln Thr Tyr Val Asp Glu His Leu Met Cys Glu Ile Glu
   1               5                   10                  15
   Gly His His Leu Ala Ser Ala Ala Ile Leu Gly His Asp Gly Thr Val
               20                  25                  30
   Trp Ala Gln Ser Ala Asp Phe Pro Gln Phe Lys Pro Glu Glu Ile Thr
           35                  40                  45
   Gly Ile Met Lys Asp Phe Asp Glu Pro Gly His Leu Ala Pro Thr Gly
       50                  55                  60
   Met Phe Val Ala Gly Ala Lys Tyr Met Val Ile Gln Gly Glu Pro Gly
   65                  70                  75                  80
   Arg Val Ile Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Cys Asn Met Val Val Glu Arg Leu Gly Asp Tyr Leu Val Glu
           115                 120                 125
   Gln Gly Met
       130
<210> 4
   <211> 131
   <212> PRT
   <213> Phleum pratense
<400> 4
   Met Ser Trp Gln Ala Tyr Val Asp Glu His Leu Met Cys Glu Ile Glu
   1               5                   10                  15
   Gly His His Leu Ala Ser Ala Ala Ile Leu Gly His Asp Gly Thr Val
               20                  25                  30
   Trp Ala Gln Ser Ala Asp Phe Pro Gln Phe Lys Pro Glu Glu Ile Thr
           35                  40                  45
   Gly Ile Met Lys Asp Phe Asp Glu Pro Gly His Leu Ala Pro Thr Gly
       50                  55                  60
   Met Phe Val Ala Thr Ala Lys Tyr Met Val Ile Gln Gly Glu Pro Gly
   65                  70                  75                  80
   Ala Val Ile Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Cys Asn Met Val Val Glu Arg Leu Gly Asp Tyr Leu Leu Lys
           115                 120                 125
   Gln Gly Leu
       130
<210> 5
   <211> 131
   <212> PRT
   <213> Phleum pratense
<400> 5
   Met Ser Trp Gln Ala Tyr Val Asp Glu His Leu Met Cys Glu Ile Glu
   1               5                   10                  15
   Gly His His Leu Ala Ser Ala Ala Ile Leu Gly His Asp Gly Thr Val
               20                  25                  30
   Trp Ala Gln Ser Ala Asp Phe Pro Gln Phe Lys Pro Glu Glu Ile Thr
           35                  40                  45
   Gly Ile Met Lys Asp Phe Asp Glu Pro Gly His Leu Ala Pro Thr Gly
       50                  55                  60
   Met Phe Val Ala Ala Ala Lys Tyr Met Val Ile Gln Gly Glu Pro Gly
   65                  70                  75                  80
   Ala Val Ile Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Cys Asn Met Val Val Glu Arg Leu Gly Asp Tyr Leu Val Lys
           115                 120                 125
   Gln Gly Leu
       130
<210> 6
   <211> 131
   <212> PRT
   <213> Phleum pratense
<400> 6
   Met Ser Trp Gln Thr Tyr Val Asp Glu His Leu Met Cys Glu Ile Glu
   1               5                   10                  15
   Gly His His Leu Ala Ser Ala Ala Ile Leu Gly His Asp Gly Thr Val
               20                  25                  30
   Trp Ala Gln Ser Ala Asp Phe Pro Gln Phe Lys Pro Glu Glu Ile Thr
           35                  40                  45
   Gly Ile Met Lys Asp Phe Asp Glu Pro Gly His Leu Ala Pro Thr Gly
       50                  55                  60
   Met Phe Val Ala Ala Ala Lys Tyr Met Val Ile Gln Gly Glu Pro Gly
   65                  70                  75                  80
   Ala Val Ile Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Cys Asn Met Val Val Glu Arg Leu Gly Asp Tyr Leu Leu Lys
           115                 120                 125
   Gln Gly Leu
       130
<210> 7
   <211> 131
   <212> PRT
   <213> Phleum pratense
<400> 7
   Met Ser Trp Gln Thr Tyr Val Asp Glu His Leu Met Cys Glu Ile Glu
   1               5                   10                  15
   Gly His His Leu Ala Ser Ala Ala Ile Leu Gly His Asp Gly Thr Val
               20                  25                  30
   Trp Ala Gln Ser Ala Asp Phe Pro Gln Phe Lys Pro Glu Glu Ile Thr
           35                  40                  45
   Gly Ile Met Lys Asp Phe Asp Glu Pro Gly His Leu Ala Pro Thr Gly
       50                  55                  60
   Met Phe Val Ala Thr Ala Lys Tyr Met Val Ile Gln Gly Glu Pro Gly
   65                  70                  75                  80
   Ala Val Ile Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Cys Asn Met Val Val Glu Arg Leu Gly Asp Tyr Leu Leu Lys
           115                 120                 125
   Gln Gly Leu
       130
<210> 8
   <211> 131
   <212> PRT
   <213> Phleum pratense
<400> 8
   Met Ser Trp Gln Ala Tyr Val Asp Glu His Leu Met Cys Glu Ile Glu
   1 5 10 15
   Gly His His Leu Ala Ser Ala Ala Ile Leu Gly His Asp Gly Thr Val
   20 25 30
   Trp Ala Gln Ser Ala Asp Phe Pro Gln Phe Lys Pro Glu Glu Ile Thr
   35 40 45
   Gly Ile Met Lys Asp Phe Asp Glu Pro Gly His Leu Ala Pro Thr Gly
   50 55 60
   Met Phe Val Ala Ala Ala Lys Tyr Met Val Ile Gln Gly Glu Pro Gly
   65 70 75 80
   Ala Val Ile Arg Gly Lys Lys Gly Ala Gly Gly Ile Thr Ile Lys Lys
   85 90 95
   Thr Gly Gln Ala Leu Val Val Gly Ile Tyr Asp Glu Pro Met Thr Pro
   100 105 110
   Gly Gln Cys Asn Met Val Val Glu Arg Leu Gly Asp Tyr Leu Leu Lys
   115 120 125
   Gln Gly Leu
   130
<210> 9
   <211> 131
   <212> PRT
   <213> Phleum pratense
<400> 9
   Met Ser Trp Gln Ala Tyr Val Asp Glu His Leu Met Cys Glu Ile Glu
   1 5 10 15
   Gly His His Leu Ala Ser Ala Ala Ile Leu Gly His Asp Gly Thr Val
   20 25 30
   Trp Ala Gln Ser Ala Asp Phe Pro Gln Phe Lys Pro Glu Glu Ile Thr
   35 40 45
   Gly Ile Met Lys Asp Phe Asp Glu Pro Gly His Leu Ala Pro Thr Gly
   50 55 60
   Met Phe Val Ala Ala Ala Lys Tyr Met Val Ile Gln Gly Glu Pro Gly
   65                  70                  75                  80
   Ala Val Thr Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Cys Asn Met Val Val Glu Arg Leu Gly Asp Tyr Leu Val Lys
           115                 120                 125
   Gln Gly Leu
       130
<210> 10
   <211> 131
   <212> PRT
   <213> Phleum pratense
<400> 10
   Met Ser Trp Gln Ala Tyr Val Asp Glu His Leu Met Cys Glu Ile Glu
   1               5                   10                  15
   Gly His His Leu Ala Ser Ala Ala Ile Leu Gly His Asp Gly Thr Val
               20                  25                  30
   Trp Ala Gln Ser Ala Asp Phe Pro Gln Phe Lys Pro Glu Glu Ile Thr
           35                  40                  45
   Gly Ile Met Lys Asp Phe Asp Glu Pro Gly His Leu Ala Pro Thr Gly
       50                  55                  60
   Met Phe Val Ala Thr Ala Lys Tyr Met Val Ile Gln Gly Glu Pro Gly
   65                  70                  75                  80
   Ala Val Ile Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Cys Ser Met Val Val Glu Arg Leu Gly Asp Tyr Leu Val Lys
           115                 120                 125
   Gln Gly Leu
       130
<210> 11
   <211> 138
   <212> PRT
   <213> Triticum aestivum
<400> 11
   Met Ser Trp Gln Thr Tyr Val Asp Asp His Leu Cys Cys Glu Ile Asp
   1               5                   10                  15
   Gly Gln His Leu Thr Ser Ala Ala Ile Leu Gly His Asp Gly Ser Val
               20                  25                  30
   Trp Thr Glu Ser Pro Asn Phe Pro Lys Phe Lys Pro Glu Glu Ile Ala
           35                  40                  45
   Gly Ile Val Lys Asp Phe Glu Glu Pro Gly His Leu Ala Pro Thr Gly
       50                  55                  60
   Leu Phe Leu Gly Gly Thr Lys Tyr Met Val Ile Gln Gly Glu Pro Gly
   65                  70                  75                  80
   Val Val Ile Arg Gly Lys Lys Gly Thr Gly Gly Ile Thr Ile Lys Lys
                   85                  90                  95
   Thr Gly Met Ala Leu Ile Leu Gly Ile Tyr Asp Glu Pro Met Thr Pro
               100                 105                 110
   Gly Gln Cys Asn Leu Val Val Glu Arg Leu Gly Asp Tyr Leu Ile Asp
           115                 120                 125
   Gln Gly Tyr Trp Val Pro Ser Ser Asn Ser
       130                 135
<210> 12
   <211> 131
   <212> PRT
   <213> Hordeum vulgare
<400> 12
   Met Ser Trp Gln Thr Tyr Val Asp Asp His Leu Cys Cys Glu Ile Asp
   1               5                   10                  15
   Gly Gln His Leu Thr Ser Ala Ala Ile Leu Gly His Asp Gly Arg Val
               20                  25                  30
   Trp Val Gln Ser Pro Asn Phe Pro Gln Phe Lys Pro Glu Glu Ile Ala
           35                  40                  45
   Gly Ile Ile Lys Asp Phe Asp Glu Pro Gly His Leu Ala Pro Thr Gly
       50                  55                  60
   Leu Phe Leu Gly Gly Thr Lys Tyr Met Val Ile Gln Gly Glu Pro Gly
   65                  70                  75                  80
   Val Val Ile Arg Gly Lys Lys Gly Thr Gly Gly Ile Thr Ile Lys Lys
                   85                  90                  95
   Thr Gly Met Pro Leu Ile Leu Gly Ile Tyr Asp Glu Pro Met Thr Pro
               100                 105                 110
   Gly Gln Cys Asn Leu Val Val Glu Arg Leu Gly Asp Tyr Leu Val Glu
           115                 120                 125
   Gln Gly Phe
       130
<210> 13
   <211> 131
   <212> PRT
   <213> Oryza sativa subsp. japonica
<400> 13
   Met Ser Trp Gln Thr Tyr Val Asp Glu His Leu Met Cys Glu Ile Glu
   1               5                   10                  15
   Gly His His Leu Thr Ser Ala Ala Ile Val Gly His Asp Gly Thr Val
               20                  25                  30
   Trp Ala Gln Ser Ala Ala Phe Pro Gln Phe Lys Pro Glu Glu Met Thr
           35                  40                  45
   Asn Ile Met Lys Asp Phe Asp Glu Pro Gly Phe Leu Ala Pro Thr Gly
       50                  55                  60
   Leu Phe Leu Gly Pro Thr Lys Tyr Met Val Ile Gln Gly Glu Pro Gly
   65                  70                  75                  80
   Ala Val Ile Arg Gly Lys Lys Gly Ser Gly Gly Ile Thr Val Lys Lys
                   85                  90                  95
   Thr Gly Gln Ala Leu Val Val Gly Ile Tyr Asp Glu Pro Met Thr Pro
               100                 105                 110
   Gly Gln Cys Asn Met Val Val Glu Arg Leu Gly Asp Tyr Leu Val Glu
           115                 120                 125
   Gln Gly Leu
       130
<210> 14
   <211> 131
   <212> PRT
   <213> Zea mays
<400> 14
   Met Ser Trp Gln Thr Tyr Val Asp Glu His Leu Met Cys Glu Ile Glu
   1               5                   10                  15
   Gly His His Leu Thr Ser Ala Ala Ile Val Gly His Asp Gly Ala Thr
               20                  25                  30
   Trp Ala Gln Ser Thr Ala Phe Pro Glu Phe Lys Pro Glu Glu Met Ala
           35                  40                  45
   Ala Ile Met Lys Asp Phe Asp Glu Pro Gly His Leu Ala Pro Thr Gly
       50                  55                  60
   Leu Ile Leu Gly Gly Thr Lys Tyr Met Val Ile Gln Gly Glu Pro Gly
   65                  70                  75                  80
   Ala Val Ile Arg Gly Lys Lys Gly Ser Gly Gly Ile Thr Val Lys Lys
                   85                  90                  95
   Thr Gly Gln Ser Leu Ile Ile Gly Ile Tyr Asp Glu Pro Met Thr Pro
               100                 105                 110
   Gly Gln Cys Asn Leu Val Val Glu Arg Leu Gly Asp Tyr Leu Leu Glu
           115                 120                 125
   Gln Gly Met
       130
<210> 15
   <211> 131
   <212> PRT
   <213> Cynodon dactylon
<400> 15
   Met Ser Trp Gln Ala Tyr Val Asp Asp His Leu Met Cys Glu Ile Glu
   1               5                   10                  15
   Gly His His Leu Thr Ser Ala Ala Ile Ile Gly His Asp Gly Thr Val
               20                  25                  30
   Trp Ala Gln Ser Ala Ala Phe Pro Ala Phe Lys Pro Glu Glu Met Ala
   35 40 45
   Asn Ile Met Lys Asp Phe Asp Glu Pro Gly Phe Leu Ala Pro Thr Gly
   50 55 60
   Leu Phe Leu Gly Pro Thr Lys Tyr Met Val Ile Gln Gly Glu Pro Gly
   65 70 75 80
   Ala Val Ile Arg Gly Lys Lys Gly Ser Gly Gly Val Thr Val Lys Lys
   85 90 95
   Thr Gly Gln Ala Leu Val Ile Gly Ile Tyr Asp Glu Pro Met Thr Pro
   100 105 110
   Gly Gln Cys Asn Met Val Ile Glu Lys Leu Gly Asp Tyr Leu Ile Glu
   115 120 125
   Gln Gly Met
   130
<210> 16
   <211> 134
   <212> PRT
   <213> Olea europaea
<400> 16
   Met Ser Trp Gln Ala Tyr Val Asp Asp His Leu Met Cys Asp Ile Glu
   1 5 10 15
   Gly His Glu Asp His Arg Leu Thr Ala Ala Ala Ile Val Gly His Asp
   20 25 30
   Gly Ser Val Trp Ala Gln Ser Ala Thr Phe Pro Gln Phe Lys Pro Glu
   35 40 45
   Glu Met Asn Gly Ile Met Thr Asp Phe Asn Glu Pro Gly His Leu Ala
   50 55 60
   Pro Thr Gly Leu His Leu Gly Gly Thr Lys Tyr Met Val Ile Gln Gly
   65 70 75 80
   Glu Ala Gly Ala Val Ile Arg Gly Lys Lys Gly Ser Gly Gly Ile Thr
   85 90 95
   Ile Lys Lys Thr Gly Gln Ala Leu Val Phe Gly Ile Tyr Glu Glu Pro
   100 105 110
   Val Thr Pro Gly Gln Cys Asn Met Val Val Glu Arg Leu Gly Asp Tyr
           115                 120                 125
   Leu Val Glu Gln Gly Met
       130
<210> 17
   <211> 131
   <212> PRT
   <213> Corylus avellana
<400> 17
   Met Ser Trp Gln Thr Tyr Gly Asp Glu His Leu Met Cys Glu Ile Glu
   1               5                   10                  15
   Gly Asn Arg Leu Ala Ala Ala Ala Ile Ile Gly His Asp Gly Ser Val
               20                  25                  30
   Trp Ala Gln Ser Ser Thr Phe Pro Gln Leu Lys Pro Glu Glu Ile Thr
           35                  40                  45
   Gly Val Met Asn Asp Phe Asn Glu Pro Gly Ser Leu Ala Pro Thr Gly
       50                  55                  60
   Leu Tyr Leu Gly Gly Thr Lys Tyr Met Val Ile Gln Gly Glu Pro Gly
   65                  70                  75                  80
   Ala Val Ile Arg Arg Lys Lys Gly Pro Gly Gly Val Thr Val Lys Lys
                   85                  90                  95
   Thr Ser Gln Ala Leu Ile Ile Gly Ile Tyr Asp Glu Pro Met Thr Pro
               100                 105                 110
   Gly Gln Cys Asn Met Ile Val Glu Arg Leu Gly Asp Tyr Leu Ile Asp
           115                 120                 125
   Gln Gly Leu
       130
<210> 18
   <211> 133
   <212> PRT
   <213> Betula pendula
<400> 18
   Met Ser Trp Gln Thr Tyr Val Asp Glu His Leu Met Cys Asp Ile Asp
   1               5                   10                  15
   Gly Gln Ala Ser Asn Ser Leu Ala Ser Ala Ile Val Gly His Asp Gly
               20                  25                  30
   Ser Val Trp Ala Gln Ser Ser Ser Phe Pro Gln Phe Lys Pro Gln Glu
           35                  40                  45
   Ile Thr Gly Ile Met Lys Asp Phe Glu Glu Pro Gly His Leu Ala Pro
       50                  55                  60
   Thr Gly Leu His Leu Gly Gly Ile Lys Tyr Met Val Ile Gln Gly Glu
   65                  70                  75                  80
   Ala Gly Ala Val Ile Arg Gly Lys Lys Gly Ser Gly Gly Ile Thr Ile
                   85                  90                  95
   Lys Lys Thr Gly Gln Ala Leu Val Phe Gly Ile Tyr Glu Glu Pro Val
               100                 105                 110
   Thr Pro Gly Gln Cys Asn Met Val Val Glu Arg Leu Gly Asp Tyr Leu
           115                 120                 125
   Ile Asp Gln Gly Leu
       130
<210> 19
   <211> 131
   <212> PRT
   <213> Arachis hypogaea
<400> 19
   Met Ser Trp Gln Thr Tyr Val Asp Asn His Leu Leu Cys Glu Ile Glu
   1               5                   10                  15
   Gly Asp His Leu Ser Ser Ala Ala Ile Leu Gly Gln Asp Gly Gly Val
               20                  25                  30
   Trp Ala Gln Ser Ser His Phe Pro Gln Phe Lys Pro Glu Glu Ile Thr
           35                  40                  45
   Ala Ile Met Asn Asp Phe Ala Glu Pro Gly Ser Leu Ala Pro Thr Gly
       50                  55                  60
   Leu Tyr Leu Gly Gly Thr Lys Tyr Met Val Ile Gln Gly Glu Pro Gly
   65                  70                  75                  80
   Ala Ile Ile Pro Gly Lys Lys Gly Pro Gly Gly Val Thr Ile Glu Lys
                   85                  90                  95
   Thr Asn Gln Ala Leu Ile Ile Gly Ile Tyr Asp Lys Pro Met Thr Pro
               100                 105                 110
   Gly Gln Cys Asn Met Ile Val Glu Arg Leu Gly Asp Tyr Leu Ile Asp
           115                 120                 125
   Thr Gly Leu
       130
<210> 20
   <211> 131
   <212> PRT
   <213> Glycine max
<400> 20
   Met Ser Trp Gln Ala Tyr Val Asp Asp His Leu Leu Cys Asp Ile Glu
   1               5                   10                  15
   Gly Asn His Leu Thr His Ala Ala Ile Ile Gly Gln Asp Gly Ser Val
               20                  25                  30
   Trp Ala Gln Ser Thr Asp Phe Pro Gln Phe Lys Pro Glu Glu Ile Thr
           35                  40                  45
   Ala Ile Met Asn Asp Phe Asn Glu Pro Gly Ser Leu Ala Pro Thr Gly
       50                  55                  60
   Leu Tyr Leu Gly Gly Thr Lys Tyr Met Val Ile Gln Gly Glu Pro Gly
   65                  70                  75                  80
   Ala Val Ile Arg Gly Lys Lys Gly Pro Gly Gly Val Thr Val Lys Lys
                   85                  90                  95
   Thr Gly Ala Ala Leu Ile Ile Gly Ile Tyr Asp Glu Pro Met Thr Pro
               100                 105                 110
   Gly Gln Cys Asn Met Val Val Glu Arg Pro Gly Asp Tyr Leu Ile Asp
           115                 120                 125
   Gln Gly Tyr
       130
<210> 21
   <211> 133
   <212> PRT
   <213> Ambrosia artemisiifolia
<400> 21
   Met Ser Trp Gln Thr Tyr Val Asp Glu His Leu Met Cys Asp Ile Glu
   1               5                   10                  15
   Gly Thr Gly Gln His Leu Ala Ser Ala Ala Ile Phe Gly Thr Asp Gly
               20                  25                  30
   Asn Val Trp Ala Lys Ser Ser Ser Phe Pro Glu Phe Lys Pro Asp Glu
           35                  40                  45
   Ile Asn Ala Ile Ile Lys Glu Phe Ser Glu Pro Gly Ala Leu Ala Pro
       50                  55                  60
   Thr Gly Leu Phe Leu Ala Gly Ala Lys Tyr Met Val Ile Gln Gly Glu
   65                  70                  75                  80
   Pro Gly Ala Val Ile Arg Gly Lys Lys Gly Ala Gly Gly Ile Cys Ile
                   85                  90                  95
   Lys Lys Thr Gly Gln Ala Met Val Phe Gly Ile Tyr Glu Glu Pro Val
               100                 105                 110
   Asn Pro Gly Gln Cys Asn Met Val Val Glu Arg Leu Gly Asp Tyr Leu
           115                 120                 125
   Val Asp Gln Gly Met
       130
<210> 22
   <211> 133
   <212> PRT
   <213> Helianthus annuus
<400> 22
   Met Ser Trp Gln Ala Tyr Val Asp Glu His Leu Met Cys Asp Ile Glu
   1               5                   10                  15
   Gly Thr Gly Gln His Leu Thr Ser Ala Ala Ile Leu Gly Leu Asp Gly
               20                  25                  30
   Thr Val Trp Ala Gln Ser Ala Lys Phe Pro Gln Phe Lys Pro Glu Glu
           35                  40                  45
   Met Lys Gly Ile Ile Lys Glu Phe Asp Glu Ala Gly Thr Leu Ala Pro
       50                  55                  60
   Thr Gly Met Phe Ile Ala Gly Ala Lys Tyr Met Val Leu Gln Gly Glu
   65                  70                  75                  80
   Pro Gly Ala Val Ile Arg Gly Lys Lys Gly Ala Gly Gly Ile Cys Ile
                   85                  90                  95
   Lys Lys Thr Gly Gln Ala Met Ile Met Gly Ile Tyr Asp Glu Pro Val
   100 105 110
   Ala Pro Gly Gln Cys Asn Met Val Val Glu Arg Leu Gly Asp Tyr Leu
   115 120 125
   Leu Glu Gln Gly Met
   130
<210> 23
   <211> 133
   <212> PRT
   <213> Artemisia vulgaris
<400> 23
   Met Ser Trp Gln Thr Tyr Val Asp Asp His Leu Met Cys Asp Ile Glu
   1 5 10 15
   Gly Thr Gly Gln His Leu Thr Ser Ala Ala Ile Phe Gly Thr Asp Gly
   20 25 30
   Thr Val Trp Ala Lys Ser Ala Ser Phe Pro Glu Phe Lys Pro Asn Glu
   35 40 45
   Ile Asp Ala Ile Ile Lys Glu Phe Asn Glu Ala Gly Gln Leu Ala Pro
   50 55 60
   Thr Gly Leu Phe Leu Gly Gly Ala Lys Tyr Met Val Ile Gln Gly Glu
   65 70 75 80
   Ala Gly Ala Val Ile Arg Gly Lys Lys Gly Ala Gly Gly Ile Cys Ile
   85 90 95
   Lys Lys Thr Gly Gln Ala Met Val Phe Gly Ile Tyr Asp Glu Pro Val
   100 105 110
   Ala Pro Gly Gln Cys Asn Met Val Val Glu Arg Leu Gly Asp Tyr Leu
   115 120 125
   Leu Asp Gln Gly Met
   130
<210> 24
   <211> 131
   <212> PRT
   <213> Hevea brasiliensis
<400> 24
   Met Ser Trp Gln Thr Tyr Val Asp Glu Arg Leu Met Cys Glu Ile Glu
   1               5                   10                  15
   Gly Asn His Leu Thr Ala Ala Ala Ile Ile Gly Gln Asp Gly Ser Val
               20                  25                  30
   Trp Ala Gln Ser Ser Asn Phe Pro Gln Phe Lys Ser Glu Glu Ile Thr
           35                  40                  45
   Ala Ile Met Ser Asp Phe Asp Glu Pro Gly Thr Leu Ala Pro Thr Gly
       50                  55                  60
   Leu His Leu Gly Gly Thr Lys Tyr Met Val Ile Gln Gly Glu Ala Gly
   65                  70                  75                  80
   Ala Val Ile Arg Gly Lys Lys Gly Pro Gly Gly Val Thr Val Arg Lys
                   85                  90                  95
   Thr Asn Gln Ala Leu Ile Ile Gly Ile Tyr Asp Glu Pro Met Thr Pro
               100                 105                 110
   Gly Gln Cys Asn Met Ile Val Glu Arg Leu Gly Asp Tyr Leu Leu Glu
           115                 120                 125
   Gln Gly Met
       130
<210> 25
   <211> 131
   <212> PRT
   <213> Fragaria ananassa
<400> 25
   Met Ser Trp Gln Thr Tyr Val Asp Asp His Leu Met Cys Glu Ile Glu
   1               5                   10                  15
   Gly Asn His Leu Ser Ala Ala Ala Ile Ile Gly Gln Asp Gly Ser Val
               20                  25                  30
   Trp Ala Gln Ser Ala Thr Phe Pro Gln Leu Lys Pro Glu Glu Val Thr
           35                  40                  45
   Gly Ile Val Arg Asp Phe Asp Glu Pro Gly Thr Leu Ala Pro Thr Gly
       50                  55                  60
   Leu Tyr Leu Gly Gly Thr Lys Tyr Met Val Ile Gln Gly Glu Pro Gly
   65                  70                  75                  80
   Ala Val Ile Arg Gly Lys Lys Gly Pro Gly Gly Val Thr Val Lys Lys
                   85                  90                  95
   Thr Thr Leu Ala Leu Leu Ile Gly Ile Tyr Asp Glu Pro Met Thr Pro
               100                 105                 110
   Gly Gln Cys Asn Met Ile Val Glu Arg Leu Gly Asp Tyr Leu Val Glu
           115                 120                 125
   Gln Gly Leu
       130
<210> 26
   <211> 131
   <212> PRT
   <213> Malus domestica
<400> 26
   Met Ser Trp Gln Ala Tyr Val Asp Asp His Leu Met Cys Asp Ile Asp
   1               5                   10                  15
   Gly Asn Arg Leu Thr Ala Ala Ala Ile Leu Gly Gln Asp Gly Ser Val
               20                  25                  30
   Trp Ser Gln Ser Ala Ser Phe Pro Ala Phe Lys Pro Glu Glu Ile Ala
           35                  40                  45
   Ala Ile Leu Lys Asp Phe Asp Gln Pro Gly Thr Leu Ala Pro Thr Gly
       50                  55                  60
   Leu Phe Leu Gly Gly Thr Lys Tyr Met Val Ile Gln Gly Glu Pro Gly
   65                  70                  75                  80
   Ala Val Ile Arg Gly Lys Lys Gly Ser Gly Gly Ile Thr Ile Lys Lys
                   85                  90                  95
   Thr Ser Gln Ala Leu Leu Ile Gly Ile Tyr Asp Glu Pro Val Thr Pro
               100                 105                 110
   Gly Gln Cys Asn Ile Val Val Glu Arg Leu Gly Asp Tyr Leu Ile Glu
           115                 120                 125
   Gln Gly Leu
       130
<210> 27
   <211> 131
   <212> PRT
   <213> Prunus avium
<400> 27
   Met Ser Trp Gln Ala Tyr Val Asp Asp His Leu Met Cys Asp Ile Asp
   1               5                   10                  15
   Gly Asn Arg Leu Thr Ala Ala Ala Ile Leu Gly Gln Asp Gly Ser Val
               20                  25                  30
   Trp Ser Gln Ser Ala Thr Phe Pro Ala Phe Lys Pro Glu Glu Ile Ala
           35                  40                  45
   Ala Ile Leu Lys Asp Leu Asp Gln Pro Gly Thr Leu Ala Pro Thr Gly
       50                  55                  60
   Leu Phe Leu Gly Gly Thr Lys Tyr Met Val Ile Gln Gly Glu Ala Gly
   65                  70                  75                  80
   Ala Val Ile Arg Gly Lys Lys Gly Ser Gly Gly Ile Thr Val Lys Lys
                   85                  90                  95
   Thr Asn Gln Ala Leu Ile Ile Gly Ile Tyr Asp Glu Pro Leu Thr Pro
               100                 105                 110
   Gly Gln Cys Asn Met Ile Val Glu Arg Leu Gly Asp Tyr Leu Ile Glu
           115                 120                 125
   Gln Gly Leu
       130
<210> 28
   <211> 131
   <212> PRT
   <213> Prunus dulcis
<400> 28
   Met Ser Trp Gln Gln Tyr Val Asp Asp His Leu Met Cys Asp Ile Asp
   1               5                   10                  15
   Gly Asn Arg Leu Thr Ala Ala Ala Ile Leu Gly Gln Asp Gly Ser Val
               20                  25                  30
   Trp Ser Gln Ser Ala Thr Phe Pro Ala Phe Lys Pro Glu Glu Ile Ala
           35                  40                  45
   Ala Ile Leu Lys Asp Phe Asp Gln Pro Gly Thr Leu Ala Pro Thr Gly
       50                  55                  60
   Leu Phe Leu Gly Gly Thr Lys Tyr Met Val Ile Gln Gly Glu Ala Gly
   65 70 75 80
   Ala Val Ile Arg Gly Lys Lys Gly Ser Gly Gly Ile Thr Val Lys Lys
   85 90 95
   Thr Asn Gln Ala Leu Ile Ile Gly Ile Tyr Asp Glu Pro Leu Thr Pro
   100 105 110
   Gly Gln Cys Asn Met Ile Val Glu Arg Leu Gly Asp Tyr Leu Ile Glu
   115 120 125
   Gln Gly Leu
   130
<210> 29
   <211> 131
   <212> PRT
   <213> Prunus persica
<400> 29
   Met Ser Trp Gln Ala Tyr Val Asp Asp His Leu Met Cys Asp Ile Asp
   1 5 10 15
   Gly Asn Arg Leu Thr Ala Ala Ala Ile Leu Gly Gln Asp Gly Ser Val
   20 25 30
   Trp Ser Gln Ser Ala Thr Phe Pro Ala Phe Lys Pro Glu Glu Ile Ala
   35 40 45
   Ala Ile Leu Lys Asp Phe Asp Gln Pro Gly Thr Leu Ala Pro Thr Gly
   50 55 60
   Leu Phe Leu Gly Gly Thr Lys Tyr Met Val Ile Gln Gly Glu Ala Gly
   65 70 75 80
   Ala Val Ile Arg Gly Lys Lys Gly Ser Gly Gly Ile Thr Val Lys Lys
   85 90 95
   Thr Asn Gln Ala Leu Ile Ile Gly Ile Tyr Asp Glu Pro Leu Thr Pro
   100 105 110
   Gly Gln Cys Asn Met Ile Val Glu Arg Leu Gly Asp Tyr Leu Ile Glu
   115 120 125
   Gln Gly Leu
   130
<210> 30
   <211> 131
   <212> PRT
   <213> Pyrus communis
<400> 30
   Met Ser Trp Gln Ala Tyr Val Asp Asp His Leu Met Cys Asp Ile Asp
   1               5                   10                  15
   Gly His His Leu Thr Ala Ala Ala Ile Leu Gly His Asp Gly Ser Val
               20                  25                  30
   Trp Ala Gln Ser Ser Thr Phe Pro Lys Phe Lys Pro Glu Glu Ile Thr
           35                  40                  45
   Ala Ile Met Lys Asp Phe Asp Glu Pro Gly Ser Leu Ala Pro Thr Gly
       50                  55                  60
   Leu His Leu Gly Gly Thr Lys Tyr Met Val Ile Gln Gly Glu Gly Gly
   65                  70                  75                  80
   Ala Val Ile Arg Gly Lys Lys Gly Ser Gly Gly Val Thr Val Lys Lys
                   85                  90                  95
   Thr Ser Gln Ala Leu Val Phe Gly Ile Tyr Glu Glu Pro Leu Thr Pro
               100                 105                 110
   Gly Gln Cys Asn Met Ile Val Glu Arg Leu Gly Asp Tyr Leu Ile Asp
           115                 120                 125
   Gln Gly Leu
       130
<210> 31
   <211> 132
   <212> PRT
   <213> Parietaria judaica
<400> 31
   Met Ser Trp Gln Ala Tyr Val Asp Asp His Leu Met Cys Asp Val Gly
   1               5                   10                  15
   Asp Gly Asn Thr Pro Ala Ser Ala Ala Ile Ile Gly His Asp Gly Ser
               20                  25                  30
   Val Trp Ala Gln Ser Ala Asn Phe Pro Gln Leu Lys Pro Glu Glu Val
           35                  40                  45
   Thr Gly Ile Met Asn Asp Phe Asn Glu Ala Gly Phe Leu Ala Pro Thr
       50                  55                  60
   Gly Leu Phe Leu Gly Gly Thr Lys Tyr Met Val Ile Gln Gly Glu Ser
   65                  70                  75                  80
   Gly Ala Val Ile Arg Gly Lys Lys Gly Ser Gly Gly Ala Thr Leu Lys
                   85                  90                  95
   Lys Thr Gly Gln Ala Ile Val Ile Gly Ile Tyr Asp Glu Pro Met Thr
               100                 105                 110
   Pro Gly Gln Cys Asn Leu Val Val Glu Arg Leu Gly Asp Tyr Leu Leu
           115                 120                 125
   Glu Gln Gly Leu
       130
<210> 32
   <211> 131
   <212> PRT
   <213> Phoenix dactylifera
<400> 32
   Met Ser Trp Gln Ala Tyr Val Asp Glu His Leu Met Cys Glu Ile Asp
   1               5                   10                  15
   Gly His His Leu Thr Ala Ala Ala Ile Leu Gly His Asp Gly Ser Val
               20                  25                  30
   Trp Ala Gln Ser Ser Ser Phe Pro Gln Phe Lys Ser Glu Glu Ile Thr
           35                  40                  45
   Asn Ile Met Asn Asp Phe Asn Glu Pro Gly Ser Leu Ala Pro Thr Gly
       50                  55                  60
   Leu Tyr Leu Gly Ser Thr Lys Tyr Met Val Ile Gln Gly Glu Pro Gly
   65                  70                  75                  80
   Ala Val Ile Arg Gly Lys Lys Gly Ser Gly Gly Val Thr Val Lys Lys
                   85                  90                  95
   Thr Asn Gln Ala Leu Ile Phe Gly Ile Tyr Glu Glu Pro Met Thr Pro
               100                 105                 110
   Gly Gln Cys Asn Met Val Val Glu Arg Leu Gly Asp Tyr Leu Ile Glu
           115                 120                 125
   Gln Gly Met
   130
<210> 33
   <211> 131
   <212> PRT
   <213> Chenopodium album
<400> 33
   Met Ser Trp Gln Thr Tyr Val Asp Asp His Leu Met Cys Asp Ile Glu
   1               5                   10                  15
   Gly Asn His Leu Ser Ser Ala Ala Ile Leu Gly His Asp Gly Thr Val
               20                  25                  30
   Trp Ala Gln Ser Pro Ser Phe Pro Gln Leu Lys Pro Glu Glu Val Ser
           35                  40                  45
   Ala Ile Met Lys Asp Phe Asn Glu Pro Gly Ser Leu Ala Pro Thr Gly
       50                  55                  60
   Leu His Leu Gly Gly Thr Lys Tyr Met Val Ile Gln Gly Glu Pro Gly
   65                  70                  75                  80
   Asp Val Ile Arg Gly Lys Lys Gly Pro Gly Gly Val Thr Ile Lys Lys
                   85                  90                  95
   Thr Asn Gln Ala Leu Ile Ile Gly Ile Tyr Gly Glu Pro Met Thr Pro
               100                 105                 110
   Gly Gln Cys Asn Met Val Val Glu Arg Ile Gly Asp Tyr Leu Val Glu
           115                 120                 125
   Gln Gly Met
       130
<210> 34
   <211> 133
   <212> PRT
   <213> Salsola kali
<400> 34
   Met Ser Trp Gln Thr Tyr Val Asp Asp His Leu Met Cys Glu Ile Glu
   1               5                   10                  15
   Gly Thr Asn Asn His Leu Thr Ala Ala Ala Ile Leu Gly Val Asp Gly
               20                  25                  30
   Ser Val Trp Ala Gln Ser Ala Asn Phe Pro Gln Phe Lys Pro Asp Glu
           35                  40                  45
   Ile Ser Ala Val Val Lys Glu Phe Asp Glu Ala Gly Thr Leu Ala Pro
       50                  55                  60
   Thr Gly Leu His Leu Gly Gly Thr Lys Tyr Met Val Ile Gln Gly Glu
   65                  70                  75                  80
   Ala Gly Gln Val Ile Arg Gly Lys Lys Gly Pro Gly Gly Ile Cys Val
                   85                  90                  95
   Lys Lys Thr Gly Gln Ala Leu Ile Phe Gly Ile Tyr Asp Glu Pro Val
               100                 105                 110
   Thr Pro Gly Gln Cys Asn Met Ile Val Glu Arg Leu Gly Asp Tyr Leu
           115                 120                 125
   Val Glu Gln Gly Met
       130
<210> 35
   <211> 133
   <212> PRT
   <213> Amaranthus retroflexus
<400> 35
   Met Ser Trp Gln Ala Tyr Val Asp Asp His Leu Met Cys Glu Ile Glu
   1               5                   10                  15
   Gly Thr Thr Asn His Leu Thr Gly Ala Ala Ile Leu Gly Leu Asp Gly
               20                  25                  30
   Ser Val Trp Ala Gln Ser Ala Asp Phe Pro Gln Phe Lys Pro Asp Glu
           35                  40                  45
   Ile Ala Ala Ile Val Glu Asp Phe Asp Glu Pro Gly Thr Leu Ala Pro
       50                  55                  60
   Thr Gly Leu His Leu Gly Gly Thr Lys Tyr Met Val Ile Gln Gly Glu
   65                  70                  75                  80
   Pro Gly Ala Val Ile Arg Gly Lys Lys Gly Ala Gly Gly Ile Cys Val
                   85                  90                  95
   Lys Lys Thr Gly Gln Ala Leu Val Met Gly Ile Tyr Asp Glu Pro Val
               100                 105                 110
   Thr Pro Gly Gln Cys Asn Met Ile Val Glu Arg Leu Gly Asp Tyr Leu
           115                 120                 125
   Ile Glu Gln Gly Tyr
   130
<210> 36
   <211> 134
   <212> PRT
   <213> Daucus carota
<400> 36
   Met Ser Trp Gln Thr Tyr Val Asp Asp His Leu Met Cys Glu Val Asp
   1 5 10 15
   Gly Asn Pro Gly Gln Gln Leu Ser Ala Ala Ala Ile Ile Gly His Asp
   20 25 30
   Gly Ser Val Trp Ala Gln Ser Ser Thr Phe Pro Lys Phe Lys Pro Glu
   35 40 45
   Glu Ile Thr Gly Ile Met Lys Asn Phe Asp Glu Pro Gly His Leu Ala
   50 55 60
   Pro Thr Gly Leu Tyr Leu Gly Gly Thr Lys Tyr Met Val Ile Gln Gly
   65 70 75 80
   Glu Pro Ile Ala Val Ile Arg Gly Lys Lys Gly Ser Gly Gly Val Thr
   85 90 95
   Ile Lys Lys Thr Gly Gln Ala Leu Val Phe Gly Val Tyr Asp Glu Pro
   100 105 110
   Val Thr Pro Gly Gln Cys Asn Leu Ile Val Glu Arg Leu Gly Asp Tyr
   115 120 125
   Leu Ile Glu Gln Gly Leu
   130
<210> 37
   <211> 134
   <212> PRT
   <213> Apium graveolens
<400> 37
   Met Ser Trp Gln Ala Tyr Val Asp Asp His Leu Met Cys Glu Val Glu
   1 5 10 15
   Gly Asn Pro Gly Gln Thr Leu Thr Ala Ala Ala Ile Ile Gly His Asp
   20 25 30
   Gly Ser Val Trp Ala Gln Ser Ser Thr Phe Pro Gln Ile Lys Pro Glu
           35                  40                  45
   Glu Ile Ala Gly Ile Met Lys Asp Phe Asp Glu Pro Gly His Leu Ala
       50                  55                  60
   Pro Thr Gly Leu Tyr Leu Gly Gly Ala Lys Tyr Met Val Ile Gln Gly
   65                  70                  75                  80
   Glu Pro Asn Ala Val Ile Arg Gly Lys Lys Gly Ser Gly Gly Val Thr
                   85                  90                  95
   Ile Lys Lys Thr Gly Gln Ala Leu Val Phe Gly Val Tyr Asp Glu Pro
               100                 105                 110
   Val Thr Pro Gly Gln Cys Asn Val Ile Val Glu Arg Leu Gly Asp Tyr
           115                 120                 125
   Leu Ile Asp Gln Gly Leu
       130
<210> 38
   <211> 131
   <212> PRT
   <213> Cucumis melo
<400> 38
   Met Ser Trp Gln Val Tyr Val Asp Glu His Leu Met Cys Glu Ile Glu
   1               5                   10                  15
   Gly Asn His Leu Thr Ser Ala Ala Ile Ile Gly Gln Asp Gly Ser Val
               20                  25                  30
   Trp Ala Gln Ser Gln Asn Phe Pro Gln Leu Lys Pro Glu Glu Val Ala
           35                  40                  45
   Gly Ile Val Gly Asp Phe Ala Asp Pro Gly Thr Leu Ala Pro Thr Gly
       50                  55                  60
   Leu Tyr Ile Gly Gly Thr Lys Tyr Met Val Ile Gln Gly Glu Pro Gly
   65                  70                  75                  80
   Ala Val Ile Arg Gly Lys Lys Gly Pro Gly Gly Ala Thr Val Lys Lys
                   85                  90                  95
   Thr Gly Met Ala Leu Val Ile Gly Ile Tyr Asp Glu Pro Met Thr Pro
               100                 105                 110
   Gly Gln Cys Asn Met Ile Val Glu Arg Leu Gly Asp Tyr Leu Ile Asp
   115 120 125
   Gln Gly Leu
   130
<210> 39
   <211> 131
   <212> PRT
   <213> Capsicum annuum
<400> 39
   Met Ser Trp Gln Thr Tyr Val Asp Asp His Leu Met Cys Glu Ile Glu
   1 5 10 15
   Gly Asn Arg Leu Thr Ser Ala Ala Ile Ile Gly Gln Asp Gly Ser Val
   20 25 30
   Trp Ala Gln Ser Ala Thr Phe Pro Gln Phe Lys Pro Glu Glu Ile Thr
   35 40 45
   Ala Ile Met Asn Asp Phe Ala Glu Pro Gly Thr Leu Ala Pro Thr Gly
   50 55 60
   Leu Tyr Leu Gly Gly Thr Lys Tyr Met Val Ile Gln Gly Glu Ala Gly
   65 70 75 80
   Ala Val Ile Arg Gly Lys Lys Gly Pro Gly Gly Ile Thr Val Lys Lys
   85 90 95
   Thr Asn Gln Ala Leu Ile Ile Gly Ile Tyr Asp Glu Pro Met Thr Pro
   100 105 110
   Gly Gln Cys Asn Met Ile Val Glu Arg Leu Gly Asp Tyr Leu Ile Glu
   115 120 125
   Gln Ser Leu
   130
<210> 40
   <211> 131
   <212> PRT
   <213> Ananas comosus
<400> 40
   Met Ser Trp Gln Ala Tyr Val Asp Asp His Leu Met Cys Glu Ile Asp
   1 5 10 15
   Gly Gln His Leu Ser Ser Ala Ala Ile Leu Gly His Asp Ser Thr Val
   20 25 30
   Trp Ala Gln Ser Pro Asn Phe Pro Gln Phe Lys Pro Glu Glu Ile Ser
   35 40 45
   Ala Ile Leu Asn Asp Phe Glu Asn Pro Gly Ser Leu Ala Pro Thr Gly
   50 55 60
   Leu Tyr Leu Gly Gly Thr Lys Tyr Met Val Ile Gln Gly Glu Pro Gly
   65 70 75 80
   Val Val Ile Arg Gly Lys Lys Gly Thr Gly Gly Ile Thr Val Lys Lys
   85 90 95
   Thr Asn Leu Ala Leu Ile Ile Gly Val Tyr Asp Glu Pro Met Thr Pro
   100 105 110
   Gly Gln Cys Asn Met Val Val Glu Arg Leu Gly Asp Tyr Leu Leu Glu
   115 120 125
   Gln Gly Phe
   130
<210> 41
   <211> 131
   <212> PRT
   <213> Crocus sativus
<400> 41
   Met Ser Trp Gln Thr Tyr Val Asp Glu His Leu Met Cys Asp Met Asp
   1 5 10 15
   Gly His Val Leu Thr Ser Ala Ala Ile Leu Gly His Asp Gly Ser Val
   20 25 30
   Trp Ala Gln Ser Ala Gly Phe Pro Glu Leu Lys Pro Ala Glu Ile Thr
   35 40 45
   Ala Ile Leu Asn Asp Phe Asn Glu Pro Gly Ser Leu Ala Pro Thr Gly
   50 55 60
   Met Tyr Ile Asn Gly Ala Lys Tyr Met Val Ile Gln Gly Glu Pro Gly
   65 70 75 80
   Val Val Ile Arg Gly Lys Lys Gly Ser Gly Gly Val Thr Ile Lys Lys
   85 90 95
   Ser Asn Met Ala Leu Ile Phe Gly Leu Tyr Asp Glu Pro Met Thr Pro
   100 105 110
   Gly Gln Cys Asn Leu Val Val Glu Arg Leu Gly Asp Tyr Leu Ile Glu
   115 120 125
   Gln Gly Tyr
   130
<210> 42
   <211> 131
   <212> PRT
   <213> Musa acuminata
<400> 42
   Met Ser Trp Gln Ala Tyr Val Asp Asp His Leu Leu Cys Asp Ile Asp
   1 5 10 15
   Gly Gln Cys Leu Thr Ala Ala Ala Ile Val Gly His Asp Gly Ser Val
   20 25 30
   Trp Ala Gln Ser Asp Ala Phe Pro Gln Cys Lys Pro Glu Glu Ile Ala
   35 40 45
   Ala Ile Met Lys Asp Phe Asp Glu Pro Gly Ser Leu Ala Pro Thr Gly
   50 55 60
   Leu Tyr Leu Gly Gly Thr Lys Tyr Met Val Ile Gln Gly Glu Pro Gly
   65 70 75 80
   Ala Val Ile Arg Gly Lys Lys Gly Ser Gly Gly Val Thr Ile Lys Lys
   85 90 95
   Thr Asn Leu Ala Leu Ile Ile Gly Ile Tyr Asn Glu Pro Met Thr Pro
   100 105 110
   Gly Gln Cys Asn Met Val Val Glu Arg Leu Gly Asp Tyr Leu Phe Asp
   115 120 125
   Gln Gly Phe
   130
<210> 43
   <211> 56
   <212> PRT
   <213> Phleum pratense
<220>
   <221> MISC_FEATURE
   <222> (1)..(56)
   <223> Amino acids 71-127 of SEQ ID NO: 1
<400> 43
   Tyr Met Val Ile Gln Gly Glu Pro Gly Ala Val Ile Arg Gly Lys Lys
   1               5                   10                  15
   Gly Ala Gly Gly Ile Thr Ile Lys Lys Thr Gly Gln Ala Leu Val Val
               20                  25                  30
   Gly Ile Tyr Asp Glu Pro Met Thr Pro Gly Gln Cys Asn Met Val Val
           35                  40                  45
   Glu Arg Leu Gly Asp Tyr Leu Val
       50                  55
<210> 44
   <211> 131
   <212> PRT
   <213> Artificial seqeunce
<220>
   <223> SEQ ID NO: 1 with substitutions C13S and C115S
<400> 44
   Met Ser Trp Gln Thr Tyr Val Asp Glu His Leu Met Ser Glu Ile Glu
   1               5                   10                  15
   Gly His His Leu Ala Ser Ala Ala Ile Leu Gly His Asp Gly Thr Val
               20                  25                  30
   Trp Ala Gln Ser Ala Asp Phe Pro Gln Phe Lys Pro Glu Glu Ile Thr
           35                  40                  45
   Gly Ile Met Lys Asp Phe Asp Glu Pro Gly His Leu Ala Pro Thr Gly
       50                  55                  60
   Met Phe Val Ala Gly Ala Lys Tyr Met Val Ile Gln Gly Glu Pro Gly
   65                  70                  75                  80
   Ala Val Ile Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Ser Asn Met Val Val Glu Arg Leu Gly Asp Tyr Leu Val Glu
           115                 120                 125
   Gln Gly Met
       130
<210> 45
   <211> 131
   <212> PRT
   <213> Artificial sequence
<220>
   <223> SEQ ID NO: 1 with substitutions C13A and C115A
<400> 45
   Met Ser Trp Gln Thr Tyr Val Asp Glu His Leu Met Ala Glu Ile Glu
   1               5                   10                  15
   Gly His His Leu Ala Ser Ala Ala Ile Leu Gly His Asp Gly Thr Val
               20                  25                  30
   Trp Ala Gln Ser Ala Asp Phe Pro Gln Phe Lys Pro Glu Glu Ile Thr
           35                  40                  45
   Gly Ile Met Lys Asp Phe Asp Glu Pro Gly His Leu Ala Pro Thr Gly
       50                  55                  60
   Met Phe Val Ala Gly Ala Lys Tyr Met Val Ile Gln Gly Glu Pro Gly
   65                  70                  75                  80
   Ala Val Ile Arg Gly Lys Lys Gly Ala Gly Gly Ile Thr Ile Lys Lys
                   85                  90                  95
   Thr Gly Gln Ala Leu Val Val Gly Ile Tyr Asp Glu Pro Met Thr Pro
               100                 105                 110
   Gly Gln Ala Asn Met Val Val Glu Arg Leu Gly Asp Tyr Leu Val Glu
           115                 120                 125
   Gln Gly Met
       130
<210> 46
   <211> 396
   <212> DNA
   <213> Artificial sequence
<220>
   <223> DNA encoding SEQ ID NO: 44
<400> 46
   atgagctggc aaacgtatgt cgatgaacac ctgatgagcg aaattgaagg tcaccacctg      60
   gcgtcggcgg ctattctggg tcacgatggc accgtttggg cacagagcgc tgattttccg     120
   caattcaaac cggaagaaat taccggcatc atgaaggatt ttgacgaacc gggtcatctg     180
   gcaccgacgg gcatgttcgt cgcaggtgcc aaatatatgg tgattcaggg tgaaccgggt     240
   gcagtcatcc gtggcaaaaa gggtgccggc ggtattacca tcaaaaagac gggccaagcc     300
   ctggtggttg gtatttacga cgaaccgatg acgccgggtc aaagcaacat ggtggtggaa     360
   cgtctgggcg actatctggt ggaacagggt atgtag                                396
<210> 47
   <211> 326
   <212> DNA
   <213> Artificial sequence
<220>
   <223> DNA encoding SEQ ID NO: 45
<400> 47
   cgattctggg tcacgatggc accgtttggg cacagagcgc tgattttccg caattcaaac      60
   cggaagaaat taccggcatc atgaaggatt ttgacgaacc gggtcatctg gcaccgacgg     120
   gcatgttcgt cgcaggtgcc aaatatatgg tgattcaggg tgaaccgggt gcagtcatcc     180
   gtggcaaaaa gggtgccggc ggtattacca tcaaaaagac gggccaagcc ctggtggttg     240
   gtatttacga cgaaccgatg acgccgggtc aagcaaacat ggtggtggaa cgtctgggtg     300
   attatctggt ggaacagggt atgtag                                          326

## Description

### Technical Field

The invention is in the field of medical science and immunology, more specifically in the field of allergy, immunotherapy and bystander tolerance.

### Background

Allergen-specific immunotherapy (SIT) is a hyposensitizing immunotherapy introduced in clinical medicine almost a century ago for the treatment of a type 1 hypersensitivity immune response. The hyposensitizing immunotherapy consists in repeatedly administration of the specific allergen(s) to which the individual(s) is/are sensitized to or administration of a cross-reacting allergen thereof, usually either by subcutaneous administration or by sublingual administration. The repeated administration is usually conducted daily, weekly or monthly during a longer period, usually more than one year, so as to achieve a sort of immunological tolerance towards the specific allergen with subsequent disappearance of allergic symptoms (Allergens and Allergen Immunotherapy 4th Ed, 2008, Ed by R Lockey and D Ledford, Informa healthcare). Hyposensitizing immunotherapy is typically associated with the generation of specific IgG4 antibodies and reduced levels of specific IgE.

However, one challenge encountered with SIT is that the specific allergen needs to be identified and that the risk of eliciting serious adverse effects may limit general application of SIT in the treatment of a hypersensitivity immune response.

Therefore, there is a need of improving the treatment of a hypersensitivity immune response.

The concept of treating an individual with one antigen in the suppression of a hypersensitivity immune response caused by another un-related antigen was demonstrated more than 20 years ago. Miller et al (1991) showed that peripheral tolerance to an antigen (MBP) (myelin basic protein) could be achieved in naïve rats treated repeatedly by intra-gastric administration of OVA. However, tolerance to MBP was only achieved if rats were co-exposed to both MPB and OVA at the time of challenging rats to MBP to develop EAE. The concept has been further investigated by Dahlman-Hoglund et al (1995) and Millington et al (2004) who showed that naïve rats fed with a first antigen could be prevented from developing a hypersensitivity immune response triggered by a second antigen if the rats were exposed to both the first antigen and the second antigen at the time of challenging the animal to the second antigen. Oliveira CR et al (2005) have suggested that bystander effect may be an interesting mechanism to control sensitization to a new allergen in individuals already sensitized to one allergen.

Profilins are naturally occurring polypeptides typically consisting of 125-135 amino acids and found ubiquitous in organisms from yeast to man (Santos and Van Ree 2011). They are present in high concentrations in the cytoplasm of cells from vertebrates, plants, etc. and specifically bind actin, poly-L-proline (PLP) and phoshatidylinositol-4.5-biphosphate (PIP₂). Profilins can be divided into four different classes depending on their source a) plant, b) mammals, c) viruses, d) other eukaryotes. Between the different classes, the amino acid sequence homology is low (∼30%), but within-class homology is much higher, such as about 50 to 90% (Thorn et al, 1997).

Some plant profilins are identified as allergens (Martinez A et al, 2002), but the prevalence of allergic patients sensitive to plant profilins are low, though variable. For example, about 5-15% of patients allergic to allergens of grass pollen were found to be allergic to a profilin of grass pollen, whereas about 24% of patients allergic to pollen of olive trees have IgE recognizing a profilin of olive pollen (Ole e 2).

### Summary of the Invention

In contrast to specific allergen immunotherapy, the present invention relates to non-specific immunotherapy for the treatment/prevention of a hypersensitivity immune response caused by a non-profilin allergen of a profilin-containing plant material.

The present inventors have provided evidence that a hypersensitivity immune response, such as asthma, triggered by exposure to a non-profillin allergen of a profilin-containing plant matererial can be suppressed via bystander tolerance in naïve mice as well as in sensitized mice treated by sublingual administration of a profilin of the profilin-containing plant material. Additionally, it was demonstrated that the same treatment may also suppress a hypersensitive immune response caused by another profilin-containing plant material, thus indicating that the creation of bystander tolerance to one profilin polypeptide may be reactivated by another profilin polypeptide so as to produce an immunological event that in turn down-regulates a hypersensitivity immune response. In accordance with previous studies of bystander tolerance, suppression could only be demonstrated where mice at the time of exposure to the non-profilin allergen were also co-exposed to the polypeptide/profilin.

Thus, the data provided herein indicates that a hypersensitivity immune response caused by a first antigen can be suppressed by treating an individual with a second and different antigen provided that the individual is exposed to both the first and second antigen or an alternative second antigen, when the individual is exposed to the "disease triggering" first antigen.

It has been recognized that repeated administration of a tolerance-inducing antigen to an individual will lead to attenuation of an immune response caused by an offensive and different antigen, such as an allergen, provided that the two antigens are both present at the time the individual is exposed to the offensive antigen. It is envisaged that the individual is made tolerant to the tolerance-inducing antigen and when an allergen-source material like pollen comes in contact with the airway mucosa of that individual, the allergen and the tolerance-inducing antigen become released at the same time and location and that the tolerance-inducing antigen will activate the previously induced immunological tolerance response which, in turn, will down-regulate the hypersensitivity immune response towards the nearby released allergens.

Thus, the present therapy comprises the administration of a first antigen to which the individual becomes tolerant and upon being exposed to an allergen source material containing both the allergen triggering the hypersensisitivity immune response and the tolerance-inducing antigen, the hypersensitivity immune response that is otherwise expected to occur will be suppressed either entirely or party.

It is believed that T cells like Treg cells, and likely also Th-2 cells, are important for achieving this bystander tolerance. For example co-localization of antigen-presenting cells (APCs), Treg cells and Th-2 cells in the draining lymph nodes may be important. Tolerogenic APCs and production of regulatory cytokines in epithelial tissue may also contribute to tolerance induction.

It has now been recognized that a polypeptide having an amino acid sequence having at least 60% identity and/or similarity to profilin of the plant species *Phleum pratense* may be used in immunotherapy for the suppression of a hypersensivity immune response caused by a non-profilin allergen of a profilin-containing plant material.

Accordingly, a first aspect of the invention relates to a polypeptide for use in the treatment or prevention, such as by immunotherapy, of a hypersensitivity immune response in an individual to a non-profilin allergen of a profilin-containing plant material, wherein
said polypeptide has/consists of, consists essentially of/comprises an amino sequence having at least 60% identity to the amino acid sequence of SEQ ID NO: 1.

Preferably, the individual to be treated is, at least not when administering the first dose of the polypeptide, neither allergic nor sensitized to the profilin of the profilin-containing plant material and/or to the polypeptide. Thus, said hypersensitivity immune response may not be caused by a profilin of said profilin-containing plant material. It is also envisaged that the amino acid sequence of the profilin of the profilin-containing plant material and the amino acid sequence of the polypeptide being administered have at least 60% identity and/or similarity.

Therefore, in one subaspect, the invention relates to a polypeptide for use in the treatment or prevention, such as by immunotherapy, of a hypersensitivity immune response in an individual to a non-profilin allergen of a profilin-containing plant material, wherein
said polypeptide has/ consists of, consists essentially of/comprises an amino sequence having at least 60% identity to the amino acid sequence of SEQ ID NO: 1;
said individual is, at least not when administering the first dose of the polypeptide, not sensitized to the profilin of the profilin-containing plant material and preferably not sensitized to the polypeptide either;
preferably the amino acid sequence of the profilin of the profilin-containing plant material has at least 60% identity and/or similarity to the amino acid sequence of said polypeptide.

The invention thus enables a method for treatment or prevention, such as by immunotherapy, of a hypersensitivity immune response in an individual to a non-profilin allergen of a profilin-containing plant material comprising administering a therapeutically effective amount of a polypeptide having/ consisting of, consisting essentially of/comprising an amino acid sequence having at least 60% identity to the amino acid sequence of SEQ ID NO: 1; wherein preferably
said individual is not sensitized to a profilin of said profilin-containing plant material and/or said polypeptide; and preferably wherein the polypeptide has an amino acid sequence having at least 60% similarity or identity to the amino acid sequence of the profilin of the profilin-containing plant material.

The invention also enables the use of a polypeptide for the manufacturing of a medicament for the treatment or prevention, such as by immunotherapy, of a hypersensitivity immune response in an individual to a non-profilin allergen of a profilin-containing plant material, wherein
said polypeptide has/ consists of, consists essentially of/comprises an amino sequence having at least 60% identity to the amino acid sequence of SEQ ID NO: 1; and preferably
said individual is not sensitized to a profilin of said profilin-containing plant material and/or said polypeptide; and preferably wherein the polypeptide has an amino acid sequence having at least 60% similarity or identity to the amino acid sequence of the profilin of the profilin-containing plant material.

The amino acid sequence of SEQ ID NO: 1 is one of the biological isomers of the profilin Phl p 12 found in grass pollen of the species *Phleum pratense.* Other biological isomers having an amino acid sequence with at least 95%, such as about 98%, identity or similarity to SEQ ID NO: 1 is reported herein as SEQ ID NOs: 2-10.

A second aspect of the invention relates to a cysteine substitution variant of a polypeptide of the present invention, such as to a polypeptide having an amino acid sequence of SEQ ID NOs: 1, 2, 3, 4, 5, 6, 7, 8, 9 or 10, wherein 1 or 2 cysteine residue(s) is/are substituted by an amino acid selected from A, G and/or S, such as to a polypeptide having an amino acid sequence of SEQ ID NOs: 1, 2, 3, 4, 5, 6, 7, 8, 9 or 10, where the amino acid cysteine in position 13 and/or 115 has been substituted by an amino acid selected from the group comprising A (alanine), G (glycine) and S (serine), such as a polypeptide having an amino acid sequence of SEQ ID NOs: 44 or 45. Notably, the SEQ ID NOs 1 to 10 and 44 and 45 have a methionine as the first amino acid at the N-terminal end (position 1), which may be removed by enzymatic degradation in a host cell producing the sequences by recombinant techniques. Therefore, it should be understood that the cysteine substitution variant has an amino acid sequence of SEQ ID NO: 44 and 45, but where the methionine group in position 1 is not present. Thus, in some embodiments, the amino acid sequence of SEQ ID NOs: 44 and/or 45 does not contain methionine in position 1. Furthermore, a second aspect thereof also relates to an isolated nucleic acid encoding the protein of SEQ ID NOs: 44 or 45, such as an isolated nucleic acid according having the nucleotide sequence of SEQ ID NOs: 46 or 47.

Obtaining a polypeptide suitable for use in treating, via bystander tolerance, a hypersensitivity immune response caused by a non-profilin allergen of a profilin-containing plant material, may entail isolating an immunogenic polypeptide from said profilin-containing plant material or recombinant producing said immunogenic polypeptide, wherein said immunogenic polypeptide has been identified in and optionally isolated from an extract made by suspending the profilin-containing plant material in an aqueous solution having pH in the range of 6-8 for a period ranging from 1 to 30 minutes. Preferably, the extract also contains a non-profilin allergen of said profilin-containing plant material, i.e. a non-profilin allergen co-extracted with the immunogenic polypeptide.

A polypeptide suitable for use in treating, via bystander tolerance, a hypersensitivity immune response caused by a non-profilin allergen of a profilin-containing plant material, may be obtained by isolating an immunogenic polypeptide from said profilin-containing plant material or recombinantly producing said immunogenic polypeptide, wherein said immunogenic polypeptide has been identified in and optionally isolated from an extract made by suspending the profilin-containing plant material in an aqueous solution having pH in the range of 6-8 for a period ranging from 1 to 30 minutes. Preferably, the extract also contains a non-profilin allergen of said profilin-containing plant material, i.e. a non-profilin allergen co-extracted with the immunogenic polypeptide.

Further embodiments of the invention and alternative wording of the invention are presented below:
- A polypeptide for use of the invention in the treatment or prevention of a hypersensitivity immune response in an individual caused by a non-profilin allergen of a profilin-containing plant material, wherein said polypeptide is a profilin of a profilin-containing plant material of a plant order selected from the group consisting of *Pinales*, *Arecales*, *Asparagales*, *Poales*, *Zingiberales, Apiales, Asterales, Brassicalis, Curcurbitales, Ericales, Fabales, Fagales, Gentianales, Lamiales, Laurales, Malvales, Malpighiales, Myrtales, Proteales, Rosales, Sapindales*, *Solanales and Vitales* or said polypeptide is a variant of said profilin; and
   *wherein* said hypersensitivity immune response is caused by a non-profilin allergen of a profilin-containing plant from a plant order selected from the group consisting of *Pinales*, *Arecales*, *Asparagales*, *Poales*, *Zingiberales*, *Apiales*, *Asterales*, *Brassicalis, Curcurbitales*, *Ericales, Fabales, Fagales, Gentianales, Lamiales, Laurales, Malvales, Malpighiales, Myrtales, Proteales, Rosales, Sapindales, Solanales and Vitales.*
- A polypeptide for use of the invention in the treatment or prevention of a hypersensitivity immune response in an individual caused by a non-profilin allergen of a profilin-containing plant material, wherein said polypeptide is a profilin of a profilin-containing plant material of a plant family selected from the group consisting of *Cupressaceae*, *Arecaceae*, *Asparagaceae, Iridaceae, Bromeliaceae, Poaceae, Musaceae, Zingiberaceae, Apiaceae, Araliaceae, Asteraceae, Brassicaceae, Amaranthaceae, Caryophyllaceae, Polygonaceae, Cucurbitaceae, Actinidiaceae, Lecythidaceae, Theaceae, Fabaceae, Betulaceae, Fagaceae, Juglandaceae, Myricaceae*, *Nothofagaceae, Ticodendraceae, Apocynaceae, Rubiaceae, Oleaceae, Pedaliacae, Plantaginaceae, Lauraceae, Malvaceae, Euphorbiaceae, Lythraceae, Platanaceae, Cannabaceae, Rosaceae, Ulmaceae, Urticaceae, Anacardiaceae, Rutaceae, Sapindaceae*, *Solanaceae and Vitaceae* or said polypeptide is a variant of said profilin; and
   *wherein* said hypersensitivity immune response is caused by a non-profilin allergen of a profilin-containing plant from a plant family selected from the group consisting of *Cupressaceae*, *Arecaceae*, *Asparagaceae*, *Iridaceae*, *Bromeliaceae*, *Poaceae*, *Musaceae, Zingiberaceae, Apiaceae, Araliaceae, Asteraceae, Brassicaceae, Amaranthaceae, Caryophyllaceae, Polygonaceae, Cucurbitaceae, Actinidiaceae, Lecythidaceae, Theaceae, Fabaceae, Betulaceae, Fagaceae, Juglandaceae, Myricaceae, Nothofagaceae, Ticodendraceae, Apocynaceae, Rubiaceae, Oleaceae, Pedaliacae, Plantaginaceae*, *Lauraceae*, *Malvaceae*, *Euphorbiaceae*, Lythraceae, *Platanaceae*, *Cannabaceae, Rosaceae, Ulmaceae, Urticaceae, Anacardiaceae, Rutaceae, Sapindaceae, Solanaceae and Vitaceae.*
- A polypeptide for use of the invention in the treatment or prevention of a hypersensitivity immune response in an individual caused by a non-profilin allergen of a profilin-containing plant material, wherein said polypeptide is a profilin of a profilin-containing plant material from a genus selected from the group consisting of *Chamaecyparis*, *Cryptomeria*, *Cupressus*, *Juniperus*, *Phoenix*, *Asparagus*, *Crocus*, *Ananas*, *Anthoxanthum*, *Cynodon*, *Dactylis*, *Festuca, Holcus, Hordeum, Lolium, Oryza, Paspalum, Phalaris, Phleum, Poa, Secale, Sorghum*, *Triticum*, *Zea*, *Musa*, *Apium*, *Daucus*, *Ambrosia*, *Artemisia*, *Helianthus*, *Lactuca*, *Arabidopsis, Brassica, Sinapis, Amaranthus, Beta, Chenopodium, Fagopyrum, Salsola,* Cucumis, *Actinidia*, *Bertholletia*, *Arachis*, *Glycine*, *Lens*, *Lupinus*, *Phaseolus*, *Pisum*, *Vigna*, *Alnus, Betula, Carpinus, Carya, Castanea, Corylus, Fagus, Juglans, Ostrya, Quercus, Catharanthus, Coffea, Fraxinus, Ligustrum, Olea, Plantago, Sesamum, Syringa, Persea, Gossypium, Hevea, Manihot, Mercurialis, Popolus, Ricinus, Sonneratia, Platanus, Fragaria, Humulus, Malus, Morus, Parietaria, Prunus, Pyrus, Rubus, Ziziphus, Anacardium, Citrus, Litchi*, *Mangifera*, *Pistacia*, *Capsicum*, *Lycopersicon*, *Solanum* and *Vitis* or said polypeptide is a variant of said profilin; and
   *wherein* said hypersensitivity immune response is caused by a non-profilin allergen of a profilin-containing plant from a genus selected from the group consisting of *Chamaecyparis*, *Cryptomeria*, *Cupressus*, *Juniperus*, *Phoenix*, *Asparagus*, *Crocus*, *Ananas*, *Anthoxanthum, Cynodon, Dactylis, Festuca, Holcus, Hordeum, Lolium, Oryza*, *Paspalum, Phalaris, Phleum, Poa, Secale, Sorghum, Triticum, Zea, Musa, Apium, Daucus, Ambrosia, Artemisia, Helianthus, Lactuca, Arabidopsis, Brassica, Sinapis, Amaranthus, Beta, Chenopodium, Fagopyrum, Salsola,* Cucumis, *Actinidia, Bertholletia, Arachis, Glycine, Lens, Lupinus, Phaseolus, Pisum, Vigna, Alnus, Betula, Carpinus, Carya, Castanea, Corylus, Fagus, Juglans, Ostrya, Quercus, Catharanthus, Coffea, Fraxinus, Ligustrum, Olea, Plantago, Sesamum, Syringa, Persea, Gossypium, Hevea, Manihot, Mercurialis, Popolus, Ricinus, Sonneratia, Platanus, Fragaria, Humulus, Malus, Morus, Parietaria, Prunus, Pyrus, Rubus, Ziziphus, Anacardium, Citrus, Litchi, Mangifera, Pistacia, Capsicum, Lycopersicon, Solanum* and *Vitis.*
- A polypeptide for use of the invention in the treatment or prevention of a hypersensitivity immune response in an individual caused by a non-profilin allergen of a profilin-containing plant material, wherein said polypeptide is a profilin of a profilin-containing plant material from a genus selected from the group consisting of *Chamaecyparis, Cryptomeria, Cupressus, Juniperus, Phoenix, Asparagus, Crocus, Ananas, Anthoxanthum, Cynodon, Dactylis, Festuca, Holcus, Hordeum, Lolium, Oryza, Paspalum, Phalaris, Phleum, Poa, Secale, Sorghum, Triticum, Zea, Musa, Apium, Daucus, Ambrosia, Artemisia, Helianthus, Lactuca, Arabidopsis, Brassica, Sinapis, Amaranthus, Beta, Chenopodium, Fagopyrum, Salsola, Cucumis, Actinidia, Bertholletia, Arachis, Glycine, Lens, Lupinus, Phaseolus, Pisum, Vigna, Alnus, Betula, Carpinus, Carya, Castanea, Corylus, Fagus, Juglans, Ostrya, Quercus, Catharanthus, Coffea, Fraxinus, Ligustrum, Olea, Plantago, Sesamum, Syringa, Persea, Gossypium, Hevea, Manihot, Mercurialis, Popolus, Ricinus, Sonneratia, Platanus, Fragaria, Humulus, Malus, Morus, Parietaria, Prunus, Pyrus, Rubus, Ziziphus, Anacardium, Citrus, Litchi, Mangifera, Pistacia, Capsicum, Lycopersicon, Solanum* and *Vitis* or said polypeptide is a variant of said profilin; and
   *wherein* said non-profilin allergen is selected from the group consisting of Cha o 1, Cha o 2, Cry j 1, Cry j 2, Cup a 1, Cup s 1, Cup s 3, Jun a 1, Jun a 2, Jun a 3, Jun o 4, Jun s 1, Jun v 1, Jun v 3, Ana c 2, Ant o 1, Aspa o 1, Cro s 1, Cro s 2, Cyn d 1, Cyn d 7, Cyn d 15, Cyn d 22w, Cyn d 23, Cyn d 24, Dac g 1, Dac g 2, Dac g 3, Dac g 4, Dac g 5, Fes p 4, Hol l 1, Hol l 5, Hor v 1, Hor v 5, Hor v 12, Hor v 15, Hor v 16, Hor v 17, Hor v 21, Lol p 1, Lol p 2, Lol p 3, Lol p 4, Lol p 5, Lol p 11, Mus a 2, Mus a 3, Mus a 4, Mus a 5, Ory s 1, Ory s 12, Pas n 1, Pha a 1, Pha a 5, Phl p 1, Phl p 2, Phl p 4, Phl p 5, Phl p 6, Phl p 7, Phl p 11, Phl p 12, Phl p 13, Pho d 2, Poa p 1, Poa p 5, Sec c 1, Sec c 5, Sec c 20, Sor h 1, Tri a 14, Tri a 15, Tri a 18, Tri a 19, Tri a 21, Tri a 25, Tri a 26, Tri a 27, Tri a 28, Tri a 29, Tri a 30, Tri a 31, Tri a 32, Tri a 33, Tri a 34, Tri a 35, Tri a 36, Tri a 37, Zea m 1, Zea m 12, Zea m 14, Zea m 25, Act c 5, Act c 8, Act c 10, Act d 1, Act d 2, Act d 3, Act d 4, Act d 5, Act d 6, Act d 7, Act d 8, Act d 10, Act d 11, Aln g 1, Aln g 4, Amb a 1, Amb a 2, Amb a 3, Amb a 4, Amb a 5, Amb a 6, Amb a 7, Amb a 9, Amb a 10, Amb p 5, Amb t 5, Ana o 1, Ana o 2, Ana o 3, Api g 1, Api g 2, Api g 3, Api g 5, Api g 6, Ara h 1, Ara h 2, Ara h 3, Ara h 4, Ara h 6, Ara h 7, Ara h 9, Ara h 10, Ara h 11, Art v 1, Art v 2, Art v 3, Art v 5, Art v 6, Ber e 1, Ber e 2, Beta v 1, Bet v 1, Bet v 3, Bet v 4, Bet v 6, Bet v 7, Bra j 1, Bra n 1, Bra o 3, Bra r 1, Bra r 2, Cap a 1w, Car b 1, Car i 1, Car i 4, Cas s 1, Cas s 5, Cas s 8, Cas s 9, Cat r 1, Che a 1, Che a 3, Cit l 3, Cit r 3, Cit s 1, Cit s 3, Cof a 1, Cor a 1, Cor a 8, Cor a 9, Cor a 10, Cor a 11, Cor a 12, Cor a 13, Cor a 14, Cuc m 1, Cuc m 3, Dau c 1, Fag e 2, Fag t 2, Fag s 1, Fra a 1, Fra a 3, Fra e 1, Gly m 1, Gly m 2, Gly m 4, Gly m 5, Gly m 6, Hel a 1, Hel a 3, Hev b 1, Hev b 2, Hev b 3, Hev b 4, Hev b 5, Hev b 6, Hev b 7, Hev b 9, Hev b 10, Hev b 11, Hev b 12, Hev b 13, Hev b 14, Hum j 1, Jug n 1, Jug n 2, Jug r 1, Jug r 2, Jug r 3, Jug r 4, Lac s 1, Len c 1, Len c 2, Len c 3, Lig v 1, Lup an 1, Lyc e 2, Lyc e 3, Lyc e 4, Mal d 1, Mal d 2, Mal d 3, Man e 5, Mer a 1, Mor n 3, Ole e 1, Ole e 3, Ole e 4, Ole e 5, Ole e 6, Ole e 7, Ole e 8, Ole e 9, Ole e 10, Ole e 11, Ost c 1, Par j 1, Par j 2, Par j 4, Par o 1, Pers a 1, Pha v 3, Pis v 1, Pis v 2, Pis v 3, Pis v 4, Pis v 5, Pis s 1, Pis s 2, Pla l 1, Pla a 1, Pla a 2, Pla or 1, Pla or 2, Pla or 3, Pru ar 1, Pru ar 3, Pru av 1, Pru av 2, Pru av 3, Pru d 3, Pru du 3, Pru du 5, Pru du 6, Pru p 1, Pru p 2, Pru p 3, Pyr c 1, Pyr c 3, Pyr c 5, Que a 1, Ric c 1, Rub i 1, Rub i 3, Sal k 1, Sal k 2, Sal k 3, Sal k 5, Ses i 1, Ses i 2, Ses i 3, Ses i 4, Ses i 5, Ses i 6, Ses i 7, Sin a 1, Sin a 2, Sin a 3, Sola t 1, Sola t 2, Sola t 3, Sola t 4, Syr v 1, Syr v 3, Vig r 1, Vit v 1 and Ziz m 1.
- A polypeptide for use of the invention in the treatment or prevention of a hypersensitivity immune response in an individual caused by a non-profilin allergen of a profilin-containing plant material, wherein said polypeptide is a plant profilin selected from the group consisting of Cry j profilin, Pho d 2, Ana c 1, Cyn d 12, Tri a 12, Mus a 1, Api g 4, Dau c 4, Amb a 8, Art v 4, Hel a 2, Ara t 8, Sin a 4, Ama r 2, Beta v 2, Che e 2, Sal k 4, Act d 9, Cuc m 2, Ara h 5, Gly m 3, Bet v 2, Cor a 2, Ole e 2, Hev b 8, Mer a 12, Pla a 3, Fra a 4, Mal d 4, Par j 3, Pru av 4, Pru du 4, Pru p 4, Pyr c 4, Cit s 2, Lit c 1, Cap a 2 and Lyc e 1 or said polypeptide is a variant of said profilin; and
   *wherein* said hypersensitivity immune response is caused by a non-profilin allergen of a profilin-containing plant is from a genus selected from the group consisting of *Chamaecyparis, Cryptomeria, Cupressus, Juniperus, Phoenix, Asparagus, Crocus, Ananas, Anthoxanthum, Cynodon, Dactylis, Festuca, Holcus, Hordeum, Lolium, Oryza, Paspalum, Phalaris, Phleum, Poa, Secale, Sorghum, Triticum, Zea, Musa, Apium, Daucus, Ambrosia, Artemisia, Helianthus, Lactuca, Arabidopsis, Brassica, Sinapis, Amaranthus, Beta, Chenopodium, Fagopyrum, Salsola,* Cucumis, *Actinidia, Bertholletia, Arachis, Glycine, Lens, Lupinus, Phaseolus, Pisum, Vigna, Alnus, Betula, Carpinus, Carya, Castanea, Corylus, Fagus, Juglans, Ostrya, Quercus, Catharanthus, Coffea, Fraxinus, Ligustrum, Olea, Plantago, Sesamum, Syringa, Persea, Gossypium, Hevea, Manihot, Mercurialis, Popolus, Ricinus, Sonneratia, Platanus, Fragaria, Humulus, Malus, Morus, Parietaria, Prunus, Pyrus, Rubus, Ziziphus, Anacardium, Citrus, Litchi, Mangifera, Pistacia, Capsicum, Lycopersicon, Solanum* and *Vitis.*
- A polypeptide for use of the invention in the treatment or prevention of a hypersensitivity immune response in an individual caused by a non-profilin allergen of a profilin-containing plant material, wherein said polypeptide is a plant profilin selected from the group consisting of Cry j profilin, Pho d 2, Ana c 1, Cyn d 12, Tri a 12, Mus a 1, Api g 4, Dau c 4, Amb a 8, Art v 4, Hel a 2, Ara t 8, Sin a 4, Ama r 2, Beta v 2, Che e 2, Sal k 4, Act d 9, Cuc m 2, Ara h 5, Gly m 3, Bet v 2, Cor a 2, Ole e 2, Hev b 8, Mer a 12, Pla a 3, Fra a 4, Mal d 4, Par j 3, Pru av 4, Pru du 4, Pru p 4, Pyr c 4, Cit s 2, Lit c 1, Cap a 2and Lyc e 1 or said polypeptide is a variant of said profilin; and
   *wherein* said hypersensitivity immune response is caused by a non-profilin allergen selected from the group consisting of Cha o 1, Cha o 2, Cry j 1, Cry j 2, Cup a 1, Cup s 1, Cup s 3, Jun a 1, Jun a 2, Jun a 3, Jun o 4, Jun s 1, Jun v 1, Jun v 3, Ana c 2, Ant o 1, Aspa o 1, Cro s 1, Cro s 2, Cyn d 1, Cyn d 7, Cyn d 15, Cyn d 22w, Cyn d 23, Cyn d 24, Dac g 1, Dac g 2, Dac g 3, Dac g 4, Dac g 5, Fes p 4, Hol l 1, Hol l 5, Hor v 1, Hor v 5, Hor v 12, Hor v 15, Hor v 16, Hor v 17, Hor v 21, Lol p 1, Lol p 2, Lol p 3, Lol p 4, Lol p 5, Lol p 11, Mus a 2, Mus a 3, Mus a 4, Mus a 5, Ory s 1, Ory s 12, Pas n 1, Pha a 1, Pha a 5, Phl p 1, Phl p 2, Phl p 4, Phl p 5, Phl p 6, Phl p 7, Phl p 11, Phl p 12, Phl p 13, Pho d 2, Poa p 1, Poa p 5, Sec c 1, Sec c 5, Sec c 20, Sor h 1, Tri a 14, Tri a 15, Tri a 18, Tri a 19, Tri a 21, Tri a 25, Tri a 26, Tri a 27, Tri a 28, Tri a 29, Tri a 30, Tri a 31, Tri a 32, Tri a 33, Tri a 34, Tri a 35, Tri a 36, Tri a 37, Zea m 1, Zea m 12, Zea m 14, Zea m 25, Act c 5, Act c 8, Act c 10, Act d 1, Act d 2, Act d 3, Act d 4, Act d 5, Act d 6, Act d 7, Act d 8, Act d 10, Act d 11, Aln g 1, Aln g 4, Amb a a 1, Amb a 2, Amb a 3, Amb a 4, Amb a 5, Amb a 6, Amb a 7, Amb a 9, Amb a 10, Amb p 5, Amb t 5, Ana o 1, Ana o 2, Ana o 3, Api g 1, Api g 2, Api g 3, Api g 5, Api g 6, Ara h 1, Ara h 2, Ara h 3, Ara h 4, Ara h 6, Ara h 7, Ara h 9, Ara h 10, Ara h 11, Art v 1, Art v 2, Art v 3, Art v 5, Art v 6, Ber e 1, Ber e 2, Beta v 1, Bet v 1, Bet v 3, Bet v 4, Bet v 6, Bet v 7, Bra j 1, Bra n 1, Bra o 3, Bra r 1, Bra r 2, Cap a 1w, Car b 1, Car i 1, Car i 4, Cas s 1, Cas s 5, Cas s 8, Cas s 9, Cat r 1, Che a 1, Che a 3, Cit l 3, Cit r 3, Cit s 1, Cit s 3, Cof a 1, Cor a 1, Cor a 8, Cor a 9, Cor a 10, Cor a 11, Cor a 12, Cor a 13, Cor a 14, Cuc m 1, Cuc m 3, Dau c 1, Fag e 2, Fag t 2, Fag s 1, Fra a 1, Fra a 3, Fra e 1, Gly m 1, Gly m 2, Gly m 4, Gly m 5, Gly m 6, Hel a 1, Hel a 3, Hev b 1, Hev b 2, Hev b 3, Hev b 4, Hev b 5, Hev b 6, Hev b 7, Hev b 9, Hev b 10, Hev b 11, Hev b 12, Hev b 13, Hev b 14, Hum j 1, Jug n 1, Jug n 2, Jug r 1, Jug r 2, Jug r 3, Jug r 4, Lac s 1, Len c 1, Len c 2, Len c 3, Lig v 1, Lup an 1, Lyc e 2, Lyc e 3, Lyc e 4, Mal d 1, Mal d 2, Mal d 3, Man e 5, Mer a 1, Mor n 3, Ole e 1, Ole e 3, Ole e 4, Ole e 5, Ole e 6, Ole e 7, Ole e 8, Ole e 9, Ole e 10, Ole e 11, Ost c 1, Par j 1, Par j 2, Par j 4, Par o 1, Pers a 1, Pha v 3, Pis v 1, Pis v 2, Pis v 3, Pis v 4, Pis v 5, Pis s 1, Pis s 2, Pla l 1, Pla a 1, Pla a 2, Pla or 1, Pla or 2, Pla or 3, Pru ar 1, Pru ar 3, Pru av 1, Pru av 2, Pru av 3, Pru d 3, Pru du 3, Pru du 5, Pru du 6, Pru p 1, Pru p 2, Pru p 3, Pyr c 1, Pyr c 3, Pyr c 5, Que a 1, Ric c 1, Rub i 1, Rub i 3, Sal k 1, Sal k 2, Sal k 3, Sal k 5, Ses i 1, Ses i 2, Ses i 3, Ses i 4, Ses i 5, Ses i 6, Ses i 7, Sin a 1, Sin a 2, Sin a 3, Sola t 1, Sola t 2, Sola t 3, Sola t 4, Syr v 1, Syr v 3, Vig r 1, Vit v 1 and Ziz m 1.

### Legends to Figures

Figure 1. Shows percentage of eosinophils isolated from BAL fluid of mice treated with Phl p 12 and challenged to OVA + Phl p 12 or OVA alone.
Figure 2. Same experiment as in Figure 1, but results shown with respect to in-vitro spleen cell proliferation upon addition of OVA.
Figure 3. Shows percentage of eosinophils isolated from BAL fluid of mice treated with Phl p 12 and challenged to Phl p extract containing Phl p 12 or Phl p extract depleted for Phl p 12.
Figure 4. Same experiment as in Figure 3, but results shown with respect to in-vitro stimulation of cervical lymph node cells upon addition of Phl p extract.
Figure 5. Same experiment as in Figure 3, but results shown with respect to levels of cytokine IL-5 in cervical lymph node cells upon addition of Phl p extract.
Figure 6. Shows percentage of eosinophils isolated from BAL fluid of mice sensitized to OVA, then treated with Phl p 12 and subsequently challenged to OVA + Phl p 12 or OVA alone.
Figure 7. Same experiment as in Figure 6, but results shown with respect to in-vitro spleen cell proliferation upon addition of OVA.
Figure 8. Shows levels of cytokine IL-5 in spleen cell culture, wherein spleen cells are isolated from mice treated with Phl p 12, sensitized to OVA and co-exposed to OVA + Bet v 2.
Figure 9. Same experiment as in Figure 8, but results shown with respect to levels of cytokine IL-13.
Figure 10. CIE diagram of extracts obtained at 20 sec (A), 60 sec (B) and at 2 min (C) extraction of raw pollen of *Phleum pratense.*
Figure 11. CIE diagram of extracts obtained at 5 min (A), 10 min (B) and 20 min (C) extraction of raw pollen of *Phleum pratense.*
Figure 12. RIE diagram of extracts obtained at 20 sec, 60 sec, 2 min, 5 min, 10 min and 20 min extraction of raw pollen of *Phleum pratense:* A) Phl p 12, B) Phl p 5), C) Phl p 1.
Figure 13. Amounts of Phl p 12, Phl p 1 and Phl p 5 released after 20 sec, 60 sec, 2 min, 5 min, 10 min and 20 min extraction of raw pollen of *Phleum pratense* and analysed by use of ELISA.
Figure 14. CIE diagram of extracts obtained at 20 sec (A) and 60 sec (B) and 2 min (C) extraction of defatted pollen of *Phleum pratense.*
Figure 15. CIE of extracts obtained at 5 min (A), 10 min (B) and 20 min (C) extraction of defatted pollen of *Phleum pratense.*
Figure 16. RIE of extracts obtained at 20 sec, 60 sec, 2 min, 5 min, 10 min and 20 min extraction of defatted pollen (*Phleum pratense*) A) Phl p 12, B) Phl p 5), C) Phl p 1.
Figure 17. Amounts of Phl p 12, Phl p 1 and Phl p 5 released at 20s, 60s, 2 min, 5 min, 10min and at 20 min extraction of defatted pollen of *Phleum pratense* and analysed by ELISA.
Figure 18. SDS PAGE of extracts obtained at 20 sec, 40 sec, 60 sec, 2, 5, 10 and 20 min extraction of A) raw and B) defatted pollen of *Phleum pratense,* respectively.
Figure 19. CIE of extracts obtained at 20 sec, 60 sec and 2 min extraction of defatted pollen of *Betula verrucosa.*
Figure 20. CIE of extracts obtained at 5 (A), 10 (B) and 20 (B) min extraction of defatted pollen of *Betula verrucosa.*
Figure 21. RIE of extracts obtained at 20 sec, 60 sec, 2 min, 5 min, 10 min and 20 min extraction of defatted pollen of *Betula verrucosa* A) Bet v 2 (profilin) and B) Bet v 1 (major allergen).
Figure 22. RIE of extracts obtained at 20 sec, 60 sec, 2 min, 5 min, 10 min and 20 min extraction of defatted pollen of *Ambrosia artemisiifolia* A) Amb a 8 (profilin) and B) Amb a 1 (major allergen).
Figure 23. RIE of extracts of pollen of *Ambrosia artemisiifolia (A), Betula verrucosa (B), Corylus avellana (C), and Phleum pratense (E) Artemisia vulgaris (F), Cryptomeria japonica (G), Humulus japonicus (I),* and extracts of mite bodies of *Dermatophagoides pteronyssinus* (D), extract of cat dander (H) and extract of *Malus domesticus* (apples) (freshly made) (J) where the plates are treated with polyclonal IgG antibodies raised against the profilin Bet v 2.
Figure 24. RIE of same extracts as figure 23, but RIE performed with polyclonal IgG antibodies raised against the profilin Phl p 2.
Figure 25. Shows in-vitro spleen cell proliferation upon stimulation with Bet v extract of spleen cells isolated from naïve mice SLIT treated with Phl p 12 and subsequently sensitized to bet v extract.
Figure 26. Same experiment as referred to in Figure 25, but shows the result of in-vitro stimulation of cervical lymph node cells upon stimulation with Bet v extract.
Figure 27. Shows in-vitro spleen cell proliferation upon stimulation with Bet v extract of spleen cells isolated from from mice sensitized to bet v extract and subsequently SLIT treated with Phl p 12.
Figure 28. Same experiment as referred to in Figure 27, but shows result of in-vitro stimulation of cervical lymph node cells upon stimulation with Bet v extract.
Figure 29. Shows levels of cytokine IL-5 released from in-vitro spleen cell proliferation upon stimulation with Phl p extract of spleen cells isolated from naïve mice SLIT treated with Ole e 2 and subsequently sensitized to Phl p extract.
Figure 30. Same experiment as referred to in Figure 29, but shows result of cytokine IL-5 release from in-vitro stimulation of cervical lymph node cells.
Figure 31. Shows in-vitro spleen cell proliferation upon stimulation with OVA of spleen cells isolated from naïve mice SLIT treated with Phl p 12 and subsequently sensitized to OVA.
Figure 32. Shows T-cell activation induced by profilin molecules evaluated by H3-thymidine incorporation (counts per minute, CPM, X-axis). A and B are T-cell lines established from two grass allergic patients. a: rPhl p 12, b: nPhl p 12, g: rOle e 2, d: medium control. Error bars indicate standard deviations of four replicates.

### Detailed Description

### Definitions

The following terms and phrases shall have the following meaning;

The term "a" or "an" refers to an indefinite number and shall not only be interpreted as "one" but also be interpreted in the meaning "some" or "several".

The terms "protein", "polypeptide" and "amino acid sequence" are used interchangeably herein. The conventional one-letter and three-letter codes for amino acid residues are used herein. The three-letter codes for amino acids are defined in conformity with the IUPAC-IUB Joint Commission on Biochemical Nomenclature (JCBN).

The term "variant" or "variants" refers to polypeptides which contain modifications/mutations compared to the "parent sequence". The term "variant" may be used interchangeably with the term "mutant".

The term "parent sequence" or "parent polypeptide" is meant to define a polypeptide on which any of the variant polypeptides are based. Unless otherwise mentioned, SEQ ID NO: 1 or alternatively SEQ ID NOs: 2-42, or alternatively any profilin mentioned in Tables 1 to 4, is to be considered the parent sequence. It should be understood that SEQ ID NOs: 1-42 are shown with a methionine in position 1, which in some embodiments may not be present as methionine may be removed enzymatically, either naturally by the host cell under recombinant production or ex-vivo. It should also be understood that the methionine may not be completely removed, such that about 2%-10% of recombinantly produced polypeptides contain methionine in position 1. A "parent nucleic acid" means a nucleic acid sequence encoding the parent polypeptide.

The term "sequence identity" is meant to define the fraction of amino acids that is the same between a pair of sequences upon alignment of the sequences. Sequence alignments and comparisons can be conducted by eye, but usually they are conducted with the aid of readily available sequence comparison computer programs and the output is a percentage identity calculated by the computer program.

The term "sequence similarity" is meant to define the fraction of amino acids which is the same or homologous between a pair of sequences upon alignment of the sequences. Most often a computer program is used for the calculation and the output is a score which is calculated using some similarity matrix forming part of the computer program.

The phrase "an allergen" shall be interpreted as one or more allergen(s).

The term "allergen" is meant to designate a proteineaous substance capable of eliciting a hypersensitivity immune response in an individual, such as in an animal, such as in a human. The allergen may be a sensitizing allergen or a cross-reacting allergen.

The term "sensitizing allergen" is meant to designate a proteineaous substance capable of triggering the immune system to produce IgE antibodies in an individual.

The term "cross-reacting allergen" defines a proteineaous substance that can be recognized by IgE antibodies originally created against a sensitizing allergen. That is to say that a cross-reacting allergen is an allergen capable of eliciting a hypersensitivity immune response when recognized and bound to cross-reactive IgE antibodies induced by a sensitizing allergen. For example the allergen Mal d 1, found in apples, is a protein homologous to Bet v 1, found in birch tree pollen, and is able to elicit a hypersensitivity immune response in an individual sensitized to Bet v 1 allergen due to its ability to trigger cross-binding and induce mast cell granulation in mast cells loaded with IgE anti-Bet v 1.

The term "major allergen" shall mean an allergen that causes sensitization in more than 50% of an average population (randomly selected) of patients having a hypersensitivity immune response triggered by exposure to an allergen source material comprising said allergen.

The term "minor allergen" shall mean an allergen that causes sensitization in less than 50% of an average population (randomly selected) of patients having a hypersensitivity immune response triggered by exposure to an allergen source material comprising said allergen.

The term "bystander suppression" or "bystander tolerance" is generally meant to encompass the ability to suppress an immune reaction in an individual towards one antigen (A) by treatment of the individual with another unrelated antigen (B).

The term "immunotherapy" is meant to encompass therapy, wherein the therapeutically active agent (herein the polypeptide) is an antigen (antigenic protein) or an immunogenic peptide. Immunotherapy usually encompass repeatedly administration of a sufficient dose of the antigen, usually in microgram quantities, over a prolonged period of time, usually for months or for years, wherein the antigen or immunogenic peptide is administered daily, several times a week, weekly, bi-weekly, or monthly.

The term "an individual" is meant to designate a mammal having an adaptive immune system, such as a human, a domestic animal such as a dog, a cat, a horse or cattle.

The phrase "an individual in need of thereof" is meant to encompass an individual having a hypersensitivity immune response, an individual sensitized to an allergen as well as an individual at risk of being sensitized to an allergen and at risk of developing a hypersensitivity immune response. The individual may present clinically symptoms of a hypersensitivity immune response or the individual may only be sensitized to an allergen and not yet presenting clinically symptoms of a hypersensitivity immune response. An individual in risk of being sensitized to an allergen may be identified due to atopic diseases in the family.

The phrase "prophylactic treatment" is meant to encompass treatment, such as by immunotherapy, of an individual with the aim to induce a response which will partly or completely prevent the individual from developing a hypersensitivity immune response. Prophylactic treatment, such as prophylactic immunotherapy, is therefore initiated before the individual becomes sensitized to an allergen. This may be realized by initiating immunotherapy before the individual has raised detectable serum IgE antibodies capable of binding specifically to the sensitizing allergen or before any other biochemical marker indicative of a hypersensitivity immune response can be detected in biological samples isolated from the individual. Furthermore, prophylactic immunotherapy shall also designate immunotherapy initiated before the individual has evolved clinical symptoms of the disease, such as symptoms of allergic rhinitis, allergic asthma or atopic dermatitis.

The term "treatment" refers to any type of treatment or prevention that conveys a benefit to a subject afflicted with or at risk of developing a hypersensitivity immune response to an allergen of interest, including improvement in the condition of the subject (e.g. in one or more symptoms), delay in the onset of symptoms, slowing the progression of symptoms, or induce disease modification etc. As used herein, "treatment" is not necessarily meant to imply cure or complete abolition of symptoms, but refers to any type of treatment that imparts a benefit to a patient.

The phrase "therapeutically effective amount" is meant to designate an amount effective to treat, such as an amount sufficient to achieve the desirable effect. For example, a therapeutically effective amount is the accumulated dose of an unrelated antigen administered during a course of immunotherapy in order to achieve the intended efficacy or the maximal dose tolerated within a give period. The total dose may be divided into single doses administered daily, twice a week or more, weekly, every second or fourth week or monthly depending on the route of administration. The total dose may be administered in different concentrations. It is expected that a single dose is in the microgram range, such as in the range of 5 to 500 microgram dependent on the antigen.

The phrase "sensitized to an allergen" is generally meant to encompass that the individual has been exposed to an allergen in a manner that the individual's adaptive immune system displays memory to the allergen, such as has raised detectable IgE antibodies against the allergen or that T-cells stimulated in-vitro are able to proliferate under the presence of the sensitizing allergen.

The term "adjuvant" refers to a substance that enhances the immune response to an antigen. Depending on the nature of the adjuvant, it can promote either a cell-mediated immune response, humoral immune response or a mixture of the two.

### Treatment/Prevention of a Hypersensitivity Immune Response

It should be understood that a polypeptide of the invention is for use in the treatment/prevention of a hypersensitivity immune response.A hypersensitivity immune response of the present invention is considered to be associated with an allergic disease/allergic immune response, such as typically a type 1 or a type 4 hypersensitivity immune response or mixes thereof, such as typically an immune response usually associated with the production of IgE antibodies, such as IgE mediated allergy. Typical examples of diseases mediated by a hypersensitivity immune response are, but not limited to, allergic diseases like atopic dermatitis, urticaria, contact dermatitis, allergic conjunctivitis, allergic rhinitis, allergic asthma, anapylaxis, food allergy and drug allergy. Typical examples of diseases mediated by a type 1 hypersensitivity immune reaction are, but not limited to, allergic diseases like atopic dermatitis, allergic conjunctivitis, allergic rhinitis, allergic asthma, anaphylaxis, food allergy and hay fever. An example of a disease mediated by a type 4 hypersensitivity immune reaction is contact dermatitis.

The polypeptide may be administered by any route of administration, but preferably administered to a mucosa or epithelia, e.g. administered to a mucosa and/or epithelia of the respiratory tract, gastroinstestinal tract and oral cavity. In principle, the administration to skin, such as by subcutaneous administration, epicutaneous administration or transdermal administration, is within the scope of administering a polypeptide of the invention and may be used in some embodiments. However, in other embodiments of the invention, the polypeptide is preferably not administered topically to skin, by transdermal means or by injection, such as subcutaneously and epicutaneously.

Following administration of the polypeptide to an epithelia and/or mucosa, the polypeptide may be taken up by an antigen presenting cell and in the absence of "danger signals" as observed with allergens, the default immunological event taking place at mucosal surfaces under physiological conditions will lead to the phenomen termed "oral tolerance" that is a normal feature of the mucosal immune system, as it can be generated through oral (including peroral), nasal, airway and sublingual administration of an antigen.

The mechanisms behind the induction of oral tolerance are still not completely understood, exept that it is widely acknowledged that orally administered antigens result in the generation of antigen-specific regulatory T-cells following presentation of the antigen by gutassociated APCs. Such presentation would preferentially induce T-cells that secrete regulatory cytokines such as TGF-beta and IL-10. These antigen-specific regulatory cells may migrate to lymphoid organs, suppressing immune responses by inhibiting the generation of effector cells, and to target organs, suppressing disease by releasing antigen-non-specific cytokines. It has recently been suggested that generation of regulatory cells in the mucosal immune system is mediated by a specialized subset of dendritic cells expressing the surface marker CD103. Oral administration of antigen has also been suggested to induce unresponsiveness of T-cell function primarily via anergy or deletion. It is considered that the mechanisms, induction of Treg cells, anergy and deletion may not exclude each other and that they may overlap considerably.

Thus, it should be understood that administering an antigen to the oral mucosa to an individual that is preferably not pre-sensitized to the antigen may induce antigen-specific Treg cells that become activated upon the following exposure to the antigen and start production of non-specific regulatory cytokines, and if the antigen is co-exposed with an allergen, the non-specific regulatory cytokines may suppress immune response towards other specificities that are ongoing in the microenvironment, such as a hypersensitivity immune response.

Thus, in order to induce oral tolerance, it should be understood that a polypeptide of the invention, is preferably administered to a mucosa of the airways, oral cavity and gastrointestinal tract. Thus, in preferred embodiments of the invention, the polypeptide is administered by inhalation, nasal administration, buccal administration (administration to cheeks or mouth cavity), oral administration (administration to the gastro-intestinal tract), such as preferably by sublingual administration. It is anticipated that the administration to a mucosa of the oral cavity may be performed by topically delivering the antigen at the surface of an epithelia of the oral cavity from where the antigen is absorbed into the mucosa and sub mucosa. For example, the antigen is administered to the epithelia of the sublingual part, including the floor of the mouth, from where the antigen is absorbed into the mucosa and sub mucosa. By buccal administration, it is the intention to apply a suitable pharmaceutical formulation of the polypeptide on a epithelia of the oral cavity, such as on a epithelia located at the lower or upper surface of the tongue, at the gum or at the cheeks, so as to allow the polypeptide to be absorbed into the mucosa and submucosa and wherein it is not the intention to deliver the polypeptide to the gastro-intestinal tract. Thus, in more preferable embodiments of the invention, the antigen is administered to the epithelia and/or mucosa of the oral cavity, such as by sublingual administration, preferably wherein the individual is instructed not to swallow before a period of at least 1 minute after the administration, such as more preferably after 2 minutes, 3 minutes, 4 minutes, or 5 minutes of administration of the polypeptide.

The polypeptide may be administered repeatedly in sufficient doses, usually in single doses of microgram quantities, over a prolonged period of time, usually for weeks, months or for years before the effect is achieved. Usually, the polypeptide is administered daily, weekly, biweekly, or monthly - dependent on the route of administration, formulation and/or the specific polypeptide. It is expected that the polypeptide should be administered, continuously or interrupted by one or more periods of no administration, during a period of a least 4 weeks to about 4-6 months, but that administration must be continued for several months or years.

Notably, it should be understood that achievement of bystander tolerance most likely requires that oral tolerance and/or specific Treg cells is/are created. Oral tolerance will, at least, be created by administering the polypeptide to an oral mucosa. However, biomarkers showing that oral tolerance (T-cell tolerance) has been achieved are not easily identified. Tolerance in sensitized allergic patients is usually linked to IgG4 production and production of the cytokines IL-10 and IL-4. However, it is believed that direct examination of T-cell responses towards the tolerogenic antigen may result in Treg cells producing IL-10 and/or TGF-b, while complete non-responsiveness or Th1/Th2 responses should be observed in the placebo group. To further substantiate this, in-vitro studies of simultaneous stimulation of Treg cells with the tolerogenic polypeptide and a test antigen, e.g. an allergen, should lead to suppression of the test antigen antigen-specific T-cell response. As an alternative, simultaneous skin challenge with the tolerogenic polypeptide and a test antigen like tetanus toxoid (TT) might mimic the in vitro stimulation and include all the immunological elements of an in vivo situation. In this situation, the lack of a delayed type hypersensitivity response towards TT in the presence of the tolerogenic antigen would be expected in the actively treated group while TT stimulation without the tolerogenic antigen should lead to a skin response.

Furthermore, achievement of bystander tolerance also requires that the same polypeptide, or alternatively a modification of the polypeptide, is present together with the non-profilin allergen at the target organ (e.g. respiratory tract) that is subject to the exposure of the profilin-containing plant material. Therefore, in addition to administering the polypeptide with the aim of achieving oral tolerance, means for providing co-exposure of the polypeptide or a modified polypeptide thereof and the non-profilin allergen are also essential for achieving bystander tolerance.

By the term "co-exposure" is meant that the polypeptide is presented/made available to the same organ as targeted by the non-profilin allergen during natural exposure to the non-profilin-containing plant material, such as presented/made available to the same target organ within a period at least coinciding partly or entirely with the individual's exposure to said non-profilin allergen. Typically, the target organ of an aero allergen is the respiratory tract, the target organ of a food allergen is the gastro-intestinal tract or oral cavity and aero allergens, food allergens and contact allergens target the skin.

Thus, in addition to administering the polypeptide repeatedly to the respiratory tract, gastroinstestinal tract and/or oral cavity (so as to achieve oral tolerance/Treg cells induction), means for ensuring co-presence/co-exposure of the polypeptide and the non-profilin allergen at the taget organ in a period partly or entirely overlapping with the period of allergen exposure should be provided.

The present inventors have now found that co-exposure may be provided by selecting as the "tolerance-inducing antigen" an antigen naturally exposed to the same target organ as the allergen triggering the hypersensitivity immune response. As such the "tolerance-inducing antigen" inherently reaches the same target organ as the "triggering" non-profilin allergen upon the natural exposure to the profilin-containing plant material. Advantageously, there is no need of providing additional means for ensuring co-exposure to the triggering allergen (non-profilin allergen) and the "tolerance-inducing antigen" (polypeptide).

According to the present invention, the bystander tolerance is achieved by administering a polypeptide, such as the profilin Phl p 12, to an individual in need of being treated for a hypersensitivity immune response caused by a non-profilin allergen of a profilin-containing plant material. It is considered that bystander tolerance is, at least, achieved where the polypeptide has an amino acid sequence having at least 60%, such as at least 65%, 70%, 75%, 80%, 85%, 90% or 95% similarity and/or identity to the amino acid sequence of the profilin of the profilin-containing plant material. That is to say that the polypeptide need not be identical to the profilin of the profilin-containing plant material as long as a certain identity and/or similarity exists between the polypeptide as administered and the profilin as naturally exposed to.

However, where the polypeptide of the invention for some reasons does not provide sufficient bystander tolerance of a hypersensitivity immune response, the polypeptide may also be administered to the target organ subject to the natural exposure of a profilin-containing plant material, such as administered to the respiratory tract, gastro-intestinal tract, or skin within a period at least coinciding partly or entirely with the individual's natural exposure to said profilin-containing plant material.

The phrase "the polypeptide" is also administered to the target organ subject to the natural exposure of a profilin-containing plant material within a period at least coinciding partly or entirely with the individual's natural exposure to said profilin-containing plant material" is meant to designate that the polypeptide is administered to the target organ at least during the entire period of allergen exposure or it may be administered in a part of that period including a period before start of the natural exposure.

It is envisaged that in some embodiments of the invention, the polypeptide is administered to an epithelia/mucosa of the respiratory tract (preferably nasal administration), oral cavity (preferably sublingual administration) or gastro-intestinal tract with the aim of achieving oral tolerance to that polypeptide. In addition, and preferably after oral tolerance has been achieved to the polypeptide, the same polypeptide or alternatively a modified polypeptide thereof, is administered to the target organ subject to the natural exposure to the profilin-containing plant material simultaneously, contemporaneously, separately or sequentially, in either order, to the period of natural exposure to the profilin-containing plant material.

For example, wherein the target organ is the respiratory tract, as is most relevant for aero allergens (pollen), the polypeptide is repeatedly administered to the oral cavity (e.g.sublingually) daily or weekly in a period ranging from 4 weeks to 12 months or more (so as to achieve oral tolerance/Treg cells), and the same polypeptide or a modified polypeptide thereof is administered to the respiratory tract, such as by nasal administration or by inhalation, in a period entirely or partly overlapping with the period of the natural exposure.

Likewise, where the target organ is the gastro-intestinal tract, as is most relevant for food allergens, the polypeptide is repeatedly administered (e.g.sublingually), such as daily or weekly administrered in a period ranging from 4 weeks to 12 months or more, and the same polypeptide or a modified polypeptide thereof (variant thereof) is administered to the gastrointestinal tract, such as by ingestion or oral administration, in a period entirely or partly overlapping with the period of the natural exposure.

### Individuals

As it is expected that oral tolerance induction is facilitated by the lack of allergic sensitization towards the polypeptide, the invididual in need of the present treatment is preferably not sensitized to a profilin of said profilin-containing plant material. That is to say that the hypersensitivity immune response is preferably not caused by a profilin of said profilin-containing plant material. Moreover, where the polypeptide is not identical to the profilin of the profilin-containing plant material, the individual is preferably not sensitized to the polypeptide.

Thus, in preferred embodiments of the invention, the individual is not, at least not before administering the first dose, sensitized to a profilin of said profilin-containing plant material and/or said polypeptide, such as neither sensitized, at least not before administering the first dose, to a profilin of said profilin-containing plant material nor to said polypeptide.

Thus, it may be determined whether the profilin of the profilin-containing plant material and/or said polypeptide binds to or otherwise associates with IgE antibodies (e.g. serum IgE antibodies) obtained from the individual to be treated. Several assays can be applied for this purpose, each having various limits of detection and quantification. Therefore, it may be understood that the profilin of the profilin-containing plant material and/or said polypeptide does not bind to or otherwise associate with IgE antibodies (e.g. serum IgE antibodies) in detectable levels and/or quantifiable levels.

The presence of specific IgE antibodies toward the profilin and/or the polypeptide in an individual may be tested by well known methods in the art, such as by use of the RAST test that is a radioimmunoassay test to detect specific IgE antibodies to suspected or known allergens. In those tests, the suspected allergen is bound to an insoluble material and the patient's serum or other body fluids containing IgE (e.g. saliva) is added. If the serum/body fluids contain antibodies to the allergen, those antibodies will bind or otherwise associate to the allergen. Radiolabeled anti-human IgE antibody is added where it binds or otherwise associates to those IgE antibodies already bound to the insoluble material. The unbound anti-human IgE antibodies are washed away. The amount of radioactivity is proportional to the serum IgE for the allergen. In recent years, a more superior test named "ImmunoCAP Specific IgE blood test" is applied for the same purpose, which in the literature may also be described as: CAP RAST, CAP FEIA (fluorenzymeimmunoassay), and Pharmacia CAP. The quantitative detection limit of such tests may be as low as about 0.1 kU/l. For use in the context of the present invention, the quantitative detection limit may be below 1 kU/l, such as below 0.5 kU/l, such as below 0.3 kU/l where the ImmunoCAP® Specific IgE blood test or a comparable test is used.

Obviously, the profilin and/or the polypeptide may not induce a hypersensitivity immune reaction in the individual in need of treatment. For example, the profilin and/or the polypeptide does not initiate, at least not before administering the first dose of polypeptide, an immediate skin reaction and/or delayed skin reaction in the individual in need of treatment upon conducting skin prick testing with various concentrations of the profilin/polypeptide.

Alternatively, it can be ascertained whether the profilin and/or polypeptide induces histamine release in an in-vitro basophil/mast cell assay using blood from the individual to be treated.

Still more alternatively, it may be ascertained that the profilin and/or the polypeptide does not bind to or otherwise associate with, at least not in detectable levels, to IgG antibodies, e.g. rabbit anti IgG antibodies, raised after immunization of an animal, such as a rabbit, with one or more or of the non-profilin allergen(s) of the profilin-containing material or with an aqueous extract of the profilin-containing material.

It should be understood that an individual in need of the present treatment, may be an individual already sensitized to a non-profilin allergen of a profilin-containing material. Such an individual may present clinically symptoms of a hypersensitivity immune response or the individual may only be sensitized to the non-profilin allergen and not yet presenting clinically symptoms of a hypersensitivity immune response. Furthermore, it should be understood that an individual may benefit from this treatment even when not sensitized to the non-profilin allergen. Such an individual may be an individual in risk of being sensitized to a non-profilin allergen, such as in risk of developing a hypersensitivity immune response to the non-profilin allergen. An individual in risk of being sensitized to an allergen may be identified due to family histories of atopic diseases.

Thus, the present invention also relates to "prophylactic treatment" that is initiated before the individual has raised detectable serum IgE antibodies to a non-profilin allergen of the profilin-containing material or initiated before any other biochemical marker indicative of a hypersensitivity immune response can be detected in biological samples isolated from the individual. Furthermore, prophylactic immunotherapy may also include treatment initiated before the individual has developed clinical symptoms of the disease, such as symptoms of allergic rhinitis or allergic asthma.

As mentioned, the polypeptide of the invention may be used in the treatment of a hypersensitivity immune response. The dosage regimen of relevance may be one usually applied in the field of allergen specific immunotherapy, for example, in terms of selecting doses, number of doses per day, duration of treatment and frequency of administration. Thus, it may be envisaged that the polypeptide be administered frequently during a longer period before the desirable effect is achieved, such as administered daily to the mucosa of the oral cavity (e.g. sublingual mucosa) for a period of at least 2-6 months. It is also envisaged that where the hypersensitivity immune response is caused by a seasonal allergen, the first dose may be administered before the allergen season. It is also envisaged that the treatment is initiated by an up-dosing phase where the polypeptide is administered in increasing doses during one day or with days or weeks between until a maintenance dose is achieved.

It should be understood that the polypeptide is preferably the only therapeutically active ingredient being administered. In particular, it is emphasized that the polypeptide should be administered in the absence of allergens to avoid competing immunological mechanisms, production of danger signals and/or inflammation. Thus, the non-profilin allergen to which the individual has specific IgE antibodies shall not be co-administered with the polypeptide, at least not within the period of establishing oral tolerance to the polypeptide. It should also be understood that the polypeptide is not administered together with a modulator of the Notch signalling pathway either as a combined preparation for simultaneous, contemporaneous, separate or sequential use for modulation of the immune system as described in the patent application WO 2004/082710.

### Polypeptides, Non-profilin Allergens and Profilin-Containing Plant Materials

A polypeptide for use in the present invention may be the profilin (Phl p 12) of the plant species *Phleum Pratense,* which exists in various isoforms such as those having an amino acid sequence of SEQ ID NOs: 1-10, optionally wherein methionine is not present in position 1. Notably, this profilin is inherently presented concomitantly with a non-profilin allergen, such as Phl p 1, 5 or 6, when the individual is exposed to grass pollen of the plant order *Poales* during the grass pollen season.

It should be understood that polypeptides even differing from the amino acid sequence of profilin Phl p 12 may replace Phl p 12 as a usable bystander antigen in suppressing a hypersensitivity immune response caused by a non-profilin allergen of grass pollen. For example, profilins homologous to Phl p 12, such as the profilin of birch tree pollen, Bet v 2, or the profilin of olive tree pollen, Ole e 2, may be used in suppressing a hypersensitivity immune response caused by a non-profilin allergen of grass pollen of the plant order *Poales.* It is also envisaged that due to high molecular similarity, such as at least 60% identity between amino acid sequences of Phl p 12 and Bet v 2 or Ole e 2, there is established sufficient immunological cross-reactivity between Phl p 12 and Bet v 2 or Ole e 2 so that treatment with Phl p 12 leads to a tolerance response which may become activated by the homologous polypeptide Bet v 2 or Ole e 2 following exposure to birch tree pollen or olive tree pollen, leading to concomitant down-regulation of a hypersensitivity immune response caused by a non-profilin allergen of birch and/or grass pollen. Likewise, the treatment of an individual with the profilin Bet v 2 may lead to a tolerance response which may become activated by exposure to the homologous polypeptide Phl p 12 or Ole e 2 following exposure to grass or olive tree pollen, respectively, leading to concomitant down-regulation of a hypersensitivity immune response caused by a non-profilin allergen of birch tree pollen and/or grass pollen and/or olive tree pollen.

Therefore, a polypeptide for use in the present invention has, consists of, consists essentially of, comprises an amino acid sequence having at least 60% identity and/or similarity, such as of at least 70%, 75, 80, 85, 90, 95 and 98% identity and/or similarity, to an amino acid sequence shown in SEQ ID NOs: 1, 2, 3, 4, 5, 6, 7, 8, 9 or 10, optionally without methionine at position 1 (N-terminal end).

Typical examples on polypeptides having an amino acid sequence of at least 60% identity and/or similarity to the amino acid sequence shown in SEQ ID NOs: 1, 2, 3, 4, 5, 6, 7, 8, 9 or 10 are profilins of other profilin-containing plant materials.

Tables 1-4 show exemplary profilins of a profilin-containing plant material (as identified by their UniProt Number, extracted from http\:uniprot.org and their percentage identity to Phl p 12 (SEQ ID NO: 1) wherein the percent identity in amino acid sequence has been determined using BLAST Alignment (Database= UniProtKB, Treshold=10, Matrix=auto, Filtering = None, Gapped=500, Hits=500). There is a notably high degree of identity between profilins of various plant species, such as at least 60% identity between the amino acid sequences of SEQ ID NO: 1 and the profilins shown in Table 4. In particular, the percentage identity within the group of grasses and grains (order *Poales,* e.g. genus *Cynodon* (Bermuda grass), *Hordeum* (barley), *Oryza* (rice), *Phleum Pratense* (Timoty grass), *Sorghum* (grass), *Triticum* (durum or wheat) and *Zea* (Mais) belonging to the plant class *Monocots* are high, such as above 80%. Furthermore, profilins of trees (tree pollen) has high identity to profilin Phl p 12, such as within 70% to 82% (Order *Fagales,* e.g. genus *Betula* (birch), genus *Corylus* (hazel); order *Arecales,* e.g. genus *Elaeis* (palm tree); order *Malvales,* e.g. genus *Gossypium* (cotton tree); order *Malpighiales,* e.g. genus *Hevea* (latex tree), order *Mercuriales,* e.g. genus *Ricinus;* order *Lamiales,* e.g. genus *Olea* (olive tree), genus *Populus* (poplar tree), order *Myrtales,* e.g. genus *Sonneratia.* Profilins of weed pollen has a percentage identity within 75 to 81% (order *Asterales,* e.g. genus *Ambrosia* (short ragweed), genus *Artemisia* of genus *Helianthus* (sunflower).

Also fruits, nuts and legumes of the order *Apiales, Fabales, Rosales, Solanales* and *Vitales* has a high percentage identity to Phl p 12, such as within 70% to 82% (order *Apiales,* e.g. genus *Apium* (celery), genus *Daucus* (carrot), genus *Petroselinum* (parsley); order *Fabales,* e.g. genus *Arachis* (peanut); order *Rosales,* e.g. genus *Glycine* (soya bean), genus *Fragaria* (strawberry), genus *Humulus,* genus *Malus* (apple), genus *Parietaria,* genus *Prunus* (peach, apricot, cherry, almond), genus *Pyrus* (pear); order *Solanales,* e.g. genus *Capsicum* (chili pepper), genus *Nicotiana* (tobacco), genus *Lycopersicon* (tomato), genus *Solanum* (potato), order *Vitales,* e.g. genus *Vitis* (grape).

Other interesting profilins are of the order *Brassicales,* e.g. genus *Arabidopsis, Brassica* (rape), order *Caryoplyllales,* e.g. genus *Chenopodium,* order *Sapindales,* e.g. genus *Citrus* (orange), genus *Litchi;* order *Curcubitales,* e.g. genus *Cucumis* (cucumber, melon).

Thus, typical examples on profilin-containing plant materials having at least 80% identity to SEQ ID NO: 1 are from a plant genus selected from *Amaranthus, Ambrosia, Brassica, Chenopodium, Corylus, Cynodon, Elaeis, Helianthus, Hevea, Hordeum, Litchi, Mercurialis, Nicotiana, Olea, Oryza, Phleum, Phoenix, Solanum, Sonneratia, Sorghum, Triticum* and *Zea.*

Typical examples on profilin-containing plant materials having at least 70% identity to SEQ ID NO: 1 are from a plant genus selected from *Amaranthus, Ambrosia, Ananas, Apium, Arabidopsis, Arachis, Artemisia, Betula, Brassica, Capsicum, Caryota, Chenopodium, Citrus, Corylus, Cucumis, Cynodon, Elaeis, Fragaria, Glycine, Helianthus, Hevea, Hordeum, Humulus, Litchi, Malus, Mercurialis, Musa, Nicotiana, Olea, Oryza, Parieraria, Petroselinum, Phaseolus, Phleum, Phoenix, Picea, Pinus, Plantago, Populus, Prunus, Pyrus, Ricinus, Salsola, Solanum, Sonneratia, Sorghum, Triticum, Vitis* and *Zea.*

Thus, there seems to be a high conservation in the amino acid sequences of profilins of plant species of the plant classes *Plantae Pinopsida* (previously known as Coniferopsida), *Plantae Monocots* (also known as monocotyledons and previously named *Liliopsida*) and *Plantae Magnoliopsida.*

Therefore, a polypeptide for use in the present invention may be a profilin of a profilin-containing plant material of a plant class selected from the group comprising *Plantae Pinopsida, Plantae Monocots* and *Plantae Magnoliopsida,* e.g. of a plant order, plant family, plant genus or plant species shown in Tables 1, 2 and 3. As described further below, a polypeptide of the invention may in addition to the natural occurring ones be a variant of a natural occurring profilin.

In accordance with the bystander tolerance concept of the present invention, it follows that the hypersensitivity immune response is triggered by a non-profilin allergen of a profilin-containing plant material that is either of the same plant species or of another plant species, plant genus, plant order or plant class as the profilin-containing plant material from which the profilin molecule is derived. Therefore, a hypersensitivity immune response is triggered by a non-profilin allergen of a profilin-containing plant material of a plant class selected from the group comprising *Plantae Pinopsida, Plantae Monocots* and *Plantae Magnoliopsida,* e.g. of a plant order, plant family, plant genus or plant species shown in Tables 1, 2 and 3.

Typical interesting examples of a profilin-containing plant material are of a plant order selected from the group comprising *Pinales, Arecales, Asparagales, Poales, Zingiberales Apiales, Asterales, Brassicalis, Curcurbitales, Ericales, Fabales, Fagales, Gentianales, Lamiales, Laurales, Malvales, Malpighiales, Myrtales, Proteales, Rosales, Sapindales, Solanales* and *Vitales,* preferably *Pinales, Poales, Arecales, Apiales, Asterales, Brassicalis, Caryophyllales, Fabales, Fagales, Lamiales, Malphighialis, Proteales, Rosales, Sapindales* and *Solanales,* more preferably, *Pinales, Arecales, Poales, Asterales, Brassicalis, Caryophyllales, Fagales, Lamiales, Rosales* and *solanales,* most preferably *Poales, Asterales, Fagales* and *Lamialis,* even more preferably *Poales.*

More specifically, a profilin-containing plant material is of a plant family selected from the group comprising *Cupressaceae,* Arecaceae, *Asparagaceae, Iridaceae, Bromeliaceae, Poaceae, Musaceae, Zingiberaceae, Apiaceae, Araliaceae, Asteraceae, Brassicaceae, Amaranthaceae, Caryophyllaceae, Polygonaceae, Cucurbitaceae, Actinidiaceae, Lecythidaceae, Theaceae, Fabaceae, Betulaceae, Fagaceae, Juglandaceae, Myricaceae, Nothofagaceae, Ticodendraceae, Apocynaceae, Rubiaceae, Oleaceae, Pedaliacae, Plantaginaceae, Lauraceae, Malvaceae, Euphorbiaceae, Lythraceae, Platanaceae, Cannabaceae, Rosaceae, Ulmaceae, Urticaceae, Anacardiaceae, Rutaceae, Sapindaceae, Solanaceae, Vitaceae,* preferably *Cupressaceae, Arecaceae, Bromeliaceae, Poaceae, Apiaceae, Asteraceae, Brassicaceae, Amaranthaceae, Caryophyllaceae, Fabaceae, Betulaceae, Fagaceae, Oleaceae, Euphorbiaceae, Platanaceae, Cannabaceae, Rosaceae* and *Solanaceae,* most preferably *Poaceae, Asteraceae, Betulaceae* and *Oleaceae.*

Even more specifically, a profilin-containing plant material is of a plant genus selected from the group comprising *Chamaecyparis, Cryptomeria, Cupressus, Juniperus, Phoenix, Asparagus, Crocus, Ananas, Anthoxanthum, Cynodon, Dactylis, Festuca, Holcus, Hordeum, Lolium, Oryza, Paspalum, Phalaris, Phleum, Poa, Secale, Sorghum, Triticum, Zea, Musa, Apium, Daucus, Ambrosia, Artemisia, Helianthus, Lactuca, Arabidopsis, Brassica, Sinapis, Amaranthus, Beta, Chenopodium, Fagopyrum, Salsola, Cucumis, Actinidia, Bertholletia, Arachis, Glycine, Lens, Lupinus, Phaseolus, Pisum, Vigna, Alnus, Betula, Carpinus, Carya, Castanea, Corylus, Fagus, Juglans, Ostrya, Quercus, Catharanthus, Coffea, Fraxinus, Ligustrum, Olea, Plantago, Sesamum, Syringa, Persea, Gossypium, Hevea, Manihot, Mercurialis, Popolus, Ricinus, Sonneratia, Platanus, Fragaria, Humulus, Malus, Morus, Parietaria, Prunus, Pyrus, Rubus, Ziziphus, Anacardium, Citrus, Litchi, Mangifera, Pistacia, Capsicum, Lycopersicon, Solanum* and *Vitis,* more preferably *Cryptomeria, Ananas, Anthoxanthum, Cynodon, Dactylis, Festuca, Holcus, Hordeum, Lolium, Oryza, Paspalum, Phalaris, Phleum, Poa, Secale, Sorghum, Triticum, Zea, Apium, Daucus, Ambrosia, Artemisia, Helianthus, Chenopodium, Salsola, Arachis, Glycine, Alnus, Betula, Carpinus, Carya, Castanea, Corylus, Fagus, Juglans, Ostrya, Quercus, Fraxinus, Olea, Plantago, Hevea, Mercurialis, Platanus, Humulus, Malus, Parietaria, Prunus, Pyrus, Rubus, Capsicum, Lycopersicon* and *Solanum,* even more preferably *Cryptomeria, Anthoxanthum, Cynodon, Dactylis, Festuca, Holcus, Hordeum, Lolium, Oryza, Phleum, Poa, Secale, Sorghum, Triticum, Zea, Ambrosia, Artemisia, Helianthus, Chenopodium, Salsola, Betula, Corylus, Olea, Parietaria* and *Solanum.*

Still more specifically, a profilin-containing plant material contains a non-profilin allergen selected from the group comprising Cha o 1, Cha o 2, Cry j 1, Cry j 2, Cup a 1, Cup s 1, Cup s 3, Jun a 1, Jun a 2, Jun a 3, Jun o 4, Jun s 1, Jun v 1, Jun v 3, Ana c 2, Ant o 1, Aspa o 1, Cro s 1, Cro s 2, Cyn d 1, Cyn d 7, Cyn d 15, Cyn d 22w, Cyn d 23, Cyn d 24, Dac g 1, Dac g 2, Dac g 3, Dac g 4, Dac g 5, Fes p 4, Hol l 1, Hol l 5, Hor v 1, Hor v 5, Hor v 12, Hor v 15, Hor v 16, Hor v 17, Hor v 21, Lol p 1, Lol p 2, Lol p 3, Lol p 4, Lol p 5, Lol p 11, Mus a 2, Mus a 3, Mus a 4, Mus a 5, Ory s 1, Ory s 12, Pas n 1, Pha a 1, Pha a 5, Phl p 1, Phl p 2, Phl p 4, Phl p 5, Phl p 6, Phl p 7, Phl p 11, Phl p 13, Pho d 2, Poa p 1, Poa p 5, Sec c 1, Sec c 5, Sec c 20, Sor h 1, Tri a 14, Tri a 15, Tri a 18, Tri a 19, Tri a 21, Tri a 25, Tri a 26, Tri a 27, Tri a 28, Tri a 29, Tri a 30, Tri a 31, Tri a 32, Tri a 33, Tri a 34, Tri a 35, Tri a 36, Tri a 37, Zea m 1, Zea m 12, Zea m 14, Zea m 25, Act c 5, Act c 8, Act c 10, Act d 1, Act d 2, Act d 3, Act d 4, Act d 5, Act d 6, Act d 7, Act d 8, Act d 10, Act d 11, Aln g 1, Aln g 4, Amb a 1, Amb a 2, Amb a 3, Amb a 4, Amb a 5, Amb a 6, Amb a 7, Amb a 9, Amb a 10, Amb p 5, Amb t 5, Ana o 1, Ana o 2, Ana o 3, Api g 1, Api g 2, Api g 3, Api g 5, Api g 6, Ara h 1, Ara h 2, Ara h 3, Ara h 4, Ara h 6, Ara h 7, Ara h 9, Ara h 10, Ara h 11, Art v 1, Art v 2, Art v 3, Art v 5, Art v 6, Ber e 1, Ber e 2, Beta v 1, Bet v 1, Bet v 3, Bet v 4, Bet v 6, Bet v 7, Bra j 1, Bra n 1, Bra o 3, Bra r 1, Bra r 2, Cap a 1 w, Car b 1, Car i 1, Car i 4, Cas s 1, Cas s 5, Cas s 8, Cas s 9, Cat r 1, Che a 1, Che a 3, Cit l 3, Cit r 3, Cit s 1, Cit s 3, Cof a 1, Cor a 1, Cor a 8, Cor a 9, Cor a 10, Cor a 11, Cor a 12, Cor a 13, Cor a 14, Cuc m 1, Cuc m 3, Dau c 1, Fag e 2, Fag t 2, Fag s 1, Fra a 1, Fra a 3, Fra e 1, Gly m 1, Gly m 2, Gly m 4, Gly m 5, Gly m 6, Hel a 1, Hel a 3, Hev b 1, Hev b 2, Hev b 3, Hev b 4, Hev b 5, Hev b 6, Hev b 7, Hev b 9, Hev b 10, Hev b 11, Hev b 12, Hev b 13, Hev b 14, Hum j 1, Jug n 1, Jug n 2, Jug r 1, Jug r 2, Jug r 3, Jug r 4, Lac s 1, Len c 1, Len c 2, Len c 3, Lig v 1, Lup an 1, Lyc e 2, Lyc e 3, Lyc e 4, Mal d 1, Mal d 2, Mal d 3, Man e 5, Mer a 1, Mor n 3, Ole e 1, Ole e 3, Ole e 4, Ole e 5, Ole e 6, Ole e 7, Ole e 8, Ole e 9, Ole e 10, Ole e 11, Ost c 1, Par j 1, Par j 2, Par j 4, Par o 1, Pers a 1, Pha v 3, Pis v 1, Pis v 2, Pis v 3, Pis v 4, Pis v 5, Pis s 1, Pis s 2, Pla l 1, Pla a 1, Pla a 2, Pla or 1, Pla or 2, Pla or 3, Pru ar 1, Pru ar 3, Pru av 1, Pru av 2, Pru av 3, Pru d 3, Pru du 3, Pru du 5, Pru du 6, Pru p 1, Pru p 2, Pru p 3, Pyr c 1, Pyr c 3, Pyr c 5, Que a 1, Ric c 1, Rub i 1, Rub i 3, Sal k 1, Sal k 2, Sal k 3, Sal k 5, Ses i 1, Ses i 2, Ses i 3, Ses i 4, Ses i 5, Ses i 6, Ses i 7, Sin a 1, Sin a 2, Sin a 3, Sola t 1, Sola t 2, Sola t 3, Sola t 4, Syr v 1, Syr v 3, Vig r 1, Vit v 1 and Ziz m 1.

The non-profilin allergen preferably is a major allergen, such as selected from the group comprising Cha o 1, Cry j 1, Cry j 2, Cup a 1, Cup s 1, Jun a 1, Jun s 1, Jun v 1, Ana c 1, Ant o 1, Aspa o 1, Cro s 1, Cyn d 1, Dac g 1, Dac g 5, Fes p 4, Hol l 1, Hol l 5, Hor v 1, Hor v 5, Hor v 12, Hor v 15, Hor v 16, Hor v 17, Hor v 21, Lol p 1, Lol p 5, Mus a 1, Ory s 1, Pas n 1, Pha a 1, Pha a 5, Phl p 1, Phl p 5, Phl p 6, Pho d 2, Poa p 1, Poa p 5, Sec c 1, Sec c 5, Sor h 1, Tri a 12, Zea m 1, Zea m 12, Zea m 14, Zea m 25, Act c 5, Act d 1, Act d 2, Aln g 1, Aln g 4, Ama r 2, Amb a 1, Amb a 2, Amb p 5, Amb t 5, Ana o 1, Ana o 2, Api g 1, Api g 5, Ara h 1, Art v 1, Ber e 1, Beta v 1, Bet v 1, Bra j 1, Bra n 1, Bra o 3, Bra r 1, Cap a 1w, Cap a 2, Car b 1, Car i 1, Cas s 1, Cat r 1, Che a 1, Cit l 3, Cit r 3, Cit s 1, Cof a 1, Cor a 1, Cor a 2, Cuc m 1, Dau c 1, Fag e 2, Fag t 2, Fag s 1, Fra a 1, Fra e 1, Gly m 1, Gly m 2, Hel a 1, Hel a 3, Hev b 1, Hum j 1, Jug n 1, Jug r 1, Lac s 1, Len c 1, Lig v 1, Lit c 1, Lup an 1, Lyc e 1, Lyc e 2, Mal d 1, Ole e 1, Ole e 2, Ost c 1, Par j 1, Par o 1, Pers a 1, Pha v 3, Pis v 1, Pis s 1, Pla l 1, Pla a 1, Pla a 2, Pla or 1, Pla or 2, Pru ar 1, Pru av 1, Pru p 1, Pyr c 1, Que a 1, Ric c 1, Rub i 1, Sal k 1, Ses i 1, Sin a 1, Sola t 1, Syr v 1, Vig r 1, Vit v 1 and Ziz m 1, such as preferably Cry j 1, Cry j 2, Cyn d 1, Dac g 1, Dac g 5, Fes p 4, Hol l 1, Hol l 5, Hor v 1, Hor v 5, Hor v 12, Hor v 15, Hor v 16, Hor v 17, Hor v 21, Lol p 1, Lol p 5, Ory s 1, Pas n 1, Pha a 1, Pha a 5, Phl p 1, Phl p 5, Phl p 6, Poa p 1, Poa p 5, Sec c 1, Sec c 5, Sor h 1, Tri a 12, Zea m 1, Zea m 12, Zea m 14, Zea m 25, Aln g 1, Aln g 4, Amb a 1, Amb a 2, Amb p 5, Amb t 5, Api g 1, Api g 5, Ara h 1, Art v 1, Bet v 1, Bra j 1, Bra n 1, Bra o 3, Bra r 1, Cap a 1w, Cap a 2, Car b 1, Car i 1, Cas s 1, Cat r 1, Che a 1, Cor a 1, Cor a 2, Cuc m 1, Dau c 1, Gly m 1, Gly m 2, Hel a 1, Hel a 3, Hev b 1, Hum j 1, Lit c 1, Lyc e 1, Lyc e 2, Mal d 1, Ole e 1, Ole e 2, Par j 1, Par o 1, Pla l 1, Pla a 1, Pla a 2, Pla or 1, Pla or 2, Pru ar 1, Pru av 1, Pru p 1, Pyr c 1, Que a 1, Ric c 1, Rub i 1, Sal k 1, Sin a 1 and Sola t 1.

Typically, the profilin-containing plant material is pollen, such as grass, grain, tree or weed pollen, but may also be food or a food ingredient, like a cereal, a nut, a fruit or a vegetable.

In some preferable embodiments of the invention, the non-profilin allergen is from a pollen of a plant genus selected from the group comprising *Cryptomeria, Anthoxanthum, Cynodon, Dactylis, Festuca, Holcus, Hordeum, Lolium, Oryza, Paspalum, Phalaris, Phleum, Poa, Secale, Sorghum, Triticum, Zea, Ambrosia, Artemisia, Alnus, Betula, Carpinus, Carya, Castanea, Corylus, Fagus, Juglans, Ostrya, Quercus, Fraxinus, Ligustrum, Olea, Salsola, Plantago, Platanus, Humulus* and *Parietaria,* such as a non-profilin allergen selected from the group comprising Cry j 1, Cry j 2, Ant o 1, Cyn d 1, Cyn d 7, Cyn d 15, Cyn d 22w, Cyn d 23, Cyn d 2, Dac g 1, Dac g 2, Dac g 3, Dac g 4, Dac g 5, Fes p 4; Hol l 1, Hol l 5, Hor v 1, Hor v 5, Lol p 1, Lol p 2, Lol p 3, Lol p 4, Lol p 5, Lol p 11, Ory s 1, Pas n 1, Pha a 1, Pha a 5, Phl p 1, Phl p 2, Phl p 4, Phl p 5, Phl p 6, Phl p 7, Phl p 11, Phl p 13, Poa p 1, Poa p 5, Sec c 1, Sec c 5, Sor h 1, Tri a 15, Tri a 21, Tri a 27, Tri a 28, Tri a 29, Tri a 30, Tri a 31, Tri a 32, Tri a 33, Tri a 34, Tri a 35, Zea m 1, Zea m 12, Amb a 1, Amb a 2, Amb a 3, Amb a 4, Amb a 5, Amb a 6, Amb a 7, Amb a 9, Amb a 10, Amb p 5, Amb t 5, Art v 1, Art v 2, Art v 3, Art v 5, Art v 6, Sal k 1, Sal k 2, Sal k 3, Sal k 5, Aln g 1, Aln g 4, Bet v 1, Bet v 3, Bet v 4, Bet v 6, Bet v 7, Car b 1, Car i 1, Car i 4, Cas s 1, Cas s 5, Cas s 8, Cas s 9, Cor a 10, Fag e 2, Fag t 2, Fag s 1, Jug n 1, Jug n 2, Jug r 1, Jug r 2, Jug r 3, Jug r 4, Ost c 1, Que a 1, Fra e 1, Lig v 1, Ole e 1, Ole e 3, Ole e 4, Ole e 5, Ole e 6, Ole e 7, Ole e 8, Ole e 9, Ole e 10 and Ole e 11, Pla l 1, Pla a 1, Pla a 2, Pla a 3, Pla or 1, Pla or 2, Hum j 1; Par j 1, Par j 2, Par j 4 and Par o 1.

In other preferable embodiments of the invention, the non-profilin allergen is from a food or food ingredient of a plant genus selected from the group comprising *Hordeum, Oryza, Secale, Triticum, Zea, Apium, Daucus, Petroselinum, Helianthus, Lactuca, Arabidopsis, Brassica, Sinapsis, Cucumis, Fagopyrum, Actinidia, Betholletim, Glycine, Lens, Lupinus, Phaseolus, Juglans, Fragaria, Malus, Morus, Pyrus, Rubus, Ziziphus, Anacardium, Citrus, Mangifera, Litchi, Pistacia, Capsicum Lycopersicon, Nicotiana, Solanaum* and *Vitis.* More specifically, the profilin-containing plant material contains a non-profilin allergen selected from the group comprising Hor v 12, Hor v 15, Hor v 16, Hor v 17, Hor v 21, Ory s 12, Sec c 20, Tri a 12, Tri a 14, Tri a 18, Tri a 19, Tri a 21, Tri a 25, Tri a 26, Tri a 36, Zea m 14, Zea m 25, Api g 1, Api g 2, Api g 3, Api g 4, Api g 5, Api g 6, Dau c 1, Dau c 4, Hel a 3, Lac s 1, Bra j 1, Bra n 1, Bra o 3, Bra r 1, Bra r 2, Sin a 1, Sin a 2, Sin a 3, Sin a 4, Cuc m 1, Cuc m 2, Cuc m 3, Fag e 2, Act c 5, Act c 8, Act c 10, Act d 1, Act d 2, Act d 3, Act d 4, Act d 5, Act d 6, Act d 7, Act d 8, Act d 9, Act d 10, Act d 11, Ber e 1, Ber e 2, Ara h 1, Ara h 2, Ara h 3, Ara h 4, Ara h 5, Ara h 6, Ara h 7, Ara h 8, Ara h 9, Ara h 10, Ara h 11, Gly m 1, Gly m 2, Gly m 3, Gly m 4, Gly m 5, Gly m 6s Len c 1, Len c 2, Len c 3s Lup an 1 Pha v 3, Pis v 1, Pis v 2, Pis v 3, Pis v 4, Pis v 5, Pis s 1, Pis s 2, Vig r 1, Vig r 6, Cor a 1, Cor a 2, Cor a 8, Cor a 9, Cor a 11, Cor a 12, Cor a 13 and Cor a 14, Jug n 1 and Jug n 2, Jug r 1, Jug r 2, Jug r 3, Jug r 4, Pru du 3, Pru du 4, Pru du 5, Pru du 6, Fra a 1, Fra a 3, Fra a 4, Mal d 1, Mal d 2, Mal d 3, Mal d 4, Mor n 3, Pru ar 1, Pru ar 3, Pru av 1, Pru av 2, Pru av 3, Pru av 4, Pru d 3, Pru p 1, Pru p 2, Pru p 3, Pru p 4, Pyr c 1, Pyr c 3, Pyr c 4, Pyr c 5, Rub i 1, Rub i 3, Ziz m 1, Ana o 1, Ana o 2, Ana o 3, Cit l 3, Cit r 3, Cit s 1, Cit s 2, Cit s 3, Lit c 1, Man e 5, Pis v 1, Pis v 2, Pis v 3, Pis v 4, Pis v 5, Cap a 1w, Cap a 2, Lyc e 1, Lyc e 2, Lyc e 3, Lyc e 4, Sola t 1, Sola t 2, Sola t 3, Sola t 4 and Vit v 1.

Typical examples of grass pollen are from the order Poales, such as particularly from the plant family *Poaceae,* such as particularly from a plant genus of *Anthoxanthum, Cynodon, Dactylis, Festuca, Holcus, Hordeum, Lolium, Oryza, Paspalum, Phalaris, Phleum, Poa, Secale, Sorghum, Triticum* and *Zea.* Typically, weed pollen derives from the plant order *Asterales,* such as of the family *Asteraceae,* such as of the genus *Ambrosia* and *Artemisia.* Typically, tree pollen from the plant order *Fagales* (such as the families *Betulaceae (Betula, Alnus, Carpinus, Corylus, Ostrya), Fagaceae* (*Fagus, Quercus, Castanea*) and Juglandaceae (*Juglans and Carya);* from the plant order Lamiales (such as the family *Oleacea,* such as from the genus *Olea);* from the plant order *Pinales* (such as the family *Cupressaceae,* such as from the genus *Juniperus*)*.*

Food or food ingredients derives typically from the plant order *Apiales* (such as from the family *Apiaceae,* such as from the genus *Apium* and *Daucus); Fabales* (such as the families *Fabaceae* commonly known as the legume, pea or bean family), including among others *Glycine max* (soybean) and *Arachis hypogaea* (peanut); *Rosales* (such as the family *Rosaceae* including among others apples, apricots, plums, cherries, peaches, pears, raspberries, and strawberries); *Poales* (such as the family *Poaceae* which among others includes barley, maize, millet, rice, and wheat).

Moreover, the profilin-containing plant material may derive from the plant genus *Hevea* (latex family), which is relevant in the treatment of a hypersensitivity immune response to a non-profilin allergen of latex or any latex-containing material.

As should be understood, it is considered that any polypeptide having an amino acid sequence having at least 60% identity and/or similarity, such as at least 65%, 70%, 75%, 80%, 85%, 90% or 95% identity and/or similarity to the amino acid sequence of SEQ ID NO: 1 is usable for the treatment of a hypersensitivity immune response caused by a non-profilin allergen of a profilin-containing material mentioned in Tables 1, 2, or 3. However, to ensure immunological cross-reactivity at the time of exposure to the profilin-containing plant material, it is desirable that the polypeptide as used in the treatment phase (as used as a tolerance-inducing antigen) has an amino acid sequence having at least 60% identity to the amino acid sequence of the profilin present in the profilin-containing material to which the individual is exposed and which contains the non-profilin allergen causing the hypersensitivity immune response. Thus, a polypeptide of the invention may also have an amino acid sequence having at least 60% identity and/or similarity, such as at least 65%, 70%, 75%, 80%, 85%, 90% identity and/or similarity to the amino acid sequence of the profilin to which the individual is exposed upon being challenged to the non-profilin allergen of the profilin-containg plant material.

Thus, in preferred embodiments of the invention, the profilin-containing plant material is from a plant family selected from the group comprising of *Poaceae, Asteraceae, Betulaceae, Fagaceae, Juglandaceae, Oleacea, Apiaceae, Rosaceae* and *Euphorbiaceae* or a variant thereof, preferably wherein the variant and the profilin has an amino acid sequence having at least 60% identity to the amino acid sequence of the profilin of the profilin-containing plant material.

Thus, the polypeptide is a profilin of a plant species of a plant family selected from the group comprising *Poaceae, Asteraceae, Betulaceae, Fagaceae, Juglandaceae, Oleacea, Apiaceae, Rosaceae* and *Euphorbiaceae* or a variant thereof, preferably wherein the variant and the profilin have an amino acid sequence having at least 60% identity to the amino acid sequence of the profilin of the profilin-containing plant material and preferably wherein the hypersensitivity immune response is caused by a non-profilin allergen of a profilin-containing plant material of a plant species of a plant family selected from the group comprising *Poaceae, Asteraceae, Betulaceae, Fagaceae, Juglandaceae, Oleacea, Apiaceae, Rosaceae* and *Euphorbiaceae.*

More particularly, in preferred embodiments of the invention, the polypeptide is a profilin of a plant species of a plant genus selected from the group comprising of *Anthoxanthum, Cynodon, Dactylis, Festuca, Holcus, Hordeum, Lolium, Oryza, Paspalum, Phalaris, Phleum, Poa, Secale, Sorghum, Triticum, Zea, Apium, Daucus, Ambrosia, Artemisia, Arachis, Glycine, Alnus, Betula, Corylus, Fagus, Quercus, Olea, Plantago, Malus, Parietaria, Prunus, Lycopersicon* and *Solanum* or a variant thereof, preferably wherein the variant and the profilin have an amino acid sequence having at least 60% identity to the amino acid sequence of the profilin of the profilin-containing plant material and preferably wherein the hypersensitivity immune response is caused by a non-profilin allergen of a profilin-containing plant material of a plant species of a plant genus selected from the group comprising of *Anthoxanthum, Cynodon, Dactylis, Festuca, Holcus, Hordeum, Lolium, Oryza, Paspalum, Phalaris, Phleum, Poa, Secale, Sorghum, Triticum, Zea, Apium, Daucus, Ambrosia, Artemisia, Arachis, Glycine, Alnus, Betula, Corylus, Fagus, Quercus, Olea, Plantago, Malus, Parietaria, Prunus, Lycopersicon* and *Solanum.*

Even more particularly, in preferred embodiments of the invention, the polypeptide has an amino acid sequence selected from the group comprising of SEQ ID NOs: 11 to 43, which are exemplary amino acid sequences of profilins of the order *Poales (SEQ ID NO: 11-15, 40),* the order *Lamiales (SEQ ID NO:* 16), the order *Fagales (SEQ ID NO: 17),* the order *Fagales (SEQ ID NO:17-18),* the order *Fabales (SEQ ID NO:19-20),* the order *Asterales (SEQ ID NO: 21-23),* the order *Malpighiales (SEQ ID NO:* 24), the order *Rosales (SEQ ID NO: 25-23),* the order *Asterales (SEQ ID NO: 21-23),* the order *Asterales (SEQ ID NO: 24-31),* the order *Arecales (SEQ NO: 32),* the order *Caryophyllales (SEQ ID NO: 33-35),* the order *Apiales (SEQ ID NO: 36-37),* the order *Cucurbitales (SEQ ID NO: 38),* the order *Solanales (SEQ ID NO:* 39), the order *Asparagales (SEQ ID NO: 41),* the order *Zingiberales (SEQ ID NO: 42)* or the polypeptide is a variant of said profilin, as further defined below.

Notably, the SEQ ID NOs: 1-42 have a methionine as the first amino acid at the N-terminal end (position 1), which may be removed by enzymatic degradation in a host cell producing the sequences by recombinant techniques. Therefore, it should be understood that, in any of the amino acid sequences mentioned herein (SEQ ID NOs: 1-42), the methionine group in position 1 is not present. Thus, in some embodiments, the amino acid sequences of SEQ ID NOs: 1-42 do not contain methionine in position 1.

A polypeptide may also be selected from known profilins, such as profilins selected from the group comprising Cry j profilin, Pho d 2, Ana c 1 ,Cyn d 12, Tri a 12, Mus a 1, Api g 4, Dau c 4, Amb a 8, Art v 4, Hel a 2, Ara t 8, Sin a 4, Ama r 2, Beta v 2, Che e 2, Sal k 4, Act d 9, Cuc m 2, Ara h 5, Gly m 3, Bet v 2, Cor a 2, Ole e 2, Hev b 8, Mer a 12, Pla a 3, Fra a 4, Mal d 4, Par j 3, Pru av 4, Pru du 4, Pru p 4, Pyr c 4, Cit s 2, Lit c 1, Cap a 2, Lyc e 1 or a variant thereof.

Moreover, in preferred embodiments of the invention, the polypeptide has a high similarity to any of the profilins shown in SEQs 1 to 42, such that at least 60%, such as at least 65%, 70%, 75%, 80%, 85%, 90% and 95% of the amino acids sequence overlap with the amino acid sequence of a profilin selected from the group of SEQ ID NOs: 1-42.

The polypeptide may be used in its naturally occurring form including or excluding various isoforms, it may be extracted/isolated from a profilin-containing plant material or it may be reproduced by use of biological methods (e.g. recombinant techniques) or synthetic methods as well known in the art.

Therefore, in some embodiments, a polypeptide used in the present invention is in a purified form and/or isolated form, whereas in other embodiments, a polypeptide of the invention may be a variant of a natural occurring profilin from a profilin-containing plant material mentioned herein. For example, the parent polypeptide (the natural occurring polypeptide) may be modified for reasons of improper solubility, bioavailability, safety, toxicology, or stability, such as by any chemical and/or biological method.

Typical examples of chemical modifications are N- or O-linked glycosylation or derivatizing of the N-terminus or of thiol groups. Typical examples of biological modifications are post-translational modifications, such as for example; glycosylation, acetylation, alkylation (methylation, ethylation), biotinylation, glutamylation, glycylation, isoprenylation, lipoylation, phosphopantetheinylation, phosphorylation, sulfation, selenation and C-terminal amidation. The polypeptide may also be modified by treatment with formaldehyde or glutaraldehyde.

It should generally be understood that the term "variant" or "variants" refers to polypeptides which contain modifications/mutations compared to the amino acid sequence of the "parent polypeptide" which is considered to be a natural occurring profilin from a profilin-containing plant material mentioned herein or at least a polypeptide having/comprising an amino acid sequence of SEQ ID NO: 1 or alternatively SEQ ID NOs: 2-42.

Thus, the present invention encompasses the use of polypeptide variants and derivatives of any amino acid sequence of a polypeptide as defined herein, particularly those of SEQ ID NOs: 1-10 or those of SEQ ID NOs: 11-42 as defined below.

The variant polypeptides of the invention may have modifications/mutations such as insertions, substitutions, deletions, duplications, insertion-deletions, frame shifts, transversions, truncations, and/or inversions at one or more locations in the parent polypeptide, such as where one amino acid or several amino acids, such as 2, 3, 4, 5, 6, 7, 8, 9, 10, 15, 20, 25, 30, 35, 40, 45 or 50 amino acids of the parent polypeptide have been subject to substitution, deletion, duplication, insertion-deletion, frame shift, transversions, truncations, and/or inversions, preferably wherein the number of amino acids in the amino acid sequence of the variant polypeptide is in the range of 50% to 150%, such as in the range of 75% to 125% of the number of amino acids of the parent polypeptide.

Native profilins usually contain an amino acid chain length of about 120 to 170 amino acids, typically about 130 to 135 amino acids. The molecular weight is typically about 14-16 kDa. It is considered that a polypeptide (parent as well as a variant thereof) has an amino acid chain length ranging between 65 and 195 amino acids, such as between 70 and 190; 75 and 185; 80 and 180; 85 and 175; 90 and 170; 95 and 165; 100 and 160; 105 and 155; 110 and 150; 115 and 145; 120 and 140 amino acids.

Thus, exemplary variant polypeptides may be substitution variants, deletion variants, duplication variants, insertion variants, insertion-deletion variants, frame shift variants, transversion variants, truncation variants, and/or inversion variants or any other suitable variants, preferably wherein the number of amino acids in the amino acid sequence of the variant polypeptide is in the range of 50% to 150%, such as in the range of 55% to 145%, 60% to 140%, 65% to 135%, 70% to 130%, 75% to 125%, 80% to 120%, 85% to 115% or 90% to 110% or 95%-105% compared to the number of amino acids of the parent polypeptide.

The variant polypeptide may be a polypeptide having a certain percent identity and/or similarity, e.g., 60%, 65%, 66%, 68%, 70%, 72%, 74%, 76%, 78%, 80%, 85%, 90%, 91%, 92%, 93%, 94%, 95%, 96%, 97%, 98%, or 99% of amino acid sequence identity with SEQ ID NO: 1 or alternatively SEQ ID NOs: 2-42.

Sequence alignment is a way of arranging the sequences of DNA, RNA, or protein whereby the sequences may be compared. It is thus possible to identify regions of identity, similarity or homology. The identity, similarity or homology may be a consequence of functional, structural, or evolutionary relationships between the sequences or it may be a coincidence.

The commercially available computer programs for determining sequence identity use complex comparison algorithms to align two or more sequences that best reflect the evolutionary events that might have led to the difference(s) between the two or more sequences. Therefore, these algorithms operate with a scoring system rewarding alignment of identical or similar amino acids and penalizing the insertion of gaps, gap extensions and alignment of non-similar amino acids. The scoring system of the comparison algorithms includes: i) assignment of a penalty score each time a gap is inserted (gap penalty score), ii) assignment of a penalty score each time an existing gap is extended with an extra position (extension penalty score), iii) assignment of high scores upon alignment of identical amino acids, and iv) assignment of variable scores upon alignment of non-identical amino acids

Most alignment programs allow the gap penalties to be modified. However, it is preferred to use the default values when using such software for sequence comparisons. The scores given for alignment of non-identical amino acids are assigned according to a scoring matrix also called a substitution matrix. The scores provided in such substitution matrices reflect the fact that the likelihood of one amino acid being substituted with another during evolution varies and depends on the physical/chemical nature of the amino acid to be substituted. For example, the likelihood of a polar amino acid being substituted with another polar amino acid is higher compared to being substituted with a hydrophobic amino acid. Therefore, the scoring matrix will assign the highest score for identical amino acids, lower score for non-identical but similar amino acids and even lower score for non-identical and non-similar amino acids. Programs can be found on hhttp\:ncbi.nlm.nih.gov.

Once the software has produced an alignment, it is possible to calculate sequence similarity and sequence identity. The software typically does this as part of the sequence comparison and generates a numerical result. When comparing sequences by eye, a Venn diagram may be used. The diagram illustrates which amino acids belong to which set or subset thus having one or more similar properties (structural or chemical). Preferably, the amino acid identity and similarity are calculated across the full-length amino acid sequence or for nucleic acid to a corresponding polynucleotide which encodes the respective full-length amino acid sequence.

For example, sequence identity and similarity may be determined by comparing two aligned amino acid sequences over a comparison window, preferably comparing full-length amino acid sequences, where the variant polypeptide may comprise additions or deletions (e.g., gaps or overhangs) as compared to the parent polypeptide for optimal alignment of the two sequences. The percentage identity is calculated by determining the number of positions at which the identical amino acid residue occurs in both sequences to yield the number of matched positions, dividing the number of matched positions by the total number of amino acids in the window of comparison and multiplying the result by 100 to yield the percentage of sequence identity. Optimal alignment of sequences for comparison may be conducted by computerized implementations of algorithms (GAP, BESTFIT, BLAST, PASTA, and TFASTA or by inspection. Given that two sequences have been identified for comparison, GAP and BESTFIT are preferably employed to determine their optimal alignment. Typically, the default values of 5.00 for gap weight and 0.30 for gap weight length are used.

When the modification/mutation is a substitution (substitution variant), the substitution may be conservative (within same set or subset) or they may be non-conservative (between sets or subsets). The substitutions may produce a silent change and result in a functionally equivalent polypeptide. Deliberate amino acid substitutions may be made on the basis of similarity, i.e. like-for-like substitution such as basic for basic, acidic for acidic, polar for polar etc.

Non-conservative substitution may also occur, i.e. from one set or subset of residues to another or alternatively involving the inclusion of unnatural amino acids such as ornithine (hereinafter referred to as Z), diaminobutyric acid ornithine (hereinafter referred to as B), norleucine ornithine (hereinafter referred to as 0), pyriylalanine, thienylalanine, naphthylalanine and phenylglycine.

Concerning conservative and similar substitutions, a table based on the Venn diagram is found in Table 6 which groups the amino acids according to their similarity in structural and functional characteristics into sets (Polar/hydrophobic/small) and subsets (aromatic/aliphatic/charge/tiny or not). Substitutions are considered conservative if the change is between amino acids of the same set and similar if the change is between amino acids of the same subset (but not same set) as specified in Table 7. Whether the substitution needs to be within a set or only within a subset in order to be a conservative or similar substitution depends on the location in the protein structure. It depends on whether the specific amino acid is in a region or position of particular importance or not, e.g. folding, binding site, active site. The skilled person would consider using the substitution guidances as shown in Table 7 as a first choice and to apply the guidance provided by Table 8 in a further attempt.

In the following, position(s) and substitutions are listed with reference to SEQ ID NO: 1 unless otherwise stated. Equivalent positions in another sequence may be found by aligning this sequence with SEQ ID NO: 1 to find an alignment with the highest percent identity and thereafter determining which amino acid aligns to and/or corresponds with an amino acid of a specific position of SEQ ID NO: 1. Such alignment and use of one sequence as a first reference is simply a matter of routine for one of ordinary skill in the art and should not limit the scope of the invention.

We have found that the known plant profilins have a high degree of sequence identity within positions corresponding to positions 71-127 of SEQ ID NO: 1. For ease of reference, this part sequence is referred to as SEQ ID NO: 43. Accordingly, a polypeptide according to the invention has at least 60%, preferably at least 65%, 70%, 75% sequence identity to SEQ ID NO: 1 and at least 80%, preferably 85%, 90%, more preferred at least 95% sequence identity to SEQ ID NO: 43.

In one embodiment, a variant polypeptide of the invention includes variants wherein one and up to 25 amino acids has/have been added or deleted with respect to SEQ ID NO: 1.

In some embodiments, the variant polypeptide of the invention has the amino acid sequence of SEQ ID NO: 1, wherein any number in the range of 1 and 25 amino acids has been substituted.

In some embodiments, the variant polypeptide of the invention has the amino acid sequence of SEQ ID NO: 1, wherein any number in the range of 1 and 12 amino acids has been substituted.

In some embodiments, the variant polypeptide of the invention has the amino acid sequence of SEQ ID NO: 1, wherein any number in the range of 3 and 9 amino acids has been substituted.

In further embodiments thereof, at least two, such as at least three, such as at least five amino acids of SEQ ID NO: 1 have been substituted.

Profilins from three distinct sources (plant, mammal, lower eucaryote) have been analyzed and compared in the article "the crystal structure of a major allergen from plants" by Thorn et al. (1997), 18 amino acids were identified which are conserved in 80% of the 35 profilin sequences analysed. With reference to SEQ ID NO: 1, amino acids K87 and G113 are involved in actin binding, amino acids W3, Y6, I25, G27, W33, A34, Y125 and L126 are involved in Poly-L-Prolin (PLP) binding, and amino acids A23, A24, E46, G64, G69 and T97 are involved in fold conservation.

Accordingly, in one embodiment of the invention, the variant polypeptide of the invention has at least 60%, preferably at least 65% or 70% and more preferred at least 75%, 80%, 85% and 90% sequence identity to SEQ ID NO: 1 and comprises the amino acids corresponding to K87 and G113 of SEQ ID NO: 1. An advantage of such variants is that the actin binding site may be used in the purification of the variant polypeptide.

In another embodiment, the variant polypeptide of the invention has at least 60%, preferably at least 65% or 70% and more preferred at least 75%, 80%, 85% and 90% sequence identity to SEQ ID NO: 1 and comprises the amino acids corresponding to W3, Y6, I25, G27, W33, A34, Y125 and L126 of SEQ ID NO: 1. An advantage of such variants is that the PLP binding site may be used in the purification of the variant polypeptide.

In yet another embodiment, the variant polypeptide of the invention has at least 60%, preferably at least 65% or 70% and more preferred at least 75%, 80%, 85% and 90% sequence identity to SEQ ID NO: 1 and comprises the amino acids corresponding to A23, A24, E46, G64, G69 and T97 of SEQ ID NO: 1. An advantage of such variants is that the folding is conserved.

In one embodiment of the invention, the variant polypeptide of the invention has at least 60%, preferably at least 65% or 70% and more preferred at least 75%, 80%, 85% and 90% sequence identity to SEQ ID NO: 1 and comprises an amino acid selected from A, G or S (tiny amino acids) in a position corresponding to position 13 and/or 115 of SEQ ID NO: 1. A preferred variant polypeptide of the invention comprises A in a position corresponding to position 13 and/or 115 of SEQ ID NO: 1. Another preferred variant polypeptide of the invention comprises S in a position corresponding to position 13 and/or 115 of SEQ ID NO: 1.

In another embodiment of the invention, the variant polypeptide of the invention has at least 60%, preferably at least 65% or 70% and more preferred at least 75%, 80%, 85% and 90% sequence identity to SEQ ID NO: 1 and has all cysteine residues of the parent polypeptide substituted with one of the other tiny amino acids A, G or S.

It is known that substitution of tryptophan (W) often has great influence on the protein. Accordingly, a preferred variant polypeptide of the invention has at least 60%, preferably at least 65% or 70% and more preferred at least 75%, 80%, 85% and 90% sequence identity to SEQ ID NO: 1 and comprises the amino acids corresponding to W3 and W33 of SEQ ID NO: 1. Notably, the presense of tryptophan is important as such polypeptides may have fluorescence, making the analytical work easier.

When aligning the sequences SEQ ID NOs: 1-42 of the invention using for example the BLAST program, some specific conservative substitutions among the various profilins can be observed. Typical examples on positions with reference to SEQ ID NO: 1 that may be subjected to amino acids variation are shown in Table 5. Thus, any amino acid mentioned in the column "amino acid variation" constitutes a typical example of amino acids which may substitute an amino acid of the parent polypeptide (SEQ ID NO: 1) in order to make a variant thereof.

A second aspect of the invention relates to a polypeptide having an amino acid sequence of SEQ ID NOs: 1, 2, 3, 4, 5, 6, 7, 8, 9 or 10, wherein 1 or 2 cysteine residue(s) is/are substituted by an amino acid selected from A, G or S, either the same amino acid replacing both cysteines or different ones. Such variants may be less susceptible to oxidation and thus less prone to dimerization, polymerization or to form aggregates. Such polypeptides may be produced recombinantly or alternatively synthesized.

In particular, a variant is provided of a polypeptide of SEQ ID NO: 1 or alternatively SEQ ID NOs: 2-10 wherein at least 1, 2 or all of the cysteine residues have been substituted by an amino acid selected from A, G or S. In particular, this variant polypeptide has an amino acid sequence of SEQ ID NO: 1, but where the amino acid cysteine in position 13 and/or 115 has been substituted by an amino acid selected from the group comprising A (alanine), G (glycine) and S (serine), so as to produce the variants C13A, C115A, C13G, C115G, C13S, C115S and mixtures thereof, such as C13A and C115S. A typical example of such a variant has an amino acid sequence of SEQ ID NO: 44 (two cysteines substitutes by serine, i.e. C13S, C115S) or 45 (two cysteines substitutes by alanine, i.e. C13A, C115A), preferably wherein methionine is not present in position 1, or at least only present in 5% of the polypeptides produced recombinantly.

Advantageously, such polypeptides of SEQ ID NOs: 44 and 45 are stable, in particularly with respect to auto oxidation and aggregation. In addition, such polypeptides may bind human actin to a lesser extent than native Phl p 12.

Furthermore, the tertiary structure of the polypeptide having SEQ ID NO: 45 was more stable than the polypeptide having SEQ ID NO: 44. Thus, substitution with alanine for cysteine stabilizes the molecular structure more than substitution with serine.

It is envisaged that the cysteine-substitution variant of SEQ ID NOs: 44 and 45 in addition to be used in non-specific immunotherapy (bystander tolerance) also can be used in specific allergen immunotherapy, wherein the hypersensitivity immune response is caused by an allergenic profilin of a profilin-containing plant material or an allergen cross-reacting with IgE antibodies specific to said profilin. Notably, in such embodiments, the individual is usually sensitized to the profilin.

As a proteinaceous antigen, the polypeptide may possess immunogenic properties and may be recognized by the individual's immune system as foreign. Thus, a polypeptide of the invention is an immunogenic polypeptide. It may induce specific IgG antibodies IgM or IgA antibodies capable of binding to, complexing to or otherwise associating to the polypeptide.

A profilin is usually characterized by the ability to bind poly-L-proline. For example, this property is advantageously used in the purification/isolation of profilins from profilin-containing materials in that an extract thereof, e.g. an aqueous extract thereof, is added to poly-L-Pro affinity chromatographic bead, such as poly-L-proline immobilized on agarose beads. The association is strong, and typically at least 6 M urea is required to elute the bound profilin from the column. Typically, a profilin is eluted from the column with 6-8 M urea, renatured by dialysis against a low ionic strength buffer without urea and, optionally further separated from other components of the extract by cation/anion exchange chromatography and/or gel filtration (Amnuaycheewa and Gonzalez de Mejia, 2010 and Vidali et al, 1995). Same method may be used in purifying a polypeptide of the invention produced by recombinant techniques.

A profilin may further be characterized by its ability to bind an actin. Profilin binds to monomeric actin (G-actin) thereby occupying an actin-actin contact site, the effect being the sequestration of G-actin from the monomeric actin pool and the subsequent prevention of polymerization of actin (to form F-actin). Thus, in a mixture of actin, profilin, and nucleotides (ADP and ATP), actin will polymerize to a certain extent, which may be estimated by the law of mass action. The ability of a polypeptide to associate with actin, such as a human or plant actin, may be tested by studying the incorporation of G-actin (preferably fluorescence labelled) in the polypeptide in the presence of the F-actin in that the F-actin pool and G-actin pool can be separated by centrifugation. Commercial kits for testing actin binding are available from Cytoskeleton Inc. Alternatively, the polypeptide may be bound to poly-L-proline sepharose beads and incubated with G-actin, and the concentration of not bound G-actin may be determined in the supernatant. Further details on actin binding assays can be found in the scientific papers of Lu and Pollard (2001) and Fechheimer and Zigmond (1993).A polypeptide of the invention preferably binds to or associates with a plant actin, but not with a human actin.

A polypeptide for use in the present invention including a variant polypeptide may be produced by recombinant DNA techniques according to known methods in the art. As host cells may be used any genetically modified cell that comprises either a nucleotide sequence encoding a polypeptide of the invention or an expression vector, such as a genetically modified cell of an eukaryotic (such as yeast *Pichia*) or non-eucaryotic cell (such as *E. coli*). In one embodiment the host cell is a bacterial cell, preferably *E. coli.* An advantage of using bacterial cells is that bacterial cells do not produce profilins natively. Profilin is thus heterologous to bacterial cells. In particular, it is heterologous to *E. coli.* Thus purification/isolation of the recombinant polypeptide is not complicated by interfering native proteins.

Exemplary *E. coli* strains suitable for producing the polypeptide of the invention are BL21(DE3), BL21(DE3)plysS or Rosetta(DE3)plysS (all commercially available from NOVAGEN) or BL21(DE3)gold (commercially available from Agilent Technologies).

Having produced the polypeptide in a host cell using the appropriate plasmids and cDNA, the polypeptide is isolated by separating the cell(s) from the broth during or after fermentation so that the polypeptide remains in the broth. The polypeptide may be further purified from the broth such that it is substantially free from other components of the culture medium in which it was produced by any suitable methods, preferably chromatographic methods (e.g. affinity chromatography, size exclusion chromatography, anion or cation exchange chromatography, optionally combined with dialysis).

### Method of Detecting/Providing a Polypeptide of the Invention

To detect polypeptides, which most likely will be present at the target organ concomitantly with the non-profilin allergen a method for screening for a polypeptide (e.g.profilin) suitable for being used in the present invention entails:
i) extracting a profilin-containing plant material in an aqueous solution having pH in the range of 6-8 for a period ranging from 1 to 30 minutes; ii) screening the extract for content of an non-profilin allergen and a profilin; iii) optionally isolating the profilin; and iv) optionally identifying the profilin; wherein when the extract contains both an non-profilin allergen and a profilin, said profilin may be selected as the polypeptide for use according to the present invention.

Having identified the profilin, the profilin or a variant as defined herein may be produced by recombinant techniques well known in the art.

Obtaining a polypeptide (e.g. profilin) usable in the present invention may entail:
i) extracting a profilin-containing plant material in an aqueous solution having pH in the range of 6-8 for a period ranging from 1 to 30 minutes; ii) screening the extract for content of an non-profilin allergen and a profilin; iii) identifying the profilin, optionally wherein the profilin is isolated; and vi) optionally producing the polypeptide or a variant thereof by recombinant techniques, wherein when the extract contains both an non-profilin allergen and a profilin, said profilin may be selected as the polypeptide for use according to the present invention and said profilin may be further modified to obtain a variant thereof.

A method for obtaining a polypeptide (e.g. a profilin), suitable for use in treating via bystander tolerance a hypersensitivity immune response caused by a non-profilin allergen of a profilin-containing plant comprises isolating an immunogenic polypeptide from said profilin-containing plant material or recombinant producing said immunogenic polypeptide, wherein
said immunogenic polypeptide has been identified in and optionally isolated from an extract made by suspending the profilin-containing plant material in an aqueous solution having pH in the range of 6-8 for a period ranging from 1 to 30 minutes. Preferably, the extract also contains a non-profilin allergen of said profilin-containing plant material, i.e. a non-profilin allergen co-extracted with the immunogenic polypeptide.

A polypeptide (e.g. a profilin), suitable for use in treating via bystander tolerance a hypersensitivity immune response caused by a non-profilin allergen of a profilin-containing plant material can be obtained by isolating an immunogenic polypeptide from said profilin-containing plant material or recombinant producing said immunogenic polypeptide, wherein
said immunogenic polypeptide has been identified in and optionally isolated from an extract made by suspending the profilin-containing plant material in an aqueous solution having pH in the range of 6-8 for a period ranging from 1 to 30 minutes. Preferably, the extract also contains a non-profilin allergen of said profilin-containing plant material, i.e. a non-profilin allergen co-extracted with the immunogenic polypeptide.

That is to say that a polypeptide of the invention (e.g. a profilin) is co-extractable with a non-profilin allergen of the profilin-containing plant material when extracting the profilin-containing plant material in an aqueous solution having pH in the range of 6-8 for a period ranging from 1 to 30 minutes. Furthermore, it is emphasized that the polypeptide of the invention (e.g. a profilin) is obtainable by a method comprising isolating a profilin from said profilin-containing plant material or recombinantly producing said profilin, wherein said profilin has been identified in an extract made by suspending the profilin-containing plant material in an aqueous solution having pH in the range of 6-8 for a period ranging from 1 to 30 minutes.

The extraction may be carried out under conditions simulating the physiological conditions of the target organ, such as at physiological conditions present in the nasal fluid. Thus, the extraction solution is substantially an aqueous solution having pH in the range of 5.5 - 8.5, such as in the range of 6-8, preferably about pH 7 and the osmolality preferably corresponds to that of a solution of 0.90% w/v of NaCl having about 300 mOsm/L. The extraction temperature is preferably in the range of 2°C to 40°C. The aqueous solution preferably comprises at least 60% of water, such as preferably at least 70, 75, 80, 85, 90 and 95% of water. In addition, the aqueous solution may contain non-aqueous water-miscible liquids, such as lower alkanols (e.g., having 1 to 6 carbon atoms; such as, ethanol, propanol), arylalkanols (e.g., having 5 to 10 carbon atoms in the rings; such as, benzyl alcohol), polyols (e.g., having 2 to 6 carbon atoms; such as glycerol, propylene glycol, or sorbitol), n-methylpyrrolidone, polyalkylene glycols (e.g., polyethylene glycol, propylene glycol, and the like), polyglycerin, triacetin, dimethyl acetimide and dimethyl sulfoxide.

To control the pH, the aqueous solution may be buffered, such as by the addition of a buffering agent/system like ascorbic acid, citric acid, phosphate buffer vehicle systems, TRIS (tris(hydroxymethyl)aminomethane), ammonium carbonate and wherein the desired pH is optionally achieved by adding a few drops of HCl or NaOH. Thus, the aqueous solution may comprise a buffering agent, such as ascorbic acid, citric acid, phosphate buffer vehicle systems, TRIS, ammonium carbonate/ammonium hydrogen carbonate buffering system.

To control the osmolality/ionic strength, isotonic vehicles may be added to the aqueous solution, such as to achieve an osmolality corresponding to that of the physiological conditions of the target organ. Thus, the aqueous solution may further comprise an osmolality providing agent such as boric acid, sodium chloride, potassium chloride, sodium citrate, sodium acetate, and the like. Typically, the ionic strength is in the range of 10 mM to 1000 mM, such as preferably in the range of 20 to 800 mM, such as 20 mM to 500 mM, such as such as 50 mM to 500 mM, such as 20 mM to 400 mM, such as 50 mM to 400 mM, such as 20 mM to 300 mM, such as 50 mM to 300 mM. More preferably the ionic strength is in the range of 150 to 180 mM, such as about 160 to 170 mM. Preferably, the osmolality is in the range of 50 to 600 mOsm/L, such as in the range of 50 to 500 mOsm/L, such as in the range of 100 to 400 mOsm/L, such as about 300 mOsm/L.

Furthermore, the viscosity may be adjusted by adding gelling agents, such as alkyl cellulose materials (e.g. carboxymethyl cellulose, carboxyethyl cellulose, hydroxypropylmethylcellulose, hydroxypropylethylcellulose, etc.), carbopol, polyvinyl alcohol, polyvinyl pyrrolidone and isopropyl myristate.

A typical example of a buffered aqueous solution is PBS buffer having pH of 7.2 and ionic strength (µ) of 165.8 mM (≈ 0.17 M) and which consists of sodium chloride (NaCl) in an amount of 8 g/L (137 mM), potassium chloride (KCl) in an amount of 0.2 g/L (2.7 mM), dinatrium hydrogen phosphate (Na₂HPO₄, 2H₂O) in an amount of 1.44 g/L (8.2 mM), potassium dihydrogen phosphate in an amount of 0.2 g/L (1.5 mM), the resulting pH is 7.2 and the resulting total ionic strength (µ) is 165.8 mM (≈ 0.17 M).

It is considered likely that a profilin capable of being co-extracted with a non-profilin allergen within a short time period of extraction is usable as the polypeptide of the invention, since this indicates that the polypeptide may co-elute with the non-profilin allergen at the target organ. As the profilin-containing plant material may reside only shortly in the epithelia/mucosa of the target organ in a short period of time before being cleared away by physiological processes, it is considered even more predictive to apply an extraction time shorter than 30 minutes, such as less than 20 minutes, more preferably less than 15, 10, 8, 7, 6, 5, 3 and 2 minutes. Moreover, the extraction may be carried out at temperatures in the range of 15 to 45°C, more preferably in the range of 18-37°C, such as at a physiological temperature or lower, such as at room temperature. However, if faced with stability problems, the extraction may be carried out at lower temperatures, such as under 10°C, such as about 3-5°C.

Having completed the extraction, the extract is screened for content of a non-profilin-allergen and a profilin by methods well known in the art. Typically, several extracts having different lengths of extraction time (such as 2, 5, 10 and 20 minutes) are produced or alternatively several samples are taken out from the same extraction solution at different time points (such as 2, 5, 10 and 20 minutes), and those samples are subjected to immunochemical methods like CIE (Crossed Immune Electrophoresis), RIE (Rocket immune electrophoresis), SDS Page (sodium dodecyl sulfate polyacrylamide gel electrophoresis), ELISA (Enzyme-linked immunosorbent assay) and/or MS (Mass Spectrometry).

Furthermore, the profilin may be recognized by its ability to bind actin and/or PLP. Immunogenic profilins may be found by performing CIE with polyclonal antibodies raised in rabbits against a purified extract of the profilin-containing plant material. Also, CIE may be made using polyclonal antibodies raised against a known profilin mentioned herein, such as Phl p 12, (SEQ ID NOs: 1-10), so as to identify immunogenic polypeptides reacting with antibodies specific to the known profilin, and thus an immunogenic polypeptide qualifying as a polypeptide with immunological cross-reactivity to the known profilin. Allergens may be detected by performing CIE with serum from an individual allergic/sensitized to a non-profilin allergen of said profilin-containing plant material. Detailed describtions and protocols of CIE methods and other immunochemical methods can be found in chapter 13 (Immuno-electrophoresis for the characterisation of allergen extracts, authors Gitte Nordskov Hansen and Jørgen Nedergaard Larsen) of the book Allergy Methods and Protocols (Methods in Molecular Medicine) by Penny Lympany (2008); ISBN: 9780896038967.

Thus, a polypeptide of the invention is able to bind to antibodies raised against a profilin selected from Cry j profilin, Pho d 2, Ana c 1 ,Cyn d 12, Tri a 12, Mus a 1, Api g 4, Dau c 4, Amb a 8, Art v 4, Hel a 2, Ara t 8, Sin a 4, Ama r 2, Beta v 2, Che e 2, Sal k 4, Act d 9, Cuc m 2, Ara h 5, Gly m 3, Bet v 2, Cor a 2, Ole e 2, Hev b 8, Mer a 12, Pla a 3, Fra a 4, Mal d 4, Par j 3, Pru av 4, Pru du 4, Pru p 4, Pyr c 4, Cit s 2, Lit c 1, Cap a 2 and Lyc e 1, such as raised against a polypeptide having SEQ ID NO: 1 or alternatively SEQ ID NOs: 2-42. The antibodies may be polyclonal or monoclonal and are typically raised in rabbits.

Having identified a profilin which is co-extractable with a non-profilin allergen, such as preferably a major allergen that is not profilin, the profilin may be further characterized either directly in the same extract or after isolation and optionally purification according to methods well known in the art.

For example, the profilin may be isolated by use of poly-L-proline, e.g. a poly-L-Pro affinity chromatographic bead, such as poly-L-proline immobilized on agarose beads or by other chromatographic methods known in the art among others size-exclusion chromatography, reverse phase chromatography, anion/cation exchange chromatography or combinations thereof.

Having isolated the profilin, it may further be analyzed to elucidate the amino acid sequence by methods known in the art, such as amino acid sequencing and/or by mass spectrometry, optionally following digesting the polypeptide by proteolytic enzymes like trypsin.

Finally, having identified the naturally occurring profilin, the amino acid sequence may be modified to obtain a variant thereof (a polypeptide variant as described above), such as a cysteine-replacement variant thereof, and producing the variant or the original (natural one) profilin by recombinant techniques well known in the art.

### Formulations

Where the polypeptide exhibits poor stability to the gastric juice, the polypeptide is preferably administered in a form avoiding the contact with the gastric juice, such as in a form preventing the degradation of the unrelated antigen in the gastric juice. This may be accomplished by incorporating the unrelated antigen in pharmaceutical formulations resistant to the gastric juice or by incorporating other pharmaceutical delivery techniques able to avoid the degradation of proteins in the gastric fluid.

The polypeptide of the invention may be formulated together with therapeutically inactive ingredients and/or immune-modifying agents like adjuvants. Typically, the formulation is a solid dosage form, such as a fast-disintegrating tablet or a liquid including a solution, a suspension, a dispersion, a gelled liquid. Alternatively, the formulation is an emulsion or a redissolvable powder, granulate or lyophilisate, which can be dissolved to form a liquid before being administered.

Excipients for use in formulations are well-known to the person skilled in the art and include solvents, emulsifiers, wetting agents, plasticizers, colouring substances, fillers, preservatives, viscosity adjusting agents, buffering agents, pH adjusting agents, isotonicity adjusting agents, mucoadhesive substances, and the like. Examples of formulation strategies are well-known to the person skilled in the art.

The adjuvant may be any conventional adjuvant, including oxygen-containing metal salts, e.g. aluminium hydroxide, chitosan, heat-labile enterotoxin (LT), cholera toxin (CT), cholera toxin B subunit (CTB), polymerised liposomes, mutant toxins, e.g. LTK63 and LTR72, microcapsules, interleukins (e.g. IL-1 BETA, IL-2, IL-7, IL-12, INFGAMMA), GM-CSF, MDF derivatives, CpG oligonucleotides, LPS, MPL, phosphophazenes, Adju-Phos(R), glucan, antigen formulation, liposomes, DDE, DHEA, DMPC, DMPG, DOC/Alum Complex, Freund's incomplete adjuvant, ISCOMs(R), LT Oral Adjuvant, muramyl dipeptide, monophosphoryl lipid A, muramyl thpeptide, and phospatidylethanolamine.

Embodiments of the invention, which may be combined in any order:
- A polypeptide for use in the treatment of a hypersensitivity immune response in an individual caused by a non-profilin allergen of a profilin-containing plant material, wherein said polypeptide has/consists of/consists essentially of/comprises an amino sequence having at least 60% identity to the amino acid sequence of SEQ ID NO: 1 , wherein methionine is optionally not present in the amino acid sequence at the N-terminal end, and:
- wherein said polypeptide alternatively has/consists of/consists essentially of /comprises an amino sequence having at least 60% identity (such as at least 65% ,70%, 75%, 80%, 85%, 90% or 95% identity) to the amino acid sequence of SEQ ID NOs: 2-10.
- wherein the polypeptide has an amino acid sequence having at least 60% identity (such as at least 65% ,70%, 75%, 80%, 85%, 90% or 95% identity) to the amino acid sequence of the profilin of the profilin-containing plant material.
- wherein the polypeptide is a profilin of a profilin-containing plant material of a plant class selected from the group consisting of *Plantae Pinopsida, Plantae Monocots* and *Plantae Magnoliopsida* or is a variant of said profilin.
- wherein the hypersensitivity immune response is caused by a non-profilin allergen of a profilin-containing plant material of a plant class selected from the group consisting of *Plantae Pinopsida, Plantae Monocots* and *Plantae Magnoliopsida.*
- wherein the polypeptide is a profilin of a profilin-containing plant material of a plant order selected from the group consisting of *Pinales, Arecales, Asparagales, Poales,* Zingiberales *Apiales, Asterales, Brassicalis, Curcurbitales, Ericales, Fabales, Fagales, Gentianales, Lamiales, Laurales, Malvales, Malpighiales, Myrtales, Proteales, Rosales, Sapindales, Solanales* and *Vitales* or is a variant of said profilin.
- wherein the hypersensitivity immune response is caused by a non-profilin allergen of a profilin-containing plant material of a plant order selected from the group consisting of *Pinales,* Arecales, *Asparagales, Poales, Zingiberales, Apiales, Asterales, Brassicalis, Curcurbitales, Ericales, Fabales, Fagales, Gentianales, Lamiales, Laurales, Malvales, Malpighiales, Myrtales, Proteales, Rosales, Sapindales, Solanales* and *Vitales.*
- wherein the polypeptide is a profilin of a profilin-containing plant material of a plant family selected from the group consisting of *Cupressaceae, Arecaceae, Asparagaceae, Iridaceae, Bromeliaceae, Poaceae, Musaceae, Zingiberaceae, Apiaceae, Araliaceae, Asteraceae, Brassicaceae, Amaranthaceae, Caryophyllaceae, Polygonaceae, Cucurbitaceae, Actinidiaceae, Lecythidaceae, Theaceae, Fabaceae, Betulaceae, Fagaceae, Juglandaceae, Myricaceae, Nothofagaceae, Ticodendraceae, Apocynaceae, Rubiaceae, Oleaceae, Pedaliacae, Plantaginaceae, Lauraceae, Malvaceae, Euphorbiaceae, Lythraceae, Platanaceae, Cannabaceae, Rosaceae, Ulmaceae, Urticaceae, Anacardiaceae, Rutaceae, Sapindaceae, Solanaceae* and *Vitaceae* or is a variant of said profilin.
- wherein the hypersensitivity immune response is caused by a non-profilin allergen of a profilin-containing plant material of a plant family selected from the group consisting of *Cupressaceae, Arecaceae, Asparagaceae, Iridaceae, Bromeliaceae, Poaceae, Musaceae, Zingiberaceae, Apiaceae, Araliaceae, Asteraceae, Brassicaceae, Amaranthaceae, Caryophyllaceae, Polygonaceae, Cucurbitaceae, Actinidiaceae, Lecythidaceae, Theaceae, Fabaceae, Betulaceae, Fagaceae, Juglandaceae, Myricaceae, Nothofagaceae, Ticodendraceae, Apocynaceae, Rubiaceae, Oleaceae, Pedaliacae, Plantaginaceae, Lauraceae, Malvaceae, Euphorbiaceae, Lythraceae, Platanaceae, Cannabaceae, Rosaceae, Ulmaceae, Urticaceae, Anacardiaceae, Rutaceae, Sapindaceae, Solanaceae* and *Vitaceae.*
- wherein the polypeptide is a profilin of a profilin-containing plant material of a plant genus selected from the group consisting of *Chamaecyparis, Cryptomeria, Cupressus, Juniperus, Phoenix, Asparagus, Crocus, Ananas, Anthoxanthum, Cynodon, Dactylis, Festuca, Holcus, Hordeum, Lolium, Oryza, Paspalum, Phalaris, Phleum, Poa, Secale, Sorghum, Triticum, Zea, Musa, Apium, Daucus, Ambrosia, Artemisia, Helianthus, Lactuca, Arabidopsis, Brassica, Sinapis, Amaranthus, Beta, Chenopodium, Fagopyrum, Salsola, Cucumis, Actinidia, Bertholletia, Arachis, Glycine, Lens, Lupinus, Phaseolus, Pisum, Vigna, Alnus, Betula, Carpinus, Carya, Castanea, Corylus, Fagus, Juglans, Ostrya, Quercus, Catharanthus, Coffea, Fraxinus, Ligustrum, Olea, Plantago, Sesamum, Syringa, Persea, Gossypium, Hevea, Manihot, Mercurialis, Popolus, Ricinus, Sonneratia, Platanus, Fragaria, Humulus, Malus, Morus, Parietaria, Prunus, Pyrus, Rubus, Ziziphus, Anacardium, Citrus, Litchi, Mangifera, Pistacia, Capsicum, Lycopersicon, Solanum* and *Vitis* or is a variant of said profilin.
- wherein the hypersensitivity immune response is caused by a non-profilin allergen of a profilin-containing plant material of a plant genus selected from the group consisting of *Chamaecyparis, Cryptomeria, Cupressus, Juniperus, Phoenix, Asparagus, Crocus, Ananas, Anthoxanthum, Cynodon, Dactylis, Festuca, Holcus, Hordeum, Lolium, Oryza, Paspalum, Phalaris, Phleum, Poa, Secale, Sorghum, Triticum, Zea, Musa, Apium, Daucus, Ambrosia, Artemisia, Helianthus, Lactuca, Arabidopsis, Brassica, Sinapis, Amaranthus, Beta, Chenopodium, Fagopyrum, Salsola, Cucumis, Actinidia, Bertholletia, Arachis, Glycine, Lens, Lupinus, Phaseolus, Pisum, Vigna, Alnus, Betula, Carpinus, Carya, Castanea, Corylus, Fagus, Juglans, Ostrya, Quercus, Catharanthus, Coffea, Fraxinus, Ligustrum, Olea, Plantago, Sesamum, Syringa, Persea, Gossypium, Hevea, Manihot, Mercurialis, Popolus, Ricinus, Sonneratia, Platanus, Fragaria, Humulus, Malus, Morus, Parietaria, Prunus, Pyrus, Rubus, Ziziphus, Anacardium, Citrus, Litchi, Mangifera, Pistacia, Capsicum, Lycopersicon, Solanum* and Vitis.wherein the polypeptide is a profilin having an amino acid sequence selected from the group consisting of SEQ ID NOs: 1-42 or a variant of said profilin.
- wherein the amino acid sequences of SEQ ID NOs: 1-42 do not contain methionine in position 1 at the N-terminal end.
- wherein the polypeptide is a profilin selected from the group consisting of Cry j profilin, Pho d 2, Ana c 1, Cyn d 12, Tri a 12, Mus a 1, Api g 4, Dau c 4, Amb a 8, Art v 4, Hel a 2, Ara t 8, Sin a 4, Ama r 2, Beta v 2, Che e 2, Sal k 4, Act d 9, Cuc m 2, Ara h 5, Gly m 3, Bet v 2, Cor a 2, Ole e 2 , Hev b 8, Mer a 12, Pla a 3, Fra a 4, Mal d 4, Par j 3, Pru av 4, Pru du 4, Pru p 4, Pyr c 4, Cit s 2, Lit c 1, Cap a 2 and Lyc e 1 or a variant of said profilin.
- wherein the hypersensitivity immune response is caused by a non-profilin allergen selected from the group consisting of Cha o 1, Cha o 2, Cry j 1, Cry j 2, Cup a 1, Cup s 1, Cup s 3, Jun a 1, Jun a 2, Jun a 3, Jun o 4, Jun s 1, Jun v 1, Jun v 3, Ana c 2, Ant o 1, Aspa o 1, Cro s 1, Cro s 2, Cyn d 1, Cyn d 7, Cyn d 15, Cyn d 22w, Cyn d 23, Cyn d 24, Dac g 1, Dac g 2, Dac g 3, Dac g 4, Dac g 5, Fes p 4, Hol l 1, Hol l 5, Hor v 1, Hor v 5, Hor v 12, Hor v 15, Hor v 16, Hor v 17, Hor v 21, Lol p 1, Lol p 2, Lol p 3, Lol p 4, Lol p 5, Lol p 11, Mus a 2, Mus a 3, Mus a 4, Mus a 5, Ory s 1, Ory s 12, Pas n 1, Pha a 1, Pha a 5, Phl p 1, Phl p 2, Phl p 4, Phl p 5, Phl p 6, Phl p 7, Phl p 11, Phl p 13, Pho d 2, Poa p 1, Poa p 5, Sec c 1, Sec c 5, Sec c 20, Sor h 1, Tri a 14, Tri a 15, Tri a 18, Tri a 19, Tri a 21, Tri a 25, Tri a 26, Tri a 27, Tri a 28, Tri a 29, Tri a 30, Tri a 31, Tri a 32, Tri a 33, Tri a 34, Tri a 35, Tri a 36, Tri a 37, Zea m 1, Zea m 12, Zea m 14, Zea m 25, Act c 5, Act c 8, Act c 10, Act d 1, Act d 2, Act d 3, Act d 4, Act d 5, Act d 6, Act d 7, Act d 8, Act d 10, Act d 11, Aln g 1, Aln g 4, Amb a 1, Amb a 2, Amb a 3, Amb a 4, Amb a 5, Amb a 6, Amb a 7, Amb a 9, Amb a 10, Amb p 5, Amb t 5, Ana o 1, Ana o 2, Ana o 3, Api g 1, Api g 2, Api g 3, Api g 5, Api g 6, Ara h 1, Ara h 2, Ara h 3, Ara h 4, Ara h 6, Ara h 7, Ara h 9, Ara h 10, Ara h 11, Art v 1, Art v 2, Art v 3, Art v 5, Art v 6, Ber e 1, Ber e 2, Beta v 1, Bet v 1, Bet v 3, Bet v 4, Bet v 6, Bet v 7, Bra j 1, Bra n 1, Bra o 3, Bra r 1, Bra r 2, Cap a lw, Car b 1, Car i 1, Car i 4, Cas s 1, Cas s 5, Cas s 8, Cas s 9, Cat r 1, Che a 1, Che a 3, Cit l 3, Cit r 3, Cit s 1, Cit s 3, Cof a 1, Cor a 1, Cor a 8, Cor a 9, Cor a 10, Cor a 11, Cor a 12, Cor a 13, Cor a 14, Cuc m 1, Cuc m 3, Dau c 1, Fag e 2, Fag t 2, Fag s 1, Fra a 1, Fra a 3, Fra e 1, Gly m 1, Gly m 2, Gly m 4, Gly m 5, Gly m 6, Hel a 1, Hel a 3, Hev b 1, Hev b 2, Hev b 3, Hev b 4, Hev b 5, Hev b 6, Hev b 7, Hev b 9, Hev b 10, Hev b 11, Hev b 12, Hev b 13, Hev b 14, Hum j 1, Jug n 1, Jug n 2, Jug r 1, Jug r 2, Jug r 3, Jug r 4, Lac s 1, Len c 1, Len c 2, Len c 3, Lig v 1, Lup an 1, Lyc e 2, Lyc e 3, Lyc e 4, Mal d 1, Mal d 2, Mal d 3, Man e 5, Mer a 1, Mor n 3, Ole e 1, Ole e 3, Ole e 4, Ole e 5, Ole e 6, Ole e 7, Ole e 8, Ole e 9, Ole e 10, Ole e 11, Ost c 1, Par j 1, Par j 2, Par j 4, Par o 1, Pers a 1, Pha v 3, Pis v 1, Pis v 2, Pis v 3, Pis v 4, Pis v 5, Pis s 1, Pis s 2, Pla l 1, Pla a 1, Pla a 2, Pla or 1, Pla or 2, Pla or 3, Pru ar 1, Pru ar 3, Pru av 1, Pru av 2, Pru av 3, Pru d 3, Pru du 3, Pru du 5, Pru du 6, Pru p 1, Pru p 2, Pru p 3, Pyr c 1, Pyr c 3, Pyr c 5, Que a 1, Ric c 1, Rub i 1, Rub i 3, Sal k 1, Sal k 2, Sal k 3, Sal k 5, Ses i 1, Ses i 2, Ses i 3, Ses i 4, Ses i 5, Ses i 6, Ses i 7, Sin a 1, Sin a 2, Sin a 3, Sola t 1, Sola t 2, Sola t 3, Sola t 4, Syr v 1, Syr v 3, Vig r 1, Vit v 1 and Ziz m 1.
- wherein the profilin variant has insertions, substitutions, deletions, duplications, insertion-deletions, frame shifts, transversions, truncations, and/or inversions at one or more locations in comparison to a naturally occurring profilin of any one of the preceeding embodiments.
- wherein the profilin variant is made from said naturally occurring profilin by subjecting 1, 2, 3, 4, 5, 6, 7, 8, 9, 10, 15, 20, 25, 30, 35, 40, 45 or 50 amino acids of the naturally occurring profilin of any one of the preceeding embodiments to substitution, deletion, duplication, insertion-deletion, frame shift, transversions, truncations, and/or inversions.

- wherein the profilin variant of any one of the embodiments mentioned herein has an amino acid sequence having at least 60% sequence identity, such as at least 70%, 75%, 80% ,85%, 90% or 95% sequence identity with SEQ ID NO: 1 or alternatively SEQ ID NOs: 2-42.
- wherein the polypeptide or the variant thereof (profilin variant) comprises an amino acid sequence of SEQ ID NO: 43.
- wherein the polypeptide or the variant thereof (profiling variant) comprises an amino acid sequence of SEQ ID NO: 43 and has at least 60% sequence identity, such as at least 70%, 75%, 80% ,85%, 90% or 95% sequence identity, to SEQ ID NO: 1.
- wherein the variant is a variant of SEQ ID NOs: 1-42 or any other naturally occurring profilin mentioned herein, wherein 1 and up to 25 amino acids are added or deleted in comparison to the parent amino acid sequence of SEQ ID NOs: 1-42 or said naturally occurring profilin.
- wherein the variant is a variant of SEQ ID NOs: 1-42 or any other naturally occurring profilin mentioned herein, wherein 1 and up to 25 amino acids are substituted with another amino acid in comparison to the parent amino acid sequence of SEQ ID NOs: 1-42 or said naturally occurring profilin.
- wherein the variant has at least 60% sequence identity, such as at least 70%, 75%, 80% ,85%, 90% or 95% sequence identity, to SEQ ID NO: 1 and comprises the amino acids corresponding to K87 and G113 of SEQ ID NO: 1.
- wherein the variant has at least 60% sequence identity, such as at least 70%, 75%, 80% ,85%, 90% or 95% sequence identity to SEQ ID NO: 1 and comprises the amino acids corresponding to W3, Y6, 125, G27, W33, A34, Y125 and L126 of SEQ ID NO: 1.
- wherein the variant polypeptide has at least 60% sequence identity, such as at least 70%, 75%, 80% ,85%, 90% or 95% sequence identity, to SEQ ID NO: 1 and comprises the amino acids corresponding to A23, A24, E46, G64, G69 and T97 of SEQ ID NO: 1.
- wherein the variant polypeptide has at least 60% sequence identity, such as at least 70%, 75%, 80% ,85%, 90% or 95% sequence identity, to SEQ ID NO: 1 and comprises an amino acid selected from A, G or S in a position corresponding to position 13 and/or 115 of SEQ ID NO: 1.
- wherein the variant polypeptide has at least 60% sequence identity, such as at least 70%, 75%, 80% ,85%, 90% or 95% sequence identity, to SEQ ID NO: 1 and has all cysteine residues of the parent polypeptide substituted with an amino acid selected from A, G and/or S.
- wherein the polypeptide or the variant thereof has an amino acid chain length ranging between 65 and 195 amino acids.
- wherein the number of amino acids in the amino acid sequence of the variant is in the range of 50% to 150% compared to the number of amino acids of the parent amino acid sequence of SEQ ID NOs: 1-42 or a naturally occurring profilin mentioned herein.
- wherein the polypeptide or the variant thereof is modified by glycosylation, acetylation, alkylation, biotinylation, glutamylation, glycylation, isoprenylation, lipoylation, phosphopantetheinylation, phosphorylation, sulfation, selenation, C-terminal amidation and thiol group derivatizing.
- wherein the polypeptide or the variant thereof is an immunogenic polypeptide.
- wherein the polypeptide or the variant thereof binds to or complexes with poly-L-proline.
- wherein the polypeptide or the variant thereof binds or complexes to actin.
- wherein the polypeptide or the variant thereof does not bind to or does not complex with human actin.
- wherein the polypeptide is co-extractable with a non-profilin allergen of the profilin-containing plant material when extracting the profilin-containing plant material in an aqueous solution having pH in the range of 6-8 for a period ranging from 1 to 30 minutes.
- wherein the polypeptide is obtainable by a method comprising isolating a profilin from said profilin-containing plant material or recombinantly producing said profilin, wherein said profilin has been identified in an extract made by suspending the profilin-containing plant material in an aqueous solution having pH in the range of 6-8 for a period ranging from 1 to 30 minutes.
- wherein the hypersensitivity immune response is a type 1 hypersensitivity immune response.
- wherein the hypersensitivity immune response is selected from the group consisting of atopic dermatitis, urticaria, contact dermatitis, allergic conjunctivitis, allergic rhinitis, allergic asthma, anaphylaxis and food allergy.
- wherein the polypeptide is administered to a mucosa or epithelia selected from the group consisting of a mucosa or epithelia of the respiratory tract, gastroinstestinal tract and oral cavity.
- wherein the polypeptide is administered by inhalation, nasal administration, buccal administration, oral administration, sublingual administration.
- wherein the first dose of said polypeptide is administered by inhalation, nasal administration, buccal administration, oral administration, sublingual administration.
- wherein the polypeptide is administered by inhalation, nasal administration, buccal administration, oral administration, sublingual administration in a period sufficient to establish oral tolerance to said polypeptide.
- wherein the polypeptide is additionally administered to the target organ subject to the natural exposure of a profilin-containing plant material, wherein the target organ is selected from the group consisting of the respiratory tract, gastro-intestinal tract and skin, within a period at least coinciding partly or entirely with the individual's natural exposure to said profilin-containing plant material.
- wherein the polypeptide is administered by sublingual administration in a period sufficient to establish oral tolerance to said polypeptide and wherein the polypeptide or a variant thereof subsequently is administered to the nasal cavity, gastro-intestinal tract and/or skin in a period simultaneously, contemporaneously, separately or sequentially, in either order, to the period of exposure to the profilin-containing plant material.
- wherein the hypersensitivity immune response is not caused by a profilin of said profilin-containing plant material.
- wherein the individual is, at least not before administering the first dose of said polypeptide, sensitized to a profilin of said profilin-containing plant material and/or said polypeptide
- wherein the individual is, at least not before administering the first dose of said polypeptide, neither sensitized to a profilin of said profilin-containing plant material nor to said polypeptide.
- wherein the polypeptide or the profilin of the profilin-containing plant material does not induce a hypersensitivity immune response in the individual, at least not before administering the first dose of polypeptide
- wherein the polypeptide or the profilin of the profilin-containing plant material does not induce, at least not before administering the first dose of polypeptide, an immediate skin reaction and/or delayed skin reaction in the individual upon conducting skin prick testing with various concentrations of the profilin of the profilin-containing plant material and/or said polypeptide.
- wherein the polypeptide or the profilin of the profilin-containing plant material does not induce, at least not before administering the first dose of polypeptide, histamine release in an in-vitro basophil/mast cell assay using blood from the individual to be treated.
- wherein the individual has been exposed to a profilin-containing plant material previous to administering the first dose of said polypeptide.
- wherein the individual is not sensitized to a non-profilin allergen of the profilin-containing plant material, at least not before administration of the first dose of said polypeptide.
- wherein the individual does not have detectable serum IgE antibodies capable of binding to or otherwise associate with a non-profilin allergen of the profilin-containing material, at least not before administering the first dose of said polypeptide.
- wherein the individual does not have clinical symptoms of a hypersensitivity immune response when exposed to a profilin-containing plant material, at least not before administering the first dose of said polypeptide.
- wherein the polypeptide is the only polypeptide administered.
- wherein the polypeptide is not co-administered with a non-profilin allergen.
- A polypeptide having an amino acid sequence of SEQ ID NOs: 1, 2, 3, 4, 5, 6, 7, 8, 9 or 10, wherein 1 or 2 cysteine residue(s) is/are substituted by an amino acid selected from A (alanine), G (glycine) and S (serine).
- A polypeptide having an amino acid sequence of SEQ ID NOs: 1, 2, 3, 4, 5, 6, 7, 8, 9 or 10, where the amino acid cysteine in position 13 and/or 115 has been substituted by an amino acid selected from the group comprising A, G and/or S.
- A polypeptide having an amino acid sequence of SEQ ID NOs: 44 or 45, optionally wherein the amino acid sequence does not contain methionine in position 1.
- An isolated nucleic acid encoding the protein of SEQ ID NOs: 44 or 45.
- wherein the isolated nucleic acid has SEQ ID NOs: 46 or 47.

### List of Tables

**Table 1**

| *Plantae Pinopsida* | | |
|---|---|---|
| Order [Family] | Genus | Species [profilin name] |
| *Pinales [Cupressaceae and Pinaceae]* | *Chamaecyparis, Cryptomeria, Cupressus, Juniperus Picea Pinus* | *Chamaecyparis obtusa* (Japanese cypress), *Cryptomeria japonica* (Japanese Cedar) [Cry j profilin), *Cupressus arizonica* (Cypress), *Cupressus sempervirens* (Common cypress), *Juniperus ashei* (Mountain cedar), *Juniperus oxycedrus* (Prickly juniper), *Juniperus sabinoides* (Mountain cedar) and *Juniperus virginiana* (Eastern red cedar), *Picea sitchencis* (Pine), *Pinus sylvestris* (Pine) |

**Table 2**

| *Plantae Monocots* | | |
|---|---|---|
| *Order [Family]* | *Genus* | *Species [profilin name]* |
| Arecales [Arecaceae] | *Elais, Phoenix* | *Elais guineensis* (Palm tree); *Phoenix dactylifera* (date palm) [Pho d 2] |
| *Asparagales [Asparaqaceae and Iridaceae]* | *Asparagus* | *Asparagus officinalis* (Asparagus) |
| | *Crocus* | *Crocus sativus* (Saffron crocus) [Cro s 2] |
| *Poales [*Bromeliaceae, *and Poaceae]* | *Ananas, Anthoxanthum, Cynodon, Dactylis, Festuca, Holcus, Hordeum, Lolium, Oryza, Paspalum,* | *Ananas comosus* (Pineapple) [Ana c 1], *Anthoxanthum odoratum* (Sweet vernal grass), *Cynodon dactylon* (Bermuda grass) [Cyn d 12], *Dactylis glomerata* (Orchard grass), *Festuca pratensis* (Meadow fescue), *Holcus lanatus* (Velvet grass), *Hordeum vulgare* (Barley), *Lolium perenne* (Rye grass), *Oryza sativa* (Rice), *Paspalum notatum* (Bahia grass), *Phalaris aquatica* (Canary grass), |
| | *Phalaris, Phleum, Poa, Secale,* | *Phleum pratense* (Timothy) [Phl p 12], *Poa pratensis* (Kentucky blue grass), *Secale cereale* (Rye), |
| | *Sorghum, Triticum, Zea* | *Sorghum halepense* (Johnson grass), *Triticum aestivum* (Wheat) [Tri a 12], *Zea mays* (Maize) |
| *Zingiberales [*Musaceae*a and* Zingiberaceae*)* | *Musa* | *Musa acuminata* (Banana)[Mus a 1], *Musa x paradisiaca* (Banana)[Mus xp 1], |

**Table 3**

| *Plantae Magnoliopsida* | | |
|---|---|---|
| *Order [Family]* | *Plant Genus* | *Plant Species [profilin name]* |
| *Apiales [Apiaceae and Araliaceae]* | *Apium, Daucus* | *Apium graveolens* (Celery) [Api g 4], *Daucus carota* (Carrot) [Dau c 4], *Petroselinum crispum* (Parsley) |
| *Asterales [Asteraceae]* | *Ambrosia, Artemisia, Helianthus, Lactuca* | *Ambrosia artemisiifolia* (Short ragweed) [Amb a 8], *Ambrosia psilostachya* (Western ragweed), *Artemisia vulgaris* (Mugwort) [Art v *4], Ambrosia trifida* (Giant ragweed), *Helianthus annuus* (Sunflower) [Hel a *2], Lactuca sativa* (Cultivated lettuce) |
| *Brassicalis [Brassicaceae, may include the Cleomaceae, and Caricaceae* | *Arabidopsis, Brassica, Sinapsis* | *Arabidopsis Lyrata, Arabidopsis thaliana* [Ara t 8], *Brassica juncea* (Oriental mustard), *Brassica napus* (Rape seed), *Brassica oleracea* (Cabbage and others), *Brassica rapa* (Turnip) and *Sinapis alba* (Yellow mustard) [Sin a 4] |
| *Caryophyllales [Amaranthaceae, Caryophyllaceae and Polygonaceae]* | *Amaranthus, Beta, Chenopodium, Fagopyrum Salsola* | *Amaranthus retroflexus* (Redroot pigweed) [Ama r 2], *Beta vulgaris* (Sugar beet) [Beta v 2], *Chenopodium album* (Pigweed) [Che e 2], *Fagopyrum esculentum* (Common buckwheat), *Fagopyrum tataricum* (Tartarian buckwheat), *Salsola kali* (Russian thistle) [Sal k 4] |
| *Curcurbitales [Cucurbitaceae]* | *Cucumis* | *Cucumis melo* (Musk melon) [Cuc m 2] |
| *Order Ericales [Actinidiaceae*, *Lecythidaceae, and Theaceae]* | *Actinidia, Betholletia* | *Actinidia chinensis* (Gold Kiwi fruit), *Actinidia deliciosa* (Kiwi fruit) [Act d *9], Bertholletia excelsa* (Brazil nut) |
| *Fabales [Fabaceae,* | *Arachis, Glycine, Lens,* | *Arachis hypogaea* (Peanut) [Ara h 5], |
| *Quillajaceae, Polygalaceae, and Surianaceae]* | *Lupinus, Phaseolus* | *Glycine max* (Soybean) [Gly m 3], *Lens culinaris* (Lentil), *Lupinus angustifolius* (Narrow-leaved blue lupin), *Phaseolus vulgaris* (Green bean, French bean), *Pisum sativum* (Pea), *Vigna radiata* (Mung bean) |
| *Fagales [Betulaceae, Fagaceae, Juglandaceae, Myricaceae, Nothofagaceae and Ticodendraceae]* | *Alnus, Betula, Carpinus, Carya, Castanea, Corylus, Fagus, Juglans, Ostrya Quercus* | *Alnus glutinosa* (Alder), *Betula verrucosa* (Birch) [Bet v 2], *Carpinus betulus* (Hornbeam), *Carya illinoinensis* (Pecan), *Castanea sativa* (Chestnut), *Corylus avellana* (Hazel) [Cor a 2], *Fagus sylvatica* (European beech), *Juglans nigra* (Black walnut), *Juglans regia* (English walnut), *Ostrya carpinifolia* (European hophornbeam), *Quercus alba* (White oak) |
| *Gentianales [*Apocynaceae *and* Rubiaceae*]* | *Catharanthus, Coffea* | *Catharanthus roseus* (Rosy periwinkle), *Coffee arabica* (Arabian coffee) |
| *Lamiales [Oleaceae, Pedaliacae and Plantaginaceae]* | *Fraxinus, Ligustrum, Olea, Plantago, Sesamum Syringa,* | *Fraxinus excelsior* (Ash), *Ligustrum vulgare* (Privet), *Olea europea* (Olive) [Ole e 2], *Plantago lanceolata* (English plantain), *Sesamum indicum* (Sesame), *Syringa vulgaris* (Lilac) |
| *Laurales [Lauraceae]* | *Persea* | *Persea americana* (Avocado) |
| *Malvales [Malvaceae]* | *Gossypium* | *Gossypium arboretum, Gossypium barbadense, Gossypium darwinii, Gossypium herbaceum, Gossypium hirsutism, Gossypium raimondii* |
| *Malpighiales [Euphorbiaceae]* | *Hevea, Manihot, Mercurialis, Popolus, Ricinus* | *Hevea brasiliensis* (Para rubber tree (latex) [Hev b 8], *Manihot esculenta* (Cassava, manioc), *Mercurialis annua* (Annual mercury) [Mer a 1], *Popolus trichocarpa* (Poplar) *Ricinus communis* (Castor bean) |
| *Myrtales [*Lythraceae] | *Sonneratia* | *Sonneratia alba, Sonneratia caseolaris* |
| *Proteales [Platanaceae]* | *Platanus* | *Platanus orientalis* (Oriental plane), *Platanus acerifolia* (London plane tree)[Pla a 3] |
| *Rosales [*Cannabaceae, Rosaceae, Ulmaceae *and* | *Fragaria, Humulus,Malus, Morus, Parietaria, Prunus,* | *Fragaria ananassa* (Strawberry) [Fra a 4], *Humulus japonicus* (Japanese hop), *Malus domestica* (Apple) [Mal d |
| Urticaceae*]* | *Pyrus, Rubus, Ziziphus* | *4], Morus nigra* (Mulberry), *Parietaria judaica* (Pellitory-of-the-Wall) [Par j *3], Parietaria officinalis* (Pellitory), *Prunus armeniaca* (Apricot), *Prunus avium* (Sweet cherry) [Pru av 4], *Prunus domestica* (European plum), *Prunus dulcis* (Almond) [Pru du 4], *Prunus persica* (Peach) [Pru p 4], *Pyrus communis* (Pear) [Pyr c 4], *Rubus idaeus* (Red raspberry), *Ziziphus mauritiana* (Chinese date) |
| *Sapindales [Anacardiaceae, Rutaceae and Sapindaceae]* | *Anacardium, Citrus, Litchi, Mangifera, Pistacia* | *Anacardium occidentale* (Cashew), *Citrus limon* (Lemon), *Citrus reticulata* (Tangerine), *Citrus sinensis* (Sweet orange) [Cit s 2], *Litchi chinensis* (Litchi) [Lit c 1], *Mangifera indica, Pistacia vera* (Pistachio) |
| *Solanales [Solanaceae]* | *Capsicum, Lycopersicon, Solanum* | *Capsicum annuum* (Bell pepper) [Cap a 2], *Lycopersicon esculentum* (Tomato) [Lyc e 1], *Nicotiana tabacum* (Common tobacco), *Solanum tuberosum* (Potato) |
| *Order Vitales, [Vitaceae]* | *Vitis* | *Vitis* vinifera (Grape) |

**Table 4**

| **Entry** | **Entry name** | **Plant** | **Identity** | **E-value** |
|---|---|---|---|---|
| Q68HB4 | PROF2_PHLPR | Phleum pratense (Common timothy) | 100.00% | 3.00E-72 |
| F4Q4X7 | PROF1_PHLPR | Phleum pratense (Common timothy) | 99.00% | 1.00E-71 |
| F0ZIL9 | PROF3 PHLPR | Phleum pratense (Common timothy) | 97.00% | 2.00E-70 |
| B7SA43 | A4KA31_PHLPR | Phleum pratense (Common timothy) | 96.00% | 1.00E-69 |
| B6EF35 | A4KA32_PHLPR | Phleum pratense (Common timothy) | 96.00% | 3.00E-70 |
| A8IJA8 | A4KA33_PHLPR | Phleum pratense (Common timothy) | 96.00% | 2.00E-70 |
| Q5VMJ3 | A4KA34_PHLPR | Phleum pratense (Common timothy) | 96.00% | 3.00E-70 |
| P83647 | A4KA36_PHLPR | Phleum pratense (Common timothy) | 96.00% | 7.00E-70 |
| Q84WD4 | A4KA37_PHLPR | Phleum pratense (Common timothy) | 96.00% | 1.00E-69 |
| D0PRB5 | A4KA38_PHLPR | Phleum pratense (Common timothy) | 96.00% | 2.00E-69 |
| A4KA61 | A4KA49_OLEEU | Olea europaea (Common olive) | 96.00% | 1.OOE-69 |
| A4KA54 | A4KA50_OLEEU | Olea europaea (Common olive) | 96.00% | 6.00E-70 |
| Q9M7M8 | A4KA52_OLEEU | Olea europaea (Common olive) | 96.00% | 1.OOE-69 |
| Q2PQ57 | A4KA53_OLEEU | Olea europaea (Common olive) | 96.00% | 6.00E-70 |
| G9B5Q4 | G9I6G4_9POAL | Triticum turgidum subsp. durum x Secale cereale | 96.00% | 6.00E-70 |
| Q9XF10 | F2EK80_HORVD | Hordeum vulgare var. distichum (Two-rowed barley) | 95.00% | 8.00E-69 |
| B4FTX3 | A4GFC3_OLEEU | Olea europaea (Common olive) | 94.00% | 3.00E-67 |
| P49233 | A4KA43_CORAV | Corylus avellana (European hazel) (Corylus maxima) | 94.00% | 2.00E-68 |
| A1KXK1 | A4KA51_OLEEU | Olea europaea (Common olive) | 94.00% | 2.00E-68 |
| D6BRE6 | A4GFC0_OLEEU | Olea europaea (Common olive) | 93.00% | 3.00E-67 |
| 022655 | A4GFB8_OLEEU | Olea europaea (Common olive) | 90.00% | 3.00E-64 |
| Q9SNW5 | F2CT70_HORVD | Hordeum vulgare var. distichum (Two-rowed barley) | 90.00% | 6.00E-66 |
| A4KA37 | A2Z5Y9_ORYSI | Oryza sativa subsp. indica (Rice) | 89.00% | 4.00E-65 |
| A4KA31 | A3C3I5_ORYSJ | Oryza sativa subsp. japonica (Rice) | 89.00% | 4.00E-65 |
| F5HFQ1 | PROFA_ORYSJ | Oryza sativa subsp. japonica (Rice) | 89.00% | 4.00E-65 |
| Q5EF31 | C5XJ77_SORBI | Sorghum bicolor (Sorghum) (Sorghum vulgare) | 87.00% | 1.00E-64 |
| Q9ST98 | C5Z1D7_SORBI | Sorghum bicolor (Sorghum) (Sorghum vulgare) | 87.00% | 4.00E-65 |
| A4GDU2 | B4FCP0_MAIZE | Zea mays (Maize) | 86.00% | 7.00E-63 |
| B0D663 | Q6RG01_CAPAN | Capsicum annuum (Bell pepper) | 86.00% | 2.00E-19 |
| A8IJ92 | A4KA58 MAIZE | Zea mays (Maize) | 85.00% | 2.00E-62 |
| C6SVT2 | A5HNY4_MAIZE | Zea mays (Maize) | 85.00% | 3.00E-62 |
| Q93YI9 | B6T699_MAIZE | Zea mays (Maize) | 85.00% | 7.00E-63 |
| A1BQK7 | PROF3_MAIZE | Zea mays (Maize) | 85.00% | 7.00E-63 |
| A8IJA4 | A4KA55_MAIZE | Zea mays (Maize) | 84.00% | 2.00E-62 |
| A8IJA0 | A4KA56_MAIZE | Zea mays (Maize) | 84.00% | 6.00E-62 |
| P0C0Y3 | A4KA59_MAIZE | Zea mays (Maize) | 84.00% | 4.00E-62 |
| Q3BCT0 | B6TEN9_MAIZE | Zea mays (Maize) | 84.00% | 7.00E-62 |
| Q9XG85 | F2E5Q1_HORVD | Hordeum vulgare var. distichum (Two-rowed barley) | 84.00% | 3.00E-62 |
| Q9FE63 | G7KQH7_MEDTR | Medicago truncatula (Barrel medic) (Medicago tribuloides) | 84.00% | 2.00E-61 |
| ABIJB6 | A4KA54_OLEEU | Olea europaea (Common olive) | 83.00% | 5.00E-60 |
| Q84V37 | A4KA60_MAIZE | Zea mays (Maize) | 83.00% | 5.00E-61 |
| P49231 | A4KA61_MAIZE | Zea mays (Maize) | 83.00% | 5.00E-60 |
| Q9XF40 | B6T6Y5_MAIZE | Zea mays (Maize) | 83.00% | 6.00E-62 |
| A4GCS1 | E2GJB9_WHEAT | Triticum aestivum (Wheat) | 83.00% | 7.00E-62 |
| G9B5Q0 | G9I6G5_9POAL | Triticum turgidum subsp. durum x Secale cereale | 83.00% | 3.00E-62 |
| B3H795 | PROF_CYNDA | Cynodon dactylon (Bermuda grass) (Panicum dactylon) | 83.00% | 1.00E-62 |
| A8PUB8 | PROF1_HORVU | Hordeum vulgare (Barley) | 83.00% | 5.00E-61 |
| Q29PL9 | PROF1_MAIZE | Zea mays (Maize) | 83.00% | 5.00E-63 |
| B4JC19 | PROF2_MAIZE | Zea mays (Maize) | 83.00% | 6.00E-62 |
| C5P4B7 | Q38HU3_SOLTU | Solanum tuberosum (Potato) | 83.00% | 1.00E-61 |
| B8RIF3 | Q38M47_SOLTU | Solanum tuberosum (Potato) | 83.00% | 3.00E-62 |
| A8IJ96 | A4KA57_MAIZE | Zea mays (Maize) | 82.00% | 5.00E-61 |
| A4GFC4 | A4ZX86_BRACM | Brassica campestris (Field mustard) | 82.00% | 3.00E-61 |
| D3K177 | A5H0M3_NICBE | Nicotiana benthamiana | 82.00% | 1.00E-46 |
| Q41344 | A8IJ88_RAPSA | Raphanus sativus (Radish) | 82.00% | 2.00E-61 |
| 081982 | A8IJB2_BRAJU | Brassica juncea var. multiceps | 82.00% | 2.00E-61 |
| A4GD57 | C0PNL3_MAIZE | Zea mays (Maize) | 82.00% | 4.00E-62 |
| A9XNJ7 | PROF_HELAN | Helianthus annuus (Common sunflower) | 82.00% | 3.00E-59 |
| A9XNJ5 | PROF_MERAN | Mercurialis annua (Annual mercury) | 82.00% | 1.00E-61 |
| Q4Q5N1 | Q9SMC0_TOBAC | Nicotiana tabacum (Common tobacco) | 82.00% | 1.00E-60 |
| 024650 | A0MEN1_ARATH | Arabidopsis thaliana (Mouse-ear cress) | 81.00% | 1.00E-61 |
| A4KA32 | A1KXK0_ELAGV | Elaeis guineensis var. tenera (Oil palm) | 81.00% | 9.00E-60 |
| G9I6G4 | A1KXK1_ELAGV | Elaeis guineensis var. tenera (Oil palm) | 81.00% | 5.00E-60 |
| Q8L5D8 | A4KA44_CORAV | Corylus avellana (European hazel) (Corylus maxima) | 81.00% | 8.00E-59 |
| A5A5J9 | B7FGT8_MEDTR | Medicago truncatula (Barrel medic) (Medicago tribuloides) | 81.00% | 1.00E-59 |
| 024171 | C5Z4B6_SORBI | Sorghum bicolor (Sorghum) (Sorghum vulgare) | 81.00% | 5.00E-59 |
| A4GDS0 | DOPRB5_WHEAT | Triticum aestivum (Wheat) | 81.00% | 3.00E-60 |
| G7L7E2 | PROF_BRANA | Brassica napus (Rape) | 81.00% | 2.00E-60 |
| B4NZS9 | PROF1_SOLLC | Solanum lycopersicum (Tomato) (Lycopersicon esculentum) | 81.00% | 3.00E-59 |
| B4I1N3 | PROF1_TOBAC | Nicotiana tabacum (Common tobacco) | 81.00% | 2.00E-59 |
| C3YHE9 | PROF3_AMBAR | Ambrosia artemisiifolia (Short ragweed) | 81.00% | 2.00E-59 |
| Q94KS3 | PROF3_ARATH | Arabidopsis thaliana (Mouse-ear cress) | 81.00% | 1.00E-60 |
| C1BUS4 | PROF3_HEVBR | Hevea brasiliensis (Para rubber tree) (Siphonia brasiliensis) | 81.00% | 1.00E-62 |
| A5J297 | PROF4_ARATH | Arabidopsis thaliana (Mouse-ear cress) | 81.00% | 1.00E-61 |
| D3BLB6 | Q1PF40_ARATH | Arabidopsis thaliana (Mouse-ear cress) | 81.00% | 1.00E-61 |
| F8QBC0 | Q29PU0_ARATH | Arabidopsis thaliana (Mouse-ear cress) | 81.00% | 1.00E-60 |
| A4KA34 | A1KXJ9_ELAGV | Elaeis guineensis var. tenera (Oil palm) | 80.00% | 6.00E-59 |
| B6T699 | A4GDQ6_OLEEU | Olea europaea (Common olive) | 80.00% | 8.00E-59 |
| Q38M47 | A4GDR8_OLEEU | Olea europaea (Common olive) | 80.00% | 5.00E-59 |
| A9PHX4 | A4GFC4_OLEEU | Olea europaea (Common olive) | 80.00% | 9.00E-60 |
| B7E355 | A4KA39_CORAV | Corylus avellana (European hazel) (Corylus maxima) | 80.00% | 2.00E-58 |
| P49232 | A4KA40_CORAV | Corylus avellana (European hazel) (Corylus maxima) | 80.00% | 2.00E-58 |
| B6T7X9 | A4KA45_CORAV | Corylus avellana (European hazel) (Corylus maxima) | 80.00% | 6.00E-59 |
| Q9AXH4 | A8IJ92_BRAOC | Brassica oleracea var. capitata (Cabbage) | 80.00% | 7.00E-60 |
| Q5EEP6 | A8IJ96_BRARA | Brassica rapa (Turnip) | 80.00% | 7.00E-60 |
| G7L7D9 | A8IJA0_BRAOA | Brassica oleracea var. alboglabra (Chinese kale) (Brassica alboglabra) | 80.00% | 7.00E-60 |
| B4FV68 | A8IJA8_BRARC | Brassica rapa var. purpuraria | 80.00% | 2.00E-60 |
| Q64LH2 | A8IJB6_BRANA | Brassica napus (Rape) | 80.00% | 7.00E-60 |
| Q5EEP8 | A9XNJ4_9MYRT | Sonneratia alba | 80.00% | 1.00E-26 |
| A4KA44 | A9XNJ5_9MYRT | Sonneratia caseolaris | 80.00% | 1.00E-26 |
| A4GDQ6 | A9XNJ6_9MYRT | Sonneratia ovata | 80.00% | 1.00E-26 |
| Q9M7M9 | A9XNJ7_9MYRT | Sonneratia apetala | 80.00% | 1.00E-26 |
| A4GDQ9 | B4FTX3_MAIZE | Zea mays (Maize) | 80.00% | 2.00E-60 |
| A4GDR7 | B6EF35_WHEAT | Triticum aestivum (Wheat) | 80.00% | 2.00E-60 |
| Q5EEP7 | B6T7X9_MAIZE | Zea mays (Maize) | 80.00% | 5.00E-60 |
| C9E9Z7 | B7E355_ORYSJ | Oryza sativa subsp. japonica (Rice) | 80.00% | 3.00E-60 |
| A4GE44 | B7SA43_BRANI | Brassica nigra (Black mustard) (Sinapis nigra) | 80.00% | 2.00E-60 |
| A4GE47 | C3W2Q7_AMARE | Amaranthus retroflexus (Redroot amaranth) (Redroot pigweed) | 80.00% | 9.00E-58 |
| A4GDT9 | D7L6H0_ARALL | Arabidopsis lyrata subsp. lyrata (Lyre-leaved rock-cress) | 80.00% | 3.00E-61 |
| A4GCR5 | D7MCM9_ARALL | Arabidopsis lyrata subsp. lyrata (Lyre-leaved rock-cress) | 80.00% | 2.00E-60 |
| G9B5S5 | PROF_CHEAL | Chenopodium album (Lamb's-quarters) | 80.00% | 9.00E-60 |
| A9XNJ6 | PROF_LITCN | Litchi chinensis (Lychee) | 80.00% | 5.00E-60 |
| P25843 | PROF1_OLEEU | Olea europaea (Common olive) | 80.00% | 1.00E-58 |
| E2APZ9 | PROF1_WHEAT | Triticum aestivum (Wheat) | 80.00% | 4.00E-60 |
| B4MUZ7 | PROF2_AMBAR | Ambrosia artemisiifolia (Short ragweed) | 80.00% | 3.00E-59 |
| F4PC86 | PROF2_SOLLC | Solanum lycopersicum (Tomato) (Lycopersicon esculentum) | 80.00% | 6.00E-59 |
| Q6QEJ7 | PROF2_TOBAC | Nicotiana tabacum (Common tobacco) | 80.00% | 1.00E-58 |
| E9H2U7 | PROF2_WHEAT | Triticum aestivum (Wheat) | 80.00% | 5.00E-60 |
| E7A2J9 | PROF4_MAIZE | Zea mays (Maize) | 80.00% | 2.00E-60 |
| P68696 | PROFX_ORYSI | Oryza sativa subsp. indica (Rice) | 80.00% | 3.00E-60 |
| D3B642 | PROFX_ORYSJ | Oryza sativa subsp. japonica (Rice) | 80.00% | 3.00E-60 |
| Q013H6 | Q2LD52_CINCA | Cinnamomum camphora (Camphor tree) (Laurus camphora) | 80.00% | 1.00E-60 |
| HOEVP8 | Q2PQ57_LITCN | Litchi chinensis (Lychee) | 80.00% | 7.00E-60 |
| B9W8I5 | Q84WD4_ARATH | Arabidopsis thaliana (Mouse-ear cress) | 80.00% | 3.00E-60 |
| C3Z897 | Q8L5D8_PHODC | Phoenix dactylifera (Date palm) | 80.00% | 5.00E-60 |
| A3LQH2 | Q8VWR0_SOLLC | Solanum lycopersicum (Tomato) (Lycopersicon esculentum) | 80.00% | 2.00E-59 |
| G2Q4F1 | Q9ZTM2_PETHY | Petunia hybrida (Petunia) | 80.00% | 2.00E-53 |
| F2EK80 | A4GCR3_0LEEU | Olea europaea (Common olive) | 79.00% | 7.00E-58 |
| A4KA43 | A4GCR6_OLEEU | Olea europaea (Common olive) | 79.00% | 1.00E-57 |
| C5Z1D7 | A4GD52_0LEEU | Olea europaea (Common olive) | 79.00% | 2.00E-58 |
| A3C3I5 | A4GD53_OLEEU | Olea europaea (Common olive) | 79.00% | 7.00E-58 |
| A2Z5Y9 | A4GD54_OLEEU | Olea europaea (Common olive) | 79.00% | 4.00E-58 |
| P35081 | A4GD57_OLEEU | Olea europaea (Common olive) | 79.00% | 7.00E-58 |
| B4FCP0 | A4GDQ8_OLEEU | Olea europaea (Common olive) | 79.00% | 1.00E-57 |
| Q9M7N0 | A4GDQ9_OLEEU | Olea europaea (Common olive) | 79.00% | 2.00E-58 |
| A4KA55 | A4GDR4_OLEEU | Olea europaea (Common olive) | 79.00% | 1.00E-57 |
| A5HNY4 | A4GDR7_OLEEU | Olea europaea (Common olive) | 79.00% | 3.00E-58 |
| C0PNL3 | A4GDSO_OLEEU | Olea europaea (Common olive) | 79.00% | 2.00E-57 |
| A4KA59 | A4GDS6_OLEEU | Olea europaea (Common olive) | 79.00% | 3.00E-58 |
| P35082 | A4GDS7_OLEEU | Olea europaea (Common olive) | 79.00% | 5.00E-58 |
| B6T6Y5 | A4GDS9_OLEEU | Olea europaea (Common olive) | 79.00% | 3.00E-57 |
| E2GJB9 | A4GDT1_OLEEU | Olea europaea (Common olive) | 79.00% | 2.00E-58 |
| A0MEN1 | A4GDU2_OLEEU | Olea europaea (Common olive) | 79.00% | 2.00E-58 |
| ABIJB2 | A4GDU5_OLEEU | Olea europaea (Common olive) | 79.00% | 1.00E-57 |
| P52184 | A4GE42_OLEEU | Olea europaea (Common olive) | 79.00% | 1.00E-57 |
| A4KA60 | A4GE44_OLEEU | Olea europaea (Common olive) | 79.00% | 4.00E-58 |
| Q38904 | A4GE47_OLEEU | Olea europaea (Common olive) | 79.00% | 1.00E-57 |
| Q2LD52 | A4GE50_OLEEU | Olea europaea (Common olive) | 79.00% | 3.00E-58 |
| Q29PU0 | A4GE53_OLEEU | Olea europaea (Common olive) | 79.00% | 3.00E-58 |
| A5ASF9 | A4GE54_OLEEU | Olea europaea (Common olive) | 79.00% | 1.00E-57 |
| Q9SNW7 | A4GE55_OLEEU | Olea europaea (Common olive) | 79.00% | 2.00E-58 |
| Q9FUB8 | A4GFB7_OLEEU | Olea europaea (Common olive) | 79.00% | 6.00E-59 |
| B9RKF5 | A4GFC2_OLEEU | Olea europaea (Common olive) | 79.00% | 2.00E-60 |
| D7MCM9 | A4K9Z8_BETPN | Betula pendula (European white birch) (Betula verrucosa) | 79.00% | 1.00E-58 |
| Q941H7 | A4KA41_CORAV | Corylus avellana (European hazel) (Corylus maxima) | 79.00% | 2.00E-59 |
| A1KXK0 | A5A5J7_OLEEU | Olea europaea (Common olive) | 79.00% | 2.00E-57 |
| B7FGT8 | A5A5J9_OLEEU | Olea europaea (Common olive) | 79.00% | 4.00E-58 |
| B6CQV0 | A5AQ89_VITVI | Vitis vinifera (Grape) | 79.00% | 2.00E-58 |
| Q64LH0 | A5ASF9_VITVI | Vitis vinifera (Grape) | 79.00% | 1.00E-60 |
| P41372 | A5BLM8_VITVI | Vitis vinifera (Grape) | 79.00% | 2.00E-58 |
| B9RKF4 | A8IJA4_BRARP | Brassica rapa subsp. pekinensis (Chinese cabbage) (Brassica pekinensis) | 79.00% | 7.00E-60 |
| A4KA45 | A9PHX4_POPTR | Populus trichocarpa (Western balsam poplar) (Populus balsamifera subsp. trichocarpa) | 79.00% | 2.00E-60 |
| P49234 | A9XNJ8_9MYRT | Sonneratia alba | 79.00% | 2.00E-25 |
| Q9ST99 | A9XNJ9_9MYRT | Sonneratia caseolaris | 79.00% | 1.00E-25 |
| A4K9Z8 | A9XNK0_9MYRT | Sonneratia ovata | 79.00% | 1.00E-25 |
| Q5FX67 | B9RKF5_RICCO | Ricinus communis (Castor bean) | 79.00% | 2.00E-60 |
| A9P8K3 | C6SVT2_SOYBN | Glycine max (Soybean) (Glycine hispida) | 79.00% | 2.00E-59 |
| A4GDT5 | D3K177_ARAHY | Arachis hypogaea (Peanut) | 79.00% | 2.00E-59 |
| A4GE48 | D6BRE6_9ROSI | Jatropha curcas | 79.00% | 2.00E-60 |
| A4GDU3 | E4MXD3_THEHA | Thellungiella halophila (Salt cress) | 79.00% | 5.00E-58 |
| Q8H2C7 | G7L7D9_MEDTR | Medicago truncatula (Barrel medic) (Medicago tribuloides) | 79.00% | 3.00E-59 |
| Q8H123 | G7L7E2_MEDTR | Medicago truncatula (Barrel medic) (Medicago tribuloides) | 79.00% | 6.00E-35 |
| G9B5S6 | PROF_CAPAN | Capsicum annuum (Bell pepper) | 79.00% | 4.00E-58 |
| Q3LVF0 | PROF_FRAAN | Fragaria ananassa (Strawberry) | 79.00% | 9.00E-60 |
| P26199 | PROF1 AMBAR | Ambrosia artemisiifolia (Short ragweed) | 79.00% | 2.00E-58 |
| E0VF14 | PROF1_LILLO | Lilium longiflorum (Trumpet lily) | 79.00% | 1.00E-60 |
| B3N5A1 | PROF1 PHAVU | Phaseolus vulgaris (Kidney bean) (French bean) | 79.00% | 9.00E-60 |
| Q3YMV5 | PROF1_RICCO | Ricinus communis (Castor bean) | 79.00% | 2.00E-60 |
| D3B3P0 | PROF2_HEVBR | Hevea brasiliensis (Para rubber tree) (Siphonia brasiliensis) | 79.00% | 2.00E-59 |
| D3TQD6 | PROF2_OLEEU | Olea europaea (Common olive) | 79.00% | 7.00E-58 |
| P26200 | PROF3_TOBAC | Nicotiana tabacum (Common tobacco) | 79.00% | 2.00E-57 |
| E9C081 | PROF3_WHEAT | Triticum aestivum (Wheat) | 79.00% | 1.00E-58 |
| C5MCP8 | Q2KN24_AMBAR | Ambrosia artemisiifolia (Short ragweed) | 79.00% | 6.00E-57 |
| A4KA51 | A4GCR5_OLEEU | Olea europaea (Common olive) | 78.00% | 3.00E-57 |
| A4GFC3 | A4GCR7_OLEEU | Olea europaea (Common olive) | 78.00% | 2.00E-57 |
| A4GFC0 | A4GCR8_OLEEU | Olea europaea (Common olive) | 78.00% | 2.00E-57 |
| F2CT70 | A4GCS1_OLEEU | Olea europaea (Common olive) | 78.00% | 6.00E-57 |
| C5XJ77 | A4GD55_OLEEU | Olea europaea (Common olive) | 78.00% | 6.00E-57 |
| A4GFB8 | A4GD56_OLEEU | Olea europaea (Common olive) | 78.00% | 3.00E-57 |
| 004725 | A4GDR1_OLEEU | Olea europaea (Common olive) | 78.00% | 2.00E-57 |
| F2E5Q1 | A4GDR6_OLEEU | Olea europaea (Common olive) | 78.00% | 4.00E-57 |
| G9I6G5 | A4GDR9_OLEEU | Olea europaea (Common olive) | 78.00% | 2.00E-57 |
| A4KA56 | A4GDT0_OLEEU | Olea europaea (Common olive) | 78.00% | 9.00E-58 |
| B6TEN9 | A4GDT3_OLEEU | Olea europaea (Common olive) | 78.00% | 6.00E-57 |
| 049894 | A4GDT5_OLEEU | Olea europaea (Common olive) | 78.00% | 2.00E-57 |
| Q38905 | A4GDT9_OLEEU | Olea europaea (Common olive) | 78.00% | 3.00E-57 |
| Q1PF40 | A4GDU0_OLEEU | Olea europaea (Common olive) | 78.00% | 6.00E-57 |
| A8IJ88 | A4GDU6_OLEEU | Olea europaea (Common olive) | 78.00% | 6.00E-57 |
| D7L6H0 | A4GE38_OLEEU | Olea europaea (Common olive) | 78.00% | 9.00E-58 |
| A4ZX86 | A4GE39_OLEEU | Olea europaea (Common olive) | 78.00% | 1.00E-57 |
| A4KA57 | A4GE45_OLEEU | Olea europaea (Common olive) | 78.00% | 2.00E-57 |
| Q9SMC0 | A4GE48_OLEEU | Olea europaea (Common olive) | 78.00% | 3.00E-57 |
| 082572 | A4GFB9_OLEEU | Olea europaea (Common olive) | 78.00% | 2.00E-59 |
| Q9LEI8 | A7XZJ9_OLEEU | Olea europaea (Common olive) | 78.00% | 8.00E-56 |
| Q9XF38 | A9P7F0_GOSHI | Gossypium hirsutum (Upland cotton) (Gossypium mexicanum) | 78.00% | 2.00E-58 |
| Q3BCS8 | A9PBI2_POPTR | Populus trichocarpa (Western balsam poplar) (Populus balsamifera subsp. trichocarpa) | 78.00% | 2.00E-59 |
| Q9XF41 | B0B0M7_MALDO | Malus domestica (Apple) (Pyrus malus) | 78.00% | 9.00E-60 |
| A4GD52 | B4FV68_MAIZE | Zea mays (Maize) | 78.00% | 3.00E-59 |
| A4GDS6 | B6CQV0_9ROSA | Prunus dulcis x Prunus persica | 78.00% | 1.00E-59 |
| Q5EEP5 | B7VFP6_MALDO | Malus domestica (Apple) (Pyrus malus) | 78.00% | 2.00E-48 |
| A4GDS7 | B9RKF4_RICCO | Ricinus communis (Castor bean) | 78.00% | 3.00E-59 |
| 024170 | B9RQD3_RICCO | Ricinus communis (Castor bean) | 78.00% | 1.00E-58 |
| A5A5J7 | C9E9Z7_9MAGN | Akebia trifoliata | 78.00% | 4.00E-58 |
| A4GCR7 | D3U1G2_GOSRA | Gossypium raimondii (New World cotton) | 78.00% | 2.00E-58 |
| Q27HX6 | D3U1G3_GOSAR | Gossypium arboreum (Tree cotton) (Gossypium nanking) | 78.00% | 2.00E-58 |
| B6CQU8 | E6Y2MO_SINAL | Sinapis alba (White mustard) (Brassica hirta) | 78.00% | 6.00E-57 |
| D7MCM8 | F4YF99_CAMSI | Camellia sinensis (Tea) | 78.00% | 5.00E-58 |
| G9B5S9 | PROF_BETPN | Betula pendula (European white birch) (Betula verrucosa) | 78.00% | 1.00E-57 |
| A9UR87 | PROF1_HEVBR | Hevea brasiliensis (Para rubber tree) (Siphonia brasiliensis) | 78.00% | 7.00E-58 |
| B4KGG2 | PROF2_LILLO | Lilium lonqiflorum (Trumpet lily) | 78.00% | 2.00E-57 |
| F8QWX6 | PROF3_OLEEU | Olea europaea (Common olive) | 78.00% | 2.00E-57 |
| B3RIX1 | PROF5_MAIZE | Zea mays (Maize) | 78.00% | 3.00E-59 |
| B8RIF9 | QOPPS3_SOYBN | Glycine max (Soybean) (Glycine hispida) | 78.00% | 2.00E-58 |
| F8P9Y4 | Q2KN23_AMBAR | Ambrosia artemisiifolia (Short ragweed) | 78.00% | 4.00E-56 |
| E9D690 | Q2XPH2_MANIN | Mangifera indica (Mango) | 78.00% | 5.00E-58 |
| G0N7P8 | Q5EEP5_PETCR | Petroselinum crispum (Parsley) (Petroselinum hortense) | 78.00% | 5.00E-58 |
| F9X7B2 | Q5EEP7_PETCR | Petroselinum crispum (Parsley) (Petroselinum hortense) | 78.00% | 4.00E-58 |
| C7Z4J3 | Q5XXQ5_ARAHY | Arachis hypogaea (Peanut) | 78.00% | 2.00E-55 |
| F0ZJC6 | Q64LH3_HUMSC | Humulus scandens (Hop) (Humulopsis scandens) | 78.00% | 1.00E-60 |
| Q9FUD1 | A4GD50_OLEEU | Olea europaea (Common olive) | 77.00% | 4.00E-57 |
| P35083 | A4GD58_OLEEU | Olea europaea (Common olive) | 77.00% | 2.00E-56 |
| A4KA58 | A4GDR3_OLEEU | Olea europaea (Common olive) | 77.00% | 2.00E-56 |
| Q38HU3 | A4GDT4_OLEEU | Olea europaea (Common olive) | 77.00% | 2.00E-56 |
| G7KQH7 | A4GDU3_OLEEU | Olea europaea (Common olive) | 77.00% | 7.00E-57 |
| Q64LH3 | A4GE49_OLEEU | Olea europaea (Common olive) | 77.00% | 3.00E-56 |
| A4GFB9 | A7XZJ7_SOYBN | Glycine max (Soybean) (Glycine hispida) | 77.00% | 2.00E-58 |
| Q84RR7 | A8VT60_AMAVI | Amaranthus viridis (Slender amaranth) | 77.00% | 2.00E-57 |
| Q9AXH5 | A9P8N4_POPTR | Populus trichocarpa (Western balsam poplar) (Populus balsamifera subsp. trichocarpa) | 77.00% | 4.00E-57 |
| 024169 | B0B0N5_MALDO | Malus domestica (Apple) (Pyrus malus) | 77.00% | 2.00E-57 |
| B9RQD3 | B0B0N6_MALDO | Malus domestica (Apple) (Pyrus malus) | 77.00% | 1.00E-56 |
| A4GE53 | B6CAT2_GERHY | Gerbera hybrida (Daisy) | 77.00% | 5.00E-58 |
| A4GD54 | B7VFP4_MALDO | Malus domestica (Apple) (Pyrus malus) | 77.00% | 5.00E-58 |
| Q84RR5 | C6JT04_9MYRT | Sonneratia alba | 77.00% | 1.00E-29 |
| A4GE45 | D3GC01_9ROSI | Jatropha curcas | 77.00% | 2.00E-58 |
| A4GDS9 | D7L6H1_ARALL | Arabidopsis lyrata subsp. lyrata (Lyre-leaved rock-cress) | 77.00% | 3.00E-54 |
| E6Y2M0 | E0XJL2_GOSHI | Gossypium hirsutum (Upland cotton) (Gossypium mexicanum) | 77.00% | 4.00E-58 |
| G9B5V1 | G9B5U3_GOSRA | Gossypium raimondii (New World cotton) | 77.00% | 5.00E-37 |
| G9B5U3 | G9B5U8_GOSAR | Gossypium arboreum (Tree cotton) (Gossypium nanking) | 77.00% | 5.00E-37 |
| G9B617 | G9B5U9_GOSHE | Gossypium herbaceum (Levant cotton) (Arabian cotton) | 77.00% | 5.00E-37 |
| G9B615 | G9B5V0_GOSHE | Gossypium herbaceum subsp. africanum | 77.00% | 5.00E-37 |
| G9B613 | G9B5V1_GOSBA | Gossypium barbadense (Sea-island cotton) (egyptian cotton) | 77.00% | 5.00E-37 |
| G9B612 | G9B5V2_GOSDA | Gossypium darwinii (Darwin's cotton) | 77.00% | 5.00E-37 |
| G9B611 | G9B5V4_GOSHI | Gossypium hirsutum (Upland cotton) (Gossypium mexicanum) | 77.00% | 5.00E-37 |
| G9B5P7 | PROF_ANACO | Ananas comosus (Pineapple) (Ananas ananas) | 77.00% | 7.00E-58 |
| E1ZQQ4 | PROF_CROSA | Crocus sativus (Saffron) | 77.00% | 2.00E-57 |
| C0Z2Z0 | PROF_CUCME | Cucumis melo (Muskmelon) | 77.00% | 7.00E-58 |
| A9XNJ9 | PROF_PRUPE | Prunus persica (Peach) (Amygdalus persica) | 77.00% | 5.00E-59 |
| A9XNJ8 | PROF_PYRCO | Pyrus communis (Pear) (Pyrus domestica) | 77.00% | 6.00E-59 |
| D8U668 | PROF1_ARATH | Arabidopsis thaliana (Mouse-ear cress) | 77.00% | 9.00E-55 |
| A1YQX7 | PROF1_ARTVU | Artemisia vulgaris (Mugwort) | 77.00% | 7.00E-55 |
| C1BP76 | PROF1_MALDO | Malus domestica (Apple) (Pyrus malus) | 77.00% | 4.00E-58 |
| B4LR73 | PROF1_SOYBN | Glycine max (Soybean) (Glycine hispida) | 77.00% | 3.00E-58 |
| D5ABJ3 | PROF2_MALDO | Malus domestica (Apple) (Pyrus malus) | 77.00% | 1.00E-58 |
| D3TKT4 | PROF2_PARJU | Parietaria judaica (Pellitory-of-the-wall) (Parietaria diffusa) | 77.00% | 4.00E-55 |
| Q8T938 | PROF2_SOYBN | Glycine max (Soybean) (Glycine hispida) | 77.00% | 5.00E-58 |
| C1DYP0 | PROF3_MALDO | Malus domestica (Apple) (Pyrus malus) | 77.00% | 2.00E-57 |
| Q95VF7 | PROF4_HEVBR | Hevea brasiliensis (Para rubber tree) (Siphonia brasiliensis) | 77.00% | 1.00E-58 |
| B3MMQ1 | PROF5_HEVBR | Hevea brasiliensis (Para rubber tree) (Siphonia brasiliensis) | 77.00% | 7.00E-60 |
| F4RL03 | Q27HX6_MANIN | Mangifera indica (Mango) | 77.00% | 3.00E-57 |
| D2D5E4 | Q3BCS2_MALDO | Malus domestica (Apple) (Pyrus malus) | 77.00% | 2.00E-57 |
| B2ZRT3 | Q3BCS4_MALDO | Malus domestica (Apple) (Pyrus malus) | 77.00% | 5.00E-59 |
| B4G6L5 | Q3BCS6_MALDO | Malus domestica (Apple) (Pyrus malus) | 77.00% | 3.00E-59 |
| A7XZK1 | Q3BCS8_MALDO | Malus domestica (Apple) (Pyrus malus) | 77.00% | 6.00E-59 |
| F1LBX8 | Q3BCT0_MALDO | Malus domestica (Apple) (Pyrus malus) | 77.00% | 4.00E-58 |
| Q6CPT8 | Q5XWE1_CITLA | Citrullus lanatus (Watermelon) (Citrullus vulgaris) | 77.00% | 8.00E-59 |
| E4V5P8 | Q84MM5_CUCMN | Cucumis melo var. cantalupensis (Netted muskmelon) (Cucumis melo var. reticulatus) | 77.00% | 2.00E-58 |
| A5DT33 | Q84RR5_MALDO | Malus domestica (Apple) (Pyrus malus) | 77.00% | 2.00E-57 |
| B8RIF7 | Q84RR6_MALDO | Malus domestica (Apple) (Pyrus malus) | 77.00% | 2.00E-58 |
| G8BA18 | Q84RR7_MALDO | Malus domestica (Apple) (Pyrus malus) | 77.00% | 5.00E-59 |
| C5FGJ6 | Q9AXH4_CORAV | Corylus avellana (European hazel) (Corylus maxima) | 77.00% | 3.00E-59 |
| Q86RQ5 | Q9AXH5_CORAV | Corylus avellana (European hazel) (Corylus maxima) | 77.00% | 6.00E-59 |
| A4KA36 | A1Z292_COCNU | Cocos nucifera (Coconut) | 76.00% | 2.00E-57 |
| A4KA49 | A1Z294_CARMI | Caryota mitis (Burmese fishtail palm) | 76.00% | 3.00E-57 |
| A7XZJ7 | B3TLV2_ELAGV | Elaeis guineensis var. tenera (Oil palm) | 76.00% | 3.00E-57 |
| Q2XPH2 | B8BG75_ORYSI | Oryza sativa subsp. indica (Rice) | 76.00% | 9.00E-52 |
| B0B0N5 | C6JWH0_SALKA | Salsola kali (Russian thistle) | 76.00% | 3.00E-55 |
| A4GD56 | D7MCM8_ARALL | Arabidopsis lyrata subsp. lyrata (Lyre-leaved rock-cress) | 76.00% | 4.00E-55 |
| A1Z294 | D7MLU1_ARALL | Arabidopsis lyrata subsp. lyrata (Lyre-leaved rock-cress) | 76.00% | 7.00E-55 |
| A9P8N4 | D7TBE7_VITVI | Vitis vinifera (Grape) | 76.00% | 7.00E-57 |
| A7XZJ9 | F2EGC6 HORVD | Hordeum vulgare var. distichum (Two-rowed barley) | 76.00% | 1.00E-56 |
| G9B603 | PROF_APIGR | Apium graveolens (Celery) | 76.00% | 2.00E-58 |
| G9B5Z9 | PROF ARAHY | Arachis hypogaea (Peanut) | 76.00% | 9.00E-57 |
| C6JT04 | PROF_DAUCA | Daucus carota (Carrot) | 76.00% | 8.00E-59 |
| A9XNJ4 | PROF_MUSAC | Musa acuminata (Banana) (Musa cavendishii) | 76.00% | 2.00E-55 |
| B8RIF1 | PROF1_PARJU | Parietaria judaica (Pellitory-of-the-wall) (Parietaria diffusa) | 76.00% | 5.00E-56 |
| P19984 | PROF3_LILLO | Lilium longiflorum (Trumpet lily) | 76.00% | 5.00E-56 |
| Q5KNQ6 | PROF6_HEVBR | Hevea brasiliensis (Para rubber tree) (Siphonia brasiliensis) | 76.00% | 3.00E-59 |
| D2DSN5 | Q5EEP6_PETCR | Petroselinum crispum (Parsley) (Petroselinum hortense) | 76.00% | 3.00E-59 |
| E9E950 | Q5EEP8_PETCR | Petroselinum crispum (Parsley) | 76.00% | 8.00E-59 |
| | | (Petroselinum hortense) | | |
| P53696 | Q7E369 CUCMN | Cucumis melo var. cantalupensis (Netted muskmelon) (Cucumis melo var. reticulatus) | 76.00% | 1.00E-57 |
| A4HK30 | Q9XF10_9ASPA | Phalaenopsis hybrid cultivar | 76.00% | 2.00E-55 |
| P35079 | A1BQK7_CUCSA | Cucumis sativus (Cucumber) | 75.00% | 5.00E-18 |
| A4KA52 | A1Z293_ROYRE | Roystonea regia (Cuban royal palm) | 75.00% | 3.00E-56 |
| A4GE50 | B6CQU8_9ROSA | Prunus dulcis x Prunus persica | 75.00% | 9.00E-57 |
| A4GCR3 | C0Z3G0_ARATH | Arabidopsis thaliana (Mouse-ear cress) | 75.00% | 9.00E-55 |
| A9XNK0 | PROF_PRUDU | Prunus dulcis (Almond) (Prunus amygdalus) | 75.00% | 9.00E-57 |
| B0W2U6 | PROF2 ARATH | Arabidopsis thaliana (Mouse-ear cress) | 75.00% | 9.00E-55 |
| Q6RG01 | PROF2 ARTVU | Artemisia vulgaris (Mugwort) | 75.00% | 1.00E-53 |
| P39825 | Q5EEP4 PETCR | Petroselinum crispum (parsley) (Petroselinum hortense) | 75.00% | 5.00E-55 |
| E7D158 | Q64LH4_HUMSC | Humulus scandens (Hop) (Humulopsis scandens) | 75.00% | 2.00E-57 |
| A7S5R6 | Q8GT40_PRUPE | Prunus persica (Peach) (Amygdalus persica) | 75.00% | 7.00E-57 |
| Q93YG7 | A9P8K3 POPTR | Populus trichocarpa (Western balsam poplar) (Populus balsamifera subsp. trichocarpa) | 74.00% | 2.00E-57 |
| A4GDT1 | B4FK52_MAIZE | Zea mays (Maize) | 74.00% | 6.00E-35 |
| E0XJL2 | B6TJ90 MAIZE | Zea mays (Maize) | 74.00% | 2.00E-36 |
| B7VFP5 | PROF_PRUAV | Prunus avium (Cherry) | 74.00% | 4.00E-56 |
| P18322 | PROF5_ARATH | Arabidopsis thaliana (Mouse-ear cress) | 74.00% | 6.00E-53 |
| A1KYY2 | Q7X9Q0_CUCME | Cucumis melo (Muskmelon) | 74.00% | 2.00E-55 |
| A1KXJ2 | Q7Y253_GOSHI | Gossypium hirsutum (Upland cotton) (Gossypium mexicanum) | 74.00% | 2.00E-56 |
| Q5A786 | Q8H123_ARATH | Arabidopsis thaliana (Mouse-ear cress) | 74.00% | 6.00E-53 |
| C4YDM2 | Q8H2C7_ARATH | Arabidopsis thaliana (Mouse-ear cress) | 74.00% | 6.00E-53 |
| F1A3K2 | Q8LCJ1_ARATH | Arabidopsis thaliana (Mouse-ear cress) | 74.00% | 4.00E-54 |
| Q8GT39 | A8VT57_AMAVI | Amaranthus viridis (Slender amaranth) | 73.00% | 3.00E-54 |
| Q3BCS4 | A9NMR7_PICSI | Picea sitchensis (Sitka spruce) (Pinus sitchensis) | 73.00% | 3.00E-51 |
| P22271 | PROF1_CITSI | Citrus sinensis (Sweet orange) (Citrus aurantium var. sinensis) | 73.00% | 1.00E-54 |
| D3U1G3 | B3H795_ARATH | Arabidopsis thaliana (Mouse-ear cress) | 72.00% | 9.00E-31 |
| F4YF99 | B8RIF1_PINSY | Pinus sylvestris (Scots pine) | 72.00% | 2.00E-20 |
| E4MXD3 | B8RIF3_PINSY | Pinus sylvestris (Scots pine) | 72.00% | 8.00E-14 |
| B6CAT2 | B8RIF9_PINSY | Pinus sylvestris (Scots pine) | 72.00% | 6.00E-16 |
| A4GD53 | C0Z2Z0_ARATH | Arabidopsis thaliana (Mouse-ear cress) | 72.00% | 9.00E-31 |
| A4GE54 | C5J3U0_CHEAL | Chenopodium album (Lamb's-quarters) | 72.00% | 3.00E-50 |
| A4GD55 | E2D0Y9_SALKA | Salsola kali (Russian thistle) | 72.00% | 4.00E-52 |
| D2XTC1 | Q3LVF0_TAROF | Taraxacum officinale (Common dandelion) (Leontodon taraxacum) | 72.00% | 1.00E-26 |
| 065810 | B7VFP5_MALDO | Malus domestica (Apple) (Pyrus malus) | 70.00% | 2.00E-26 |
| A4S1F7 | Q1EMQ3_PLAMJ | Plantago major (Common plantain) | 70.00% | 6.00E-53 |
| C5Z4B6 | A9NNS7_PICSI | Picea sitchensis (Sitka spruce) (Pinus sitchensis) | 68.00% | 6.00E-51 |
| A4GDR8 | A9P2A1_PICSI | Picea sitchensis (Sitka spruce) (Pinus sitchensis) | 68.00% | 2.00E-50 |
| B3TLV2 | D5ABJ3_PICSI | Picea sitchensis (Sitka spruce) (Pinus sitchensis) | 67.00% | 3.00E-19 |
| Q9STB6 | A5AW47_VITVI | Vitis vinifera (Grape) | 65.00% | 2.00E-24 |

**Table 5**

| Position | Amino acids variation | Position | Amino acids variation |
|---|---|---|---|
| 5 | T or A | 59 | HorYorTorForS |
| 8 | D or Y or N | 63 | T or I |
| 9 | D or E | 65 | M or L |
| 12 | M or C or L | 66 | F or L or I or H or Y |
| 14 | E or D | 67 | V or I or L |
| 15 | I or V | 68 | A or G |
| 16 | E or D | 69 | G or A or T or S or P |
| 18 | H or L or Q or N | 70 | A or T |
| 19 | HQ | 81 | A or R or V |
| 20 | LH | 83 | I or T |
| 21 | AorSorTorG | 89 | A or S or T or P |
| 22 | S or A | 92 | I or V |
| 23 | A or T | 94 | L or I or V |
| 26 | F or L or I or V or A | 98 | G or N |
| 28 | H or Q | 99 | Q or M |
| 29 | D or G or A | 100 | A or S |
| 31 | TorAorS | 101 | LorM |
| 32 | V or T or A | 102 | V or I |
| 37 | AorTorPorS | 103 | L or I or V |
| 38 | D or A or T or N | 105 | I or V |
| 41 | Q or S or E or A or L | 107 | D or E |
| 43 | K or A or G | 110 | M or L |
| 44 | PorTorA | 116 | N or S or D |
| 45 | E or A or N | 117 | M or L |
| 46 | E or D | 118 | V or I |
| 47 | I or M or V | 119 | V or I |
| 48 | TorAorS | 120 | E or G |
| 49 | GorNorA | 121 | R or K |
| 50 | I or V | 127 | V or L or M |
| 51 | M or I | 128 | E or K |
| 52 | K or N | 131 | M or L or Q or F |
| 55 | D or A | | |

**Table 6**

| SET | | SUB-SET | |
|---|---|---|---|
| Hydrophobic | FWYHKMILVTAGC | Aromatic | FWYH |
| | | Aliphatic | ILV |
| Polar | WYHKREDCSTNQ | Charged | HKRED |
| | | Positively charged | HKR |
| | | Nagatively charged | ED |
| Small | VCAGSPTND | Tiny | AGCS |

**Table 7**

| His (H) | aromatic, polar, hydrophilic, charged (+) | (Arg, Lys) |
|---|---|---|
| Thr (T) | polar, hydrophilic, neutral, small | Ser, Cys |
| Ile (I) | aliphatic, hydrophobic, neutral | Leu, Val |
| Val (V) | aliphatic, hydrophobic, neutral | Leu, Ile |
| Lys (K) | polar, hydrophilic, charqed(+) | Arg |
| Trp (W) | aromatic, polar, hydrophobic, neutral | Tyr, Phe |
| Leu (L) | aliphatic, hydrophobic, neutral | Ile, Val |
| Tyr (Y) | aromatic, polar, hydrophobic | Trp, Phe |

**Table 8**

| Amino acid codes | Properties (physical, chemical, structural) | Suitable conservative substitutions |
|---|---|---|
| Ala (A) | aliphatic, hydrophobic, neutral, small | - |
| Met (M) | hydrophobic, neutral | - |
| Cys (C) | polar, hydrophobic, neutral, small | Ser, Thr |
| Asn (N) | polar, hydrophilic, neutral, small | Gln |
| Asp (D) | polar, hydrophilic, charged (-) | Glu |
| Pro (P) | hydrophobic, neutral, small | (Thr) |
| Glu (E) | polar, hydrophilic, charged (-) | Asp |
| Gln (Q) | polar, hydrophilic, neutral | Asn |
| Phe (F) | aromatic, hydrophobic, neutral | Tyr, Trp |
| Arg (R) | polar, hydrophilic, charged (+) | Lys |
| Gly (G) | aliphatic, hydrophobic, neutral, small | - |
| Ser (S) | polar, hydrophilic, neutral, small | Thr, Cys |
| His (H) | aromatic, polar, hydrophilic, charged (+) | (Arg, Lys) |
| Thr (T) | polar, hydrophilic, neutral, small | Ser, Cys |
| Ile (I) | aliphatic, hydrophobic, neutral | Leu, Val |
| Val (V) | aliphatic, hydrophobic, neutral | Leu, Ile |
| Lys (K) | polar, hydrophilic, charqed(+) | Arg |
| Trp (W) | aromatic, polar, hydrophobic, neutral | Tyr, Phe |
| Leu (L) | aliphatic, hydrophobic, neutral | Ile, Val |
| Tyr (Y) | aromatic, polar, hydrophobic | Trp, Phe |

### List of Sequences

SEQ ID NO: 1: >sp|024650|PROF2_PHLPR Profilin-2/4 OS=*Phleum pratense* GN=PRO2 PE=1 SV=1
SEQ ID NO: 2: >sp|024282|PROF3_PHLPR Profilin-3 OS=*Phleum pratense* GN=PRO3 PE=1 SV=1
SEQ ID NO: 3: >sp|P35079|PROF1_PHLPR Profilin-1 *OS=Phleum pratense* GN=PRO1 PE=1 SV=1
SEQ ID NO: 4: >tr|A4KA31|A4KA31_PHLPR Profilin OS=*Phleum pratense* PE=2 SV=1
SEQ ID NO: 5: >tr|A4KA32|A4KA32_PHLPR Profilin OS=*Phleum pratense* PE=2 SV=1
SEQ ID NO: 6: >tr|A4KA33|A4KA33_PHLPR Profilin OS=*Phleum pratense* PE=2 SV=1
SEQ ID NO: 7: >tr|A4KA34|A4KA34_PHLPR Profilin OS=*Phleum pratense* PE=2 SV=1
SEQ ID NO: 8: >tr|A4KA36|A4KA36_PHLPR Profilin OS=*Phleum pratense* PE=2 SV=1
SEQ ID NO: 9: >tr|A4KA37|A4KA37_PHLPR Profilin OS=*Phleum pratense* PE=2 SV=1
SEQ ID NO: 10: >tr|A4KA38|A4KA38_PHLPR Profilin OS=*Phleum pratense* PE=2 SV=1
SEQ ID NO: 11: Tri a 12.0101 (4 isoforms in allergen.org) (Wheat, (*Poales*) >sp|P49232|PROF1_WHEAT Profilin-1 (Fragment) OS=Triticum aestivum GN=PRO1 PE=2 SV=1
SEQ ID NO: 12: Hor v 12.0101 (Barley (*Poales*)) >sp|P52184|PROF1_HORVU Profilin-1 OS=Hordeum vulgare GN=PRO1 PE=2 SV=1
SEQ ID NO: 13: Ory s 12.0101 (Rice(*Poales*)) >sp|Q9FUD1|PROFA_ORYSJ Profilin-A OS=Oryza sativa subsp. japonica GN=Os10g0323600 PE=2 SV=1
SEQ ID NO: 14: Zea m 12.0101 (Maize(*Poales*))) >sp|P35081|PROF1_MAIZE Profilin-1 OS=Zea mays GN=PRO1 PE=1 SV=1
SEQ ID NO: 15: Cyn d 12.0101 (Bermuda grass(*Poales*)) >sp|004725|PROF_CYNDA Profilin OS=Cynodon dactylon GN=PRO1 PE=1 SV=1
SEQ ID NO: 16: Ole e 2.0101 (Olive *(Lamiales*) >sp|024169|PROF1-OLEEU Profilin-1 OS=Olea europaea GN=PRO1 PE=1 SV=1
SEQ ID NO: 17: Cor a 2.0101 (European hazel *(Fagales)*) >tr|Q9AXH5|Q9AXH5_CORAV Profilin OS=Corylus avellana PE=2 SV=1
SEQ ID NO: 18: Bet v 2.0101 *(Fagales)* >sp|P25816|PROF_BETPN Profilin OS=Betula pendula GN=BETVII PE=1 SV=1
SEQ ID NO: 19: Ara h 5.0101(peanut *(Fabales)* >sp|Q9SQI9|PROF_ARAHY Profilin OS=Arachis hypogaea PE=1 SV=1
SEQ ID NO: 20: Gly m 3.0101 (soya bean *(Fabales)* >sp|065809|PROF1_SOYBN Profilin-1 OS=Glycine max GN=PRO1 PE=1 SV=1
SEQ ID NO: 21: Amb a 8.0101 (ragweed -short (Asterales*)) >tr*|*Q2KN24*|*Q2KN24_AMBAR Profilin OS=Ambrosia artemisiifolia PE*=*2 SV*=*1*
SEQ ID NO: 22: Hel a 2.0101 (sunflower (*Asterales*)) >sp|081982|PROF_HELAN Profilin OS=Helianthus annuus PE=1 SV=1
SEQ ID NO: 23: Art v 4.0101 (*Asterales*)) >sp|Q8H2C9|PROF1_ARTVU Profilin-1 OS=Artemisia vulgaris PE=1 SV=3
SEQ ID NO: 24: Hev b 8.0101 (*Malpighiales*) >sp|065812|PROF1_HEVBR Profilin-1 OS=Hevea brasiliensis PE=1 SV=1
SEQ ID NO: 25: Fra a 4 *(Rosales)*) >sp|P0C0Y3|PROF_FRAAN Profilin OS=Fragaria ananassa PE=1 SV=1
SEQ ID NO: 26: Mal d 4.0101 *(Rosales)*) >sp|Q9XF42|PROF3_MALDO Profilin-3 OS=Malus domestica PE=1 SV=1
SEQ ID NO: 27: Pru av 4.0101 (Cherry *(Rosales))* >sp|Q9XF39|PROF_PRUAV Profilin OS=Prunus avium PE=1 SV=1
SEQ ID NO: 28: Pru du 4.0101 (almond *(Rosales))* >sp|Q8GSL5|PROF_PRUDU Profilin OS=Prunus dulcis PE=1 SV=1
SEQ ID NO: 29: Pru p 4.0101 (peach, *(Rosales))* >tr|Q8GT40|Q8GT40_PRUPE Profilin OS=Prunus persica PE=2 SV=1
SEQ ID NO: 30: Pyr c 4.0101 (pear *(Rosales))* >sp|Q9XF38|PROF_PYRCO Profilin OS=Pyrus communis PE=1 SV=1
SEQ ID NO: 31: Par j 3.0101) >sp|Q9XG85|PROF1_PARJU Profilin-1 OS=Parietaria judaica GN=PRO1 PE=1 SV=1
SEQ ID NO: 32: Pho d 2.0101 (date palm *(Arecales))* >tr|Q8L5D8|Q8L5D8_PHODC Profilin OS=Phoenix dactylifera GN=pro PE=2 SV=1
SEQ ID NO: 33: Che a 2.0101 *(Caryophyllales)* >sp|Q84V37|PROF_CHEAL Profilin OS=Chenopodium album PE=1 SV=1
SEQ ID NO: 34: Sal k 4.0101 *(Caryophyllales)* >tr|C6JWH0|C6JWH0_SALKA Profilin (Fragment) OS=Salsola kali PE=2 SV=1
SEQ ID NO: 35: Ama r 2.0101 (redroot pigweed *(Caryophyllales)*) >tr|C3W2Q7|C3W2Q7_AMARE Profilin (Fragment) OS=Amaranthus retroflexus PE=2 SV=1
SEQ ID NO: 36: Dau c 4.0101 (Carrot, *(Apiales)* >sp|Q8SAE6|PROF_DAUCA Profilin OS=Daucus carota PE=1 SV=1
SEQ ID NO: 37: Api g 4.0101 (Celery, *(Apiales)*) >sp|Q9XF37|PROF_APIGR Profilin OS=Apium graveolens PE=1 SV=1
SEQ ID NO: 38: Cuc m 2.0101 (musk melon, *Cucurbitales*) >sp|Q5FX67|PROF_CUCME Profilin OS=Cucumis melo PE=1 SV=1
SEQ ID NO: 39: Cap a 2.0101 (bell peber, *Solanales)* >sp|Q93YI9|PROF_CAPAN Profilin OS=Capsicum annuum PE=1 SV=1
SEQ ID NO: 40: Ana c 1.0101 (pineapple, *Poales*) >sp|Q94JN2|PROF_ANACO Profilin OS=Ananas comosus PE=1 SV=1
SEQ ID NO: 41: Cro s 2. (*Asparagales*)) >sp|Q5EF31|PROF_CROSA Profilin OS=Crocus sativus PE=1 SV=1
SEQ ID NO: 42: Mus a 1.0101 (Banana *(Zingiberales)*) >sp|Q94N3|PROF_MUSAC Profilin OS=Musa acuminata PE=1 SV=1
SEQ ID NO: 43: Recombinant (positions 71-127 of SEQ ID NO: 1) YMVIQGEPGAVIRGKKGAGGITIKKTGQALVVGIYDEPMTPGQCNMVVERLGDYLV
SEQ ID NO: 44: Recombinant (seq ID no 1 with substitutions C13S and C115S)
SEQ ID NO: 45: Recombinant (seq ID no 1 with substitutions C13A and C115A)
SEQ ID NO: 46: Nucleotides sequence to produce SEQ ID NO 44: > V1rPhl p 12_ALK_IP1002
SEQ ID NO: 47: Nucleotides sequence to produce SEQ ID NO: 45: > V2rPhl p 12_ALK_IP1003

### List of References

Amnuaycheewa P and Gonzalez de Mejia E. Purification, characterisation, and quantification of the soy allergen profilin (Gly m 3) in soy products, Food Chemistry 119, 1671-1680, 2010.
Dahlmann-Hoglund et al. Bystander suppression of the immune response to human serum albumin in rats, Immunology 86, 128-133, 1995.
Fechheimer M and Zigmond S. H., Focusing on Unpolymerized Actin, The Journal of Cell Biology, Volume 123, No 1, 1-5, 1993
Lockey R and Ledford D. Allergens and Allergen Immunotherapy 4th Ed, 2008, Ed by R Lockey and D Ledford, Informa healthcare).
Lu J and Pollard T, Profilin Binding to Poly-L-Proline and Actin Monomers along with Ability to Catalyze Actin Nucleotide Exchange Is Required for Viability of Fission Yeast, Molecular Biology of the Cell, Vol. 12, 1161-1175, 2001.
Martinez A et al, The allergenic relevance of profilin (Ole e 2) from Olea europaea pollen, Allergy, 57, suppl. 71, 17-23, 2002.
Miller et al. Antigen-driven Bystander Suppression after Oral Administration of Antigens, J. Exp. Med. 174, 791-798, 1991.
Millington et al. Induction of Bystander Suppression by Feeding Antigen Occurs despite Normal Clonal Expansion of the Bystander T Cell Population. Immunology, 173: 6059-6064, 2004.
Oliveira CR et al. Bystander effect in synergi to anergy in oral tolerance of Blomia Tropcalis/Ovalbumin Murine Co-Immunization model. J Clin Immunol, 25, 153-161, 2005.
Santos A and Van Ree R, Profilins: Mimickers of Allergy or Relevant Allergens?, Int Arch Allergy Immunol, 155, 191-204, 2011.
Thorn et al, Crystal structure of a major allergen from plants, Structure, vol 5, no 1, 1997.
Vidali L, H. E. Perez, V. V. Lopez, R. Noguez, F. Zamudio and F. Sanchez, Purification, Characterization, and cDNA Cloning of Profilin from Phaseolus vulgaris, Plant Physiology, vol. 108, no. 1, 115-123, 1995.

### Examples

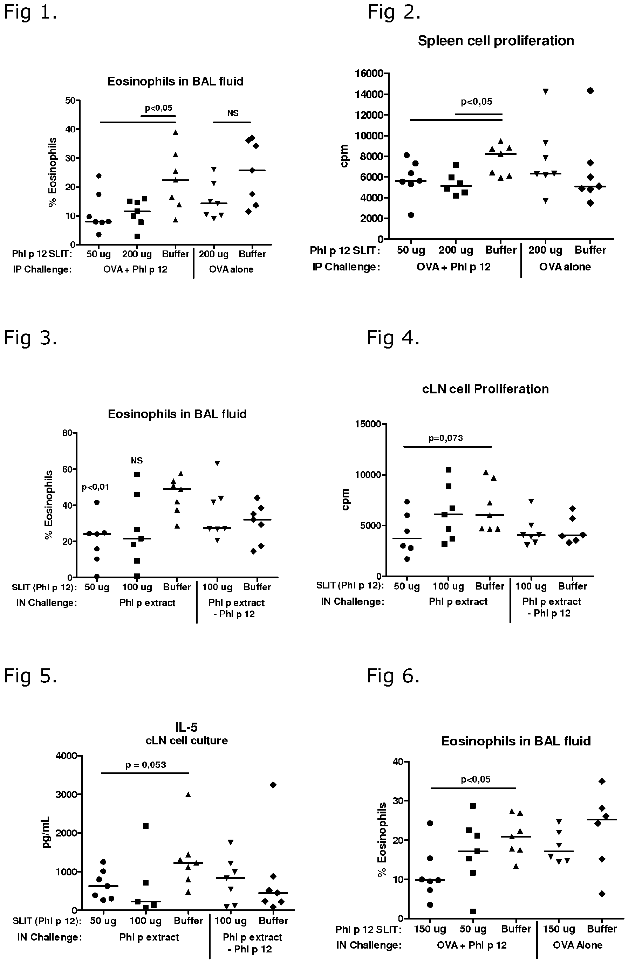

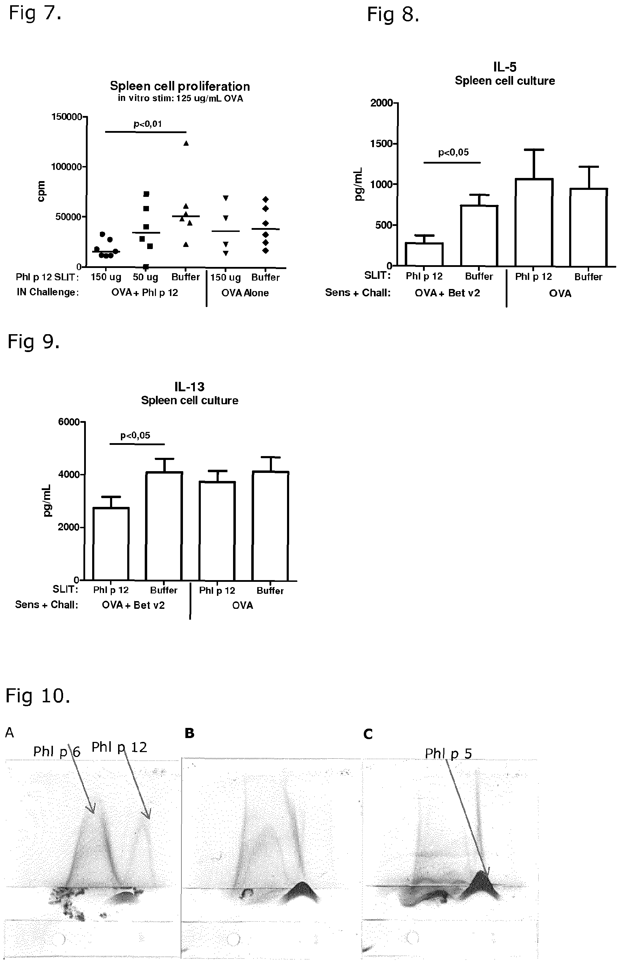

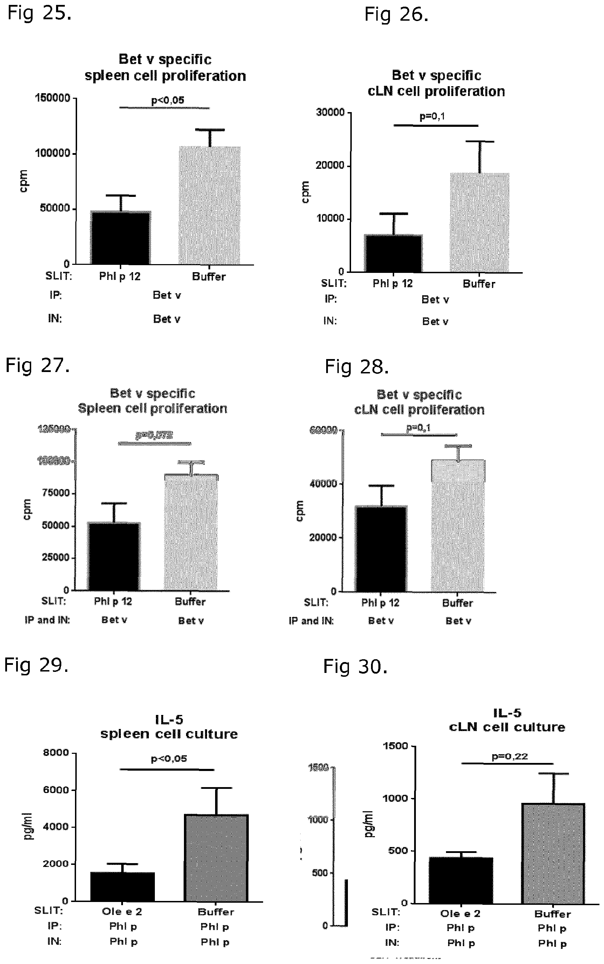

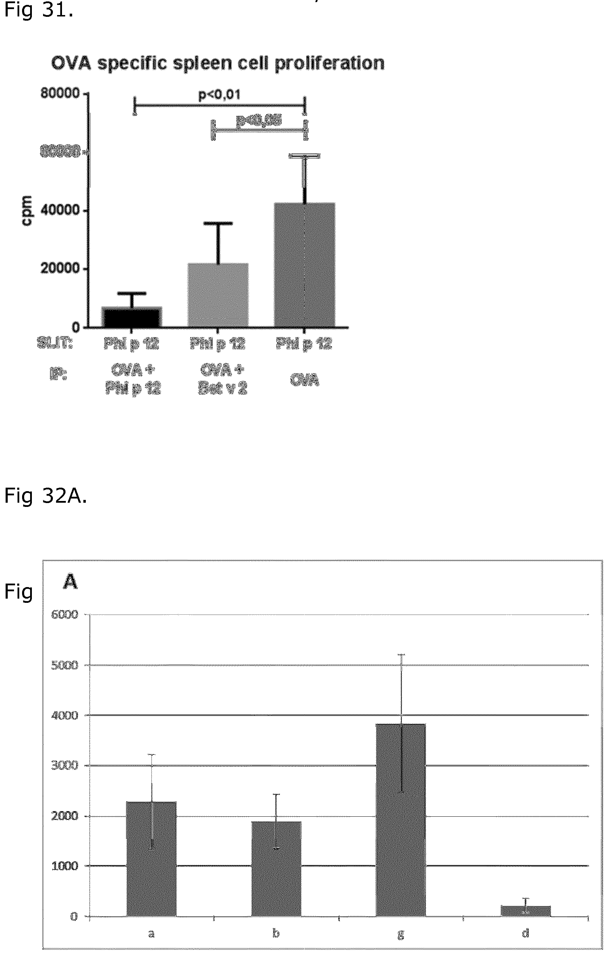

Examples 1 (Figs 1 and 2), 2 (Figs 3, 4, and 5), 4 (Figs 8 and 9), 8 (Figs 25 and 26), 10 (Figs 29 and 30) and 11 (Fig 31) refer to the investigation of bystander suppression in a prophylactic treatment model with un-sensitized mice. In short, the mice are treated daily by sublingual administration with a polypeptide of the invention for a period (typically two weeks) so as to induce tolerance against the polypeptide. Subsequently, the mice are sensitized to an antigen by i.p. administration to induce a hypersensitivity immune response and, optionally, the mice are also challenged to the sensitizing antigen by intranasal administration to induce allergic inflammation in the respiratory tract. Finally, the mice are sacrified and blood, bronchoalveolar fluid (BAL) and spleen are collected for analysis. Bystander suppression is then determined by comparing the fraction of eosinophils in the BAL fluid of mice exposed to both the tolerance-inducing antigen and the sensitizing antigen with the fraction of eosinophils of mice only exposed to the sensitizing antigen. Alternatively, or additionally, spleen and/or cervical lymph node cells are isolated, cultured and stimulated with the sensitizing allergen. The cell proliferation or cytokine output (IL-5, IL-13) is measured and bystander suppression is determined by comparing the cell proliferation or cytokine output in samples taken from mice exposed to both the tolerance-inducing antigen and the sensitizing antigen with the cell proliferation or cytokine output of samples of mice only exposed to the sensitizing antigen.

Examples 3 (Figs 6 and 7) and 9 (Figs 27 and 28) refer to the investigation of bystander suppression in a therapeutic treatment model with sensitized mice. In short, the experimental set-up is slightly different to the prophylactic model in that the mice are initially sensitized by i.p. administration with the sensitizing allergen and the mice are subsequently treated with the tolerance-inducing antigen (polypeptide for SLIT). All subsequent steps are similar to the prophylactic model.

An overview of the examples referring to mice data on bystander suppression is given below, (P) refers to the prophylactic model and (T) to the therapeutic model:

| **Example** | **Polypeptide for SLIT treatment (tolerance - inducing antigen)** | **Antigen for sensitization by i.p. administration** | **Antigen for nasal challenge** |
|---|---|---|---|
| 1 (P) | Phl p 12 | OVA | OVA |
| | | OVA + Phl p 12 | |
| 2 (P) | Phl p 12 | Phl p extract (without and with Phl p 12) | Phl p extract with Phl p 12) |
| 3 (T) | Phl p 12 | OVA | OVA |
| | | | Phl p 12 |
| 4 (P) | Phl p 12 | OVA | OVA |
| | | | OVA + Bet v2 |
| 8 (P) | Phl p 12 | Bet v extract | Bet v extract |
| 9 (T) | Phl p 12 | Bet v extract | Bet v extract |
| 10 (P) | Ole e 2 | Phl p extract | None |
| 11 (P) | Phl p 12 | OVA + Phl p 12 | None |
| | | OVA + Bet v 2 | |
| | | OVA | |

### Example 1

Bystander suppression of a hypersensitivity immune response (asthma) caused by OVA antigen in Phl p 12-treated naïve mice.

Methods: Phl p 12 was isolated from aqueous extracts of pollen of *Phleum pratense* by use of PLP agarose or it was produced recombinantly based on the amino acid sequence SEQ ID NO: 1.

Animals: Female, 6-10 week-old BALB/cJ mice were bred in-house and maintained on a defined diet not containing components cross reacting with rabbit antisera to *Phleum pratense* (Phl p). Each experimental group consisted of 7-8 animals.

Animal Experiments: Naive mice were treated 5 days a week by sublingual immunotherapy (SLIT) with 50 or 200 µg Phl p 12 for 2 weeks (treatment phase), followed by two weekly i.p. injections of either a mix of 10 µg OVA and 10 µg Phl p 12 or 10 µg OVA alone adsorbed to aluminium hydroxide (exposure phase to sensitize the mice). Subsequently, the mice were challenged intranasally with 50 µg of OVA for four days (induction of hypersensitivity immune response such as astma). The mice were sacrificed one day after the last challenge and blood, bronchoalveolar fluid (BAL) and spleens were collected for analysis.

Differential Counting of BAL Fluid: The BAL fluid was centrifuged and the supernatant was removed. The pellet was re-suspended in PBS and the fraction of eosinophils was determined by an automated cell counter (Sysmex).

T-cell Proliferation Assay: Spleens were teased into single cell suspension and washed three times in medium. Cells were counted and 3 x 10⁵ cells were added to each well of a 96 well flat-bottomed culture plate and stimulated by 0, 5, 25 and 125 µg/mL OVA. The cells were cultured for 6 days at 37°C and 5% CO₂. Proliferation was measured by adding 0.5 µCi of 3H-thymidine to each well for the last 18 hours of the culture period, followed by harvesting of the cells and counting the incorporated radiolabel.

### Results:

In this experiment, it was examined whether non-sensitized mice treated by a polypeptide of the invention (e.g. Profilin Phl p 12) could be prevented from developing a hypersensitivity immune response induced by a non-profilin allergen (OVA) or at least could suppress a hypersensitivity immune response (e.g. asthma) induced by a non-profilin allergen (OVA), e.g. could a hypersensitivity immune response as induced by OVA be supressed/prevented via bystander suppression achieved by administering an unrelated antigen; profilin Phl p 12. Figure 1 shows that Phl p 12-treated mice displayed a reduced fraction of eosinophils in BAL fluid compared to buffer-treated mice, but only when the mice were exposed/sensitized to both OVA and Phl p 12 and not only OVA. Figure 2 indicates the same findings as in Figure 1, but with respect to in vitro proliferation of spleen cells; OVA-specific in vitro proliferation of spleen cells is down-regulated in Phl p 12-treated mice, but only in mice co-exposed/co-sensitized to both Phl p 12 and OVA and not only to OVA.

### Example 2

Bystander suppression of a hypersensitivity immune response (asthma) caused by non-profilin allergens of pollens of *Phleum pratense* by treatment of naïve mice with Phl p 12.

Animal Experiments: Phl p 12 was obtained as in Example 1 and similar animals were used. Naive mice were treated by sublingual immunotherapy (SLIT) with 50 or 100 µg Phl p 12 for 2 weeks (treatment phase) followed by three weekly i.p. injections of either 8 µg Phl p extract or 8 µg Phl p extract depleted for Phl p 12, both adsorbed to aluminium hydroxide (exposure phase to sensitize the mice). Subsequently, the mice were challenged intranasally with the either 80 µg Phl p extract or the same dose of extract depleted for Phl p 12 (induction of hypersensitivity immune response such as astma). The mice were sacrificed one day after the last challenge and blood, bronchoalveolar fluid (BAL) and cervical lymph nodes were collected for analysis.

Differential Counting of BAL Fluid: Same procedure as presented in Example 1.

T-cell Proliferation Assay: Same procedure as presented in Example 1, but where cells were counted and adjusted to 1.67 x 10⁶ cells/mL and 3 x 10⁵ cells were added to each well of a 96 well flat-bottomed culture plate and the cells were stimulated by 0, 5 and 40 µg/mL Phl p extract. Cell culture supernatants for cytokine analysis were harvested on day 5.

### Results:

In this experiment, it was examined whether non-sensitized mice treated by a polypeptide of the invention (e.g. Profilin Phl p 12) could be prevented from developing a hypersensitivity immune response induced by non-profilin allergens of pollen of *Phleum pratense* (e.g. allergens Phl p 1, Phl p 5, Phl p 6) or at least could suppress a hypersensitivity immune response (e.g. asthma) induced by those non-profilin allergens, e.g. could a hypersensitivity immune response as induced by non-profilin allergens of pollen of *Phleum pratense* be supressed/prevented via bystander tolerance achieved by administering an unrelated antigen; profilin Phl p 12. This was done by SLIT treatment of naïve mice with Phl p 12 or buffer. After the SLIT treatment, allergic asthma was induced by IP sensitization (Phl p extract with and without Phl p 12) followed by intranasal challenge with grass pollen extract of *Phleum pratense* (Phl p extract containing at least the allergens Phl p 1, 5 and 6 and the profilin Phl p 12) or grass pollen extract depleted for content of profilin (Phl p extract depleted for Phl p 12). As seen in Figure 3, Phl p 12-treated mice have a reduced number of eosinophils in the BAL fluid compared to buffer-treated mice and that this effect is only achieved when the mice are exposed to both the non-profilin allergens and Phl p 12 (Phl p extract containing Phl p 12), but not when mice were exposed to Phl p extract depleted from Phl p 12. These results were confirmed by in vitro stimulation of cervical lymph node cells. Although the differences were not significant, Figures 4 and 5 show that proliferation and the production of the Th2 associated cytokine IL-5, respectively, are down-regulated in Phl p 12-treated mice in the same way as described above.

### Example 3

Bystander suppression of a hypersensitivity immune response (asthma) in Phl p 12-treated OVA-sensitized mice.

Animal Experiments: Phl p 12 was obtained as in Example 1 and similar animals were used. Mice were sensitized by two weekly i.p. injection of 10 µg OVA adsorbed to aluminium hydroxide (sensitization phase). Following this, the mice were SLIT treated with 0, 50 or 150 µg Phl p 12 for 6 weeks (treatment phase) and subsequently they were intranasally challenged for 4 days with 40 µg OVA together with 40 µg Phl p or with OVA alone (exposure phase). The mice were sacrificed one day after the last challenge and blood, bronchoalveolar fluid (BAL), spleen and cervical lymph nodes were collected for analysis.
Differential Counting of BAL Fluid: Same procedure as presented in Example 1
T-cell Proliferation Assay: Same procedure as presented in Example 1.

### Results:

In this set of experiments, it was investigated whether Phl p 12 treated OVA-sensitized mice could be prevented from developing or suppressing clinical and immunological signs of allergic asthma upon exposure to the "triggering" allergen OVA. As shown in Figure 6, the fraction of eosinophils in the BAL fluid was significantly decreased in Phl p 12-treated OVA-sensitized mice compared to buffer-treated OVA-sensitized mice upon being exposed to the non-profilin allergen (OVA) intranasally (exposure to the airways). Notably, this down-regulation of eosinophils could not be observed if the mice were exposed to OVA only, i.e. without the co-exposure of Phl p 12. Furthermore, as seen in Figure 7, OVA-specific in vitro proliferation of spleen cells is down-regulated in Phl p 12-treated mice, but only in mice co-exposed/co-sensitized to both Phl p 12 and OVA, and not only to OVA.

### Example 4

Bystander suppression of a hypersensitivity immune response (asthma) caused by a non-profilin allergen (OVA) by treating naïve mice with Phl p 12 and exposing the mice to OVA and a different profilin Bet v 2.

Methods: Phl p 12 was obtained as described above and Bet v 2 was isolated from pollen extract of *Betula verrucosa.*

Animal Experiments: Naive mice (same type as in Example 1) were treated 5 days a week by sublingual immunotherapy (SLIT) with 75 µg Phl p 12 for 2 weeks (treatment phase), followed by two weekly i.p. injections of either a mix of 10 µg OVA and 10 µg Bet v 2 or 10 µg OVA alone adsorbed to aluminium hydroxide (exposure phase to sensitize the mice). Subsequently, the mice were challenged intranasally with 40 µg of OVA for 4 days (induction of hypersensitivity immune response such as astma). The mice were sacrificed one day after the last challenge and blood, bronchoalveolar fluid (BAL) and spleen were collected for analysis.

T-cell Activation Assay: Spleens were teased into single cell suspension and washed three times in medium. Cells were counted and 3 x 10⁵ cells were added to each well of a 96 well flat-bottomed culture plate and stimulated by 0, 5, 25 and 125 µg/mL OVA. The cells were cultured for 5 days at 37 °C and 5% CO2. Cell culture supernatants for cytokine analysis were harvested on day 5.

### Results:

In this experiment, it was examined whether non-sensitized mice treated by a polypeptide of the invention (e.g. Profilin Phl p 12) could be prevented from developing a hypersensitivity immune response induced by a non-profilin allergen (OVA) or at least could suppress a hypersensitivity immune response (e.g. asthma) induced by a non-profilin allergen (OVA). Furthermore, and in addition to Example 1, it was tested if co-exposure to OVA and Bet v 2 (profilin having about 78% amino acid sequence identity to Phl p 12) could replace the co-exposure to OVA and Phl p 12, so as to investigate whether profilin of another profilin-containing plant material can reactivate Treg cells that are specific to Phl p 12 to produce non-specific regulatory cytokines and thus suppress an allergic response caused by OVA.

As shown in Figures 8 and 9, treatment with Phl p 12 is able to down-regulate the production of the Th2-associated cytokines IL-5 and IL-13, respectively, compared to buffer treated mice. However, this down-regulation was only observed when the mice were co-exposed to Bet v 2 together with OVA at the time of sensitization and intranasal challenge. Thus, data indicates that a hypersensitivity immune response caused by allergens of another profilin-containing plant material than (Phl p containing Phl p 12) could be prevented upon exposing the mice to both the triggering allergen (OVA) and Bet v 2 in mice treated with Phl p 12.

### Example 5

Screening for co-extracted polypeptides and non-profilin allergens in profilin-containing plant materials.

### Materials for Extraction:

• Raw grass pollen of the species *Phleum pratense* (frozen just after harvest), defatted pollen of the species *Phleum pratense, Betula verrucosa, Ambrosia artemisiifolia, Corylus avellana, Artemisia vulgaris, Cryptomeria japonica, Humulus japonicas,* cat dander, mite bodies and feces of *Dermatophagoides pteronyssinus* and apples.
• Glass bottles (100 ml) for extraction
• PD-10 columns with PE bed support combined with 10 ml syringe with silicone tubing.
• Filter units (Millex 5 µm + Millex 0.8µm)
• Test tubes
• Ice bath, pipettes, sample rotator, measuring cylinder, stop watch
• PBS buffer, pH 7.2 containing the following salts:

| *Salt* | | *M_{w} (g*/*mol)* | *Conc. g*/*L* | *Conc. mM* |
|---|---|---|---|---|
| Sodium chloride | NaCl | 58.44 | 8.0 | 137 |
| Potassium chloride | KCl | 74.55 | 0.2 | 2.7 |
| Na-phosphate | Na₂HPO₄, 2H₂O | 175.98 | 1.44 | 8.2 |
| K-phosphate | KH₂PO₄ | 136.09 | 0.2 | 1.5 |
| Phosphate conc.: | 8.2 + 1.5 = 9.7 mM phosphate | | | |
| NaCl: | µ = ½ * (137 * 12 + 137 * 12) = 137 mM | | | |
| KCl: | µ = ½ * (2.7 * 12 + 2.7 * 12) = 2.7 mM | | | |
| Na2HPO4: | µ = ½ * ((8.2 * 2 * 12) + (8.2 * 22)) = 24.6 mM | | | |
| KH2PO4: | µ = ½ * ((1.5 * 12) + (1.5 * 12)) = 1.5 mM | | | |
| Total ionic strength: | µ = 165.8 mM ≈ 0.17 M | | | |

### Extraction Procedure (at room temperature, 21-24 °C):

5.0 g of pollen/mite bodies/mite feces/cat dander are weighed into a glass bottle and 50 ml of PBS is added and the bottle is immediately rotated, first 5 minutes by hand and thereafter rotated in a sample rotator during the entire extraction.

5 ml of slurry is taken out after 20 sec, transferred to a column with a bed filter and dragged through the filter with a syringe. The syringe is immediately transferred to a filter unit and the extract is pushed through the combined filters into a labelled test tube. The tube is stored in an ice bath until the sample is pipetted in aliquots for further analysis and frozen. 5.2 ml of the suspension is taken out at time points 40 sec, 60 sec, 2 min, 5 min, 10 min and 20 min.Apples were squeezed and the resulting juice was analyzed immediately by CIE/RIE.

### Determination of Co-Eluted/Co-Extracted Non-Profilin Allergen and Profilin

Samples are analysed for the content of non-profilin allergens and profilin by use of one more of the methods CIE (Crossed Immune Electrophoresis), RIE (Rocket immune electrophoresis), SDS Page (sodium dodecyl sulfate polyacrylamide gel electrophoresis), ELISA (Enzyme-linked immunosorbent assay) and/or MS (Mass Spectrometry).

### CIE (Crossed Intermediate Immune Electrophoresis)

### Materials for CIE/RIE

- Electrophoresis apparatus (2 buffer vessels, 2 electrodes, cooled surface, chamber)
- Glass plates (std. sizes, e.g. 5x5, 5x7, 10x7 cm)
- Buffer for electrode vessels and agarose gel (ionic strength 0.02, pH 8.6) 5.5-diethylbarbituric acid (Veronal) 112.1 g; Tris (Sigma 7-9) 221.5 g; Calcium lactate (purum) 2.7 g; Purified water ad 5 L. Dilute 1+4 before use.
- Agarose plates:_1% (w/v) Litex Agarose type HSA (Mᵣ = -0.13) is made using the "Buffer for agarose gel", the agarose is heated under stirring and boiled for two minutes and kept fluid in a water bath at 56°C. Then apply gel to plates (about 5 ml).
- Staining solution: Coomassie Brilliant Blue R-250 5 g, Ethanol 96% 450 ml, purified water 500 ml, Glacial acidic acid 50 ml. Dissolve overnight by stirring and filtrate.
- Destaining solution: Ethanol 96% 450 ml, purified water 500 ml, Glacial acidic acid 50 ml.
- Polyclonal Rabbit antibodies; Rabbits are immunized repeatedly with the polypeptide, e.g. aqueous extract containing the profilin of the profilin-containing plant material such as native Phl p 12, at days 0, 2, 4, 6, 10, 14 and 18 and blood is collected at days 8, 12, 16, 20 and 24. The IgG fraction is obtained after precipitation with ammonium sulfate (≈ 1.75 M (NH₄)₂SO₄) and washed in 1.75M (NH₄)₂SO₄. Purified further by dialysis against purified water and acetate buffer pH 5.0. Stored at +5°C in 0.9% NaCl with 0.09% NaN₃ and protease inhibitor (Trasylol) for preservation.

Procedure: Wells are punched out in the agarose gel plates and the extraction sample is added. Electrophoresis is performed for 25-30 minutes in an apparatus operated at 15°C and at 10 V/cm. Gel is removed and agarose gel mixed thoroughly with polyclonal antibodies is poured onto the cathodic side and the anodic side. Perform electrophoresis overnight at voltage 2 V/cm. Then wells are washed with distilled water and with 0.1 M NaCl for 15-30 minutes and plates are then dried in a stream of hot air. Plates are stained for app. 5 minutes in Commassie Brilliant Blue staining solution, then washed in distilled water for a few seconds, destained and finally dried in hot air.

Polypeptides recognized by the antibodies are seen as blue sharp bands on the CIE plate. To further identify the polypeptides, bands can be cut out from the CIE plate and analysed by Mass Spectrometry and/or other immunochemical methods and/or amino acid analysis.

### RIE

Agarose gel plates containing the relevant polyclonal antibodies are made on glass plates. Wells are punched out about 1.5 cm from the lower edge of the plate, the plates are placed in electrophoresis apparatus, extraction samples are added and electrophoresis continued overnight at voltage 2 V/cm. Plates are washed/pressed and stained with Coomassie Brilliant Blue staining solution as described above for CIE.

### SDS PAGE

Materials: Sample Buffer (4X) NuPage®; Invitrogen NP0007, NuPAGE® 10% Bis-Tris SDS-PAGE; Invitrogen NP0302, NuPAGE® MES SDS Running Buffer(20X); Invitrogen NP0002-02,Marker: SeeBlue® Plus2Prestained Standard; Invitrogen LC5925

SDS PAGE (reduced or non-reduced conditions) was performed in accordance with NuPAGE® Technical Guide General information and protocols for using the NuPAGE® electrophoresis system Rev. date: 29 October 2010 Manual part no. IM-1001.

Alternatively, sodium dodecyl sulfate polyacrylamide gel electrophoresis may be used in accordance to methods known in the art.

Following electrophoresis, the gel may be stained (most commonly with Coomassie Brilliant Blue R-250 or silver stain), allowing visualization of the separated proteins, or processed further (e.g. Western blot). After staining, different proteins will appear as distinct bands within the gel. A molecular weight size marker of known molecular weight may be added in a separate lane in the gel, in order to calibrate the gel and determine the approximate molecular mass of unknown proteins by comparing the distance travelled relative to the marker.

### ELISA

Materials: Monoclonal antibodies (mAb) are obtained by immunizing rabbits repeatedly with a single polypeptide with no content of isomers at days 0, 2, 4, 6, 10, 14 and 18 and blood is collected at days 8, 12, 16, 20 and 24. The IgG fraction is obtained after precipitation with ammonium sulfate (≈ 1.75 M (NH₄)₂SO₄) and washed in 1.75M (NH₄)₂SO₄. Purified further by dialysis against purified water and acetate buffer pH 5.0. Stored at +5°C in 0.9% NaCl with 0.09% NaN₃ and protease inhibitor (Trasylol) for preservation.

Determination of single antigens: Phl p 12: Plates were coated with 2.5 ug/ml of monoclonal antibodies (mAb) of Pho d 2 (profilin of date palm) overnight at 4°C. After saturation for 30 minutes at room temperature, purified nPhl p 12 (reference standard) and extraction samples were diluted in order to obtain the full sigmoid curves (serial dilution 1/3, 8 points) and were incubated for 1 hour at room temperature. After washing, bound Phl p 12 was detected by incubation with rabbit polyclonal anti-Pho d 2 at dilutionl/4,000 for 1 hour at room temperature. After washes, peroxidase-conjugated Goat polyclonal anti Rabbit IgG antibody was added for 1 hour at RT and the signal was then revealed by adding OPD together with H₂O₂. After 30 minutes the reaction was stopped with 2N HCl and the plates were read at 490 nm.

Determination of single antigens: Phl p 5: Plates were coated with mAbs against Phl p 5 and Lol p 5 overnight at 4°C, saturated and dried. On the day of the quantification, the plates were rehydrated and extraction samples were diluted accordingly in order to obtain the full sigmoid curves (serial dilution 1/3) and were incubated in the plates for 1h at RT. After washes, bound Phl p 5 was detected after incubation for 1 hour at RT with a Rabbit polyclonal directed against a mix of grass pollen at 1/10,000. After washes, peroxidase-conjugated Goat polyclonal anti Rabbit IgG antibody was added for 1 hour at RT and the signal was then revealed by adding OPD together with H₂O₂. After 30 minutes, the reaction was stopped with 2N HCl and the plates were read at 490 nm.

Determination of single antigens: Phl p 1: Plates were coated with 2.5ug/ml mAb Phl p 1, 5ug/ml mAb Phl p 1 and 5ug/ml mAb Phl p 1 overnight at 4°C. After saturation for 30 minutes at RT, extraction samples were diluted accordingly in order to obtain the full sigmoid curves (Serial dilution 1/3) and were incubated in the plates for 1 hour at RT. After washes, bound Phl p 1 was detected by incubation with a Rabbit polyclonal anti-*P*. *pratense* antibody at 1/10,000 for 1 hour at RT. After washes, peroxidase-conjugated Goat polyclonal anti Rabbit Ig antibody was added for 1 hour at RT and the signal was then revealed by adding OPD together with H₂O₂. After 30 minutes, the reaction was stopped with 2N HCl and the plates were read at 490 nm.

### Mass Spectrometry

Buffers/solutions for reduction, alkylation and digestion of the sample:

| | |
|---|---|
| Sample buffer: | 8 M urea in 0.4 M NH₄HCO₃ |
| DTT (45 mM): | Make it fresh from the frozen stock 1.0 M: 45 µl 1 M DTT + 955 µl water |
| Iodoacetamide (IAA): | Make fresh solution, Iodoacetamid 100 mM, |
| Trypsin: | Sigma T6567, Dissolve one vial in 20 µl of 1 mM HCl. This results in a solution containing 1 µg/µl trypsin. After reconstitution in 1 mM HCl frozen aliquots can be stored for up to 4 weeks. |

Enzymatic digestion with trypsin in solution for mass spectrometry: Dilute the dried sample in 5 µl of water, add 15 µl of sample buffer (8 M Urea in 0.4 M NH₄HCO₃), add 5 µl 45 mM DTT, incubate at 56°C for 15 min, cool it to room temperature, add 5 µl of 100 mM Iodoacetamide, incubate in the dark in room temperature for 15 min, add 90 µl of water to lower the concentration of urea <1-2 M, add 1 µg trypsin, incubate at 37°C over night.

Chromatography: Reverse phase chromatography (Ultimate 3000 HPLC, Dionex) was performed using a C18 pre- and analytical column. The eluting peptides were sprayed directly into an ESI-QTOF mass spectrometer (MaXis, Bruker). After washing the trap column with 0.05% v/v formic acid for 5 min with a flow rate of 30 µl/min, the peptides were eluted with an acetonitrile gradient at a flow rate of 2 µl/min using solvent A: 0.05% v/v formic acid and solvent B: 80% v/v acetonitrile/0.04% v/v formic acid and the gradient: 4-50% B in 200 minutes; 50-80% B in10 minutes; 100% B in 10 min, 4% B in 5 min.

Spectra were acquired in the mass range 50-2599 m/z and a spectra rate of 1.5 Hz. The instrument was tuned and calibrated using ESI-L Low concentration Tunning Mix from Agilent Technology.

Data acquisition and instrument control were carried out with Bruker Compass HyStar 3.2. Data processing was performed using DataAnalysis 4.0 (Bruker). Protein identification was performed using the program Biotools3.2 (Bruker) and two different data bases, i.e. Swiss prot and NCBInr. The MS/MS data sets for the tryptic digest were analysed using the following parameters; peptide tolerance 10 ppm and fragment tolerance 0.05 Da.

Procedure: The extraction samples were all evaporated (50 µl) and re-suspended in 5 µl of water. The sample is then reduced, alkylated and digested with trypsin. Resulting peptides are separated and identified by reversed phase chromatography followed by MS/MS.

### Results:

Results concerning extraction of raw pollen of *Phleum pratense* are shown in:
- Figure 10 (CIE of 20 sec, 60 sec and 2 min extracts using polyclonal antibodies raised against extract of pollen of *Phleum pratense*)

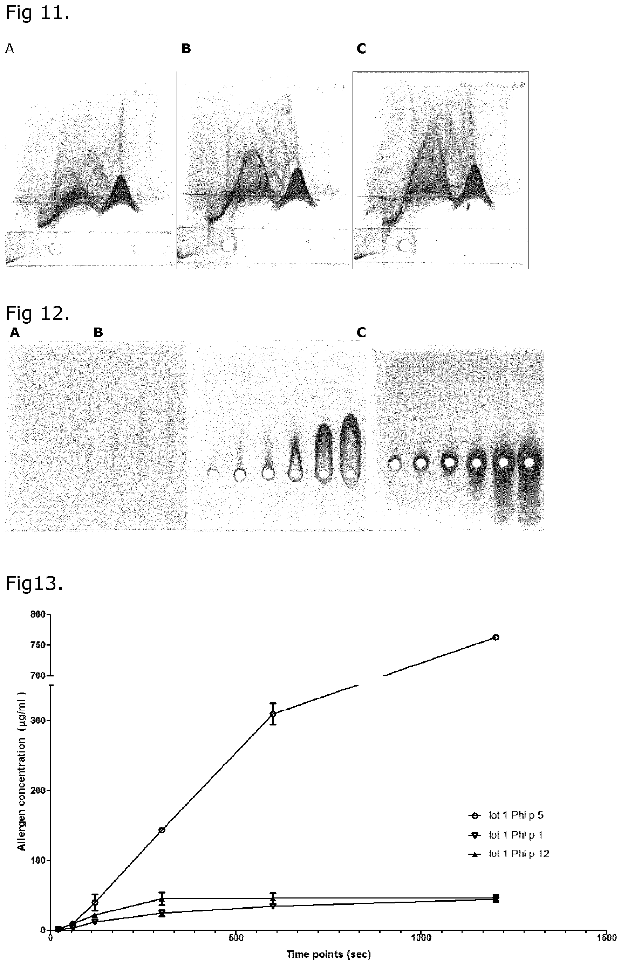

- Figure 11 (CIE of 5, 10 and 20 min extracts using polyclonal antibodies raised against extract of pollen of *Phleum pratense*)
- Figure 12 (RIE of 20 sec, 60 sec, 2 min, 5 min, 10 min and 20 min extracts using polyclonal antibodies against purified Phl p 12 (A), Phl p 5 (B) and Phl p 1 (C))
- Figure 13 (Amounts of Phl p 12, Phl p 1 and Phl p 5 released after 20 sec, 60 sec, 2 min, 5 min, 10 min and 20 min extraction time and analysed by use of ELISA).

All results indicate that the profilin Phl p 12 is indeed co-extracted with the major allergens of *Phleum pratense* (Phl p 1 and Phl p 5) within the same time window and even within the first 10 minutes of extraction, thus indicating that the profilin will be presented to the target organ within the same time window as the major allergens causing the hypersensitivity immune response upon the individuals exposure to a profilin-containing plant material. Extracts were also analysed by Mass Spectrometry analysis and it could be demonstrated that Phl p 12 was co-extracted with a number of proteins, among others, the allergens Phl p 1, 5 and 6.

Results concerning extraction of defatted pollen of *Phleum pratense* are shown in Figures:

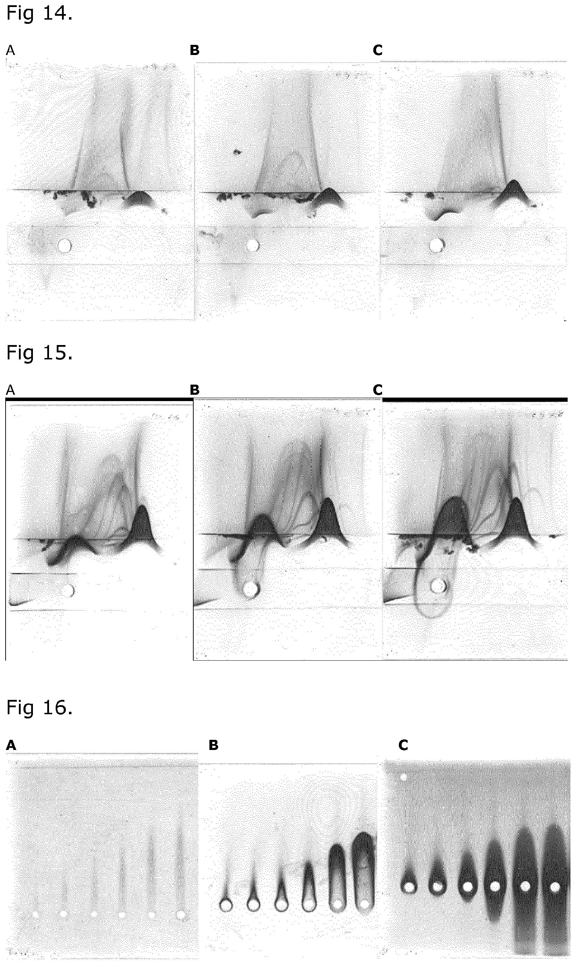

- Figure 14 (CIE of 20 sec, 60 sec and 2 min extracts using polyclonal antibodies raised against extract of pollen of *Phleum pratense*)
- Figure 15 (CIE of 5, 10 and 20 min extracts using polyclonal antibodies raised against extract of pollen of *Phleum pratense*)
- Figure 16 (RIE of 20 sec, 60 sec, 2 min, 5 min, 10 min and 20 min extracts using polyclonal antibodies against purified Phl p 12 (A), Phl p 5 (B) and Phl p 1 (C))

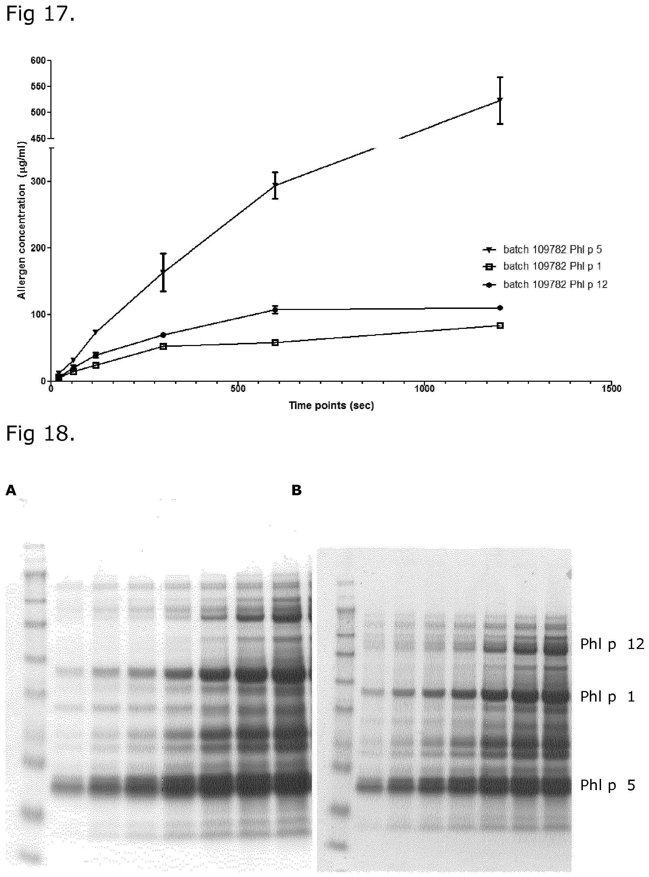

- Figure 17 (Amounts of Phl p 12, Phl p 1 and Phl p 5 released after 20 sec, 60 sec, 2 min, 5 min, 10 min and 20 min extraction time, analysed by ELISA by use of monoclonal antibodies raised in rabbit against purified Phl p 12, Phl p 1 and Phl p 5)
- Figure 18 (SDS page of extracts of A) raw and B) defatted pollen, extracts obtained at 20 sec, 40 sec, 60 sec, 2 min, 5 min, 10 min and 20 min extraction are shown from left to right)

All results indicate that defatted pollen may be used instead of raw pollen in screening for co-extracted profilin and non-profilin allergens of a profilin-containing plant material.

Results concerning extraction of defatted pollen of *Betula Verrucosa* are shown in Figures:

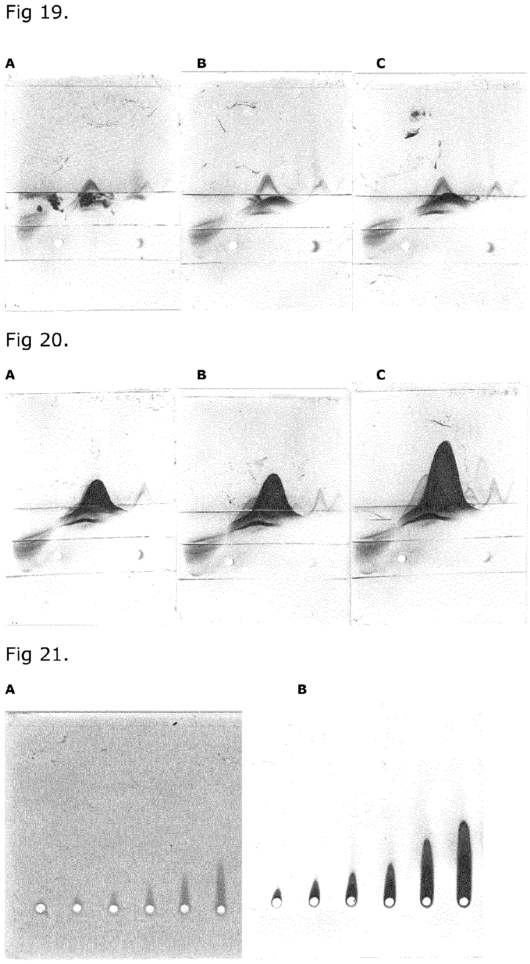

- Figure 19 (CIE of 20 sec, 60 sec and 2 min extracts using polyclonal antibodies raised against extract of pollen of *Betula Verrucosa*);
- Figure 20 (CIE of 5, 10 and 20 min extracts using polyclonal antibodies raised against extract of pollen of *Betula Verrucosa*)
- Figure 21 (RIE of 20 sec, 60 sec, 2 min, 5 min, 10 min and 20 min extracts using polyclonal antibodies against purified Bet v 2 (profilin) (A) and Bet v 1 (major allergen)(B)

Results indicate that Bet v 2 (profilin) is co-extracted with the major allergen (Bet v 1) from birch pollen as early as 1 minute after start of extraction.

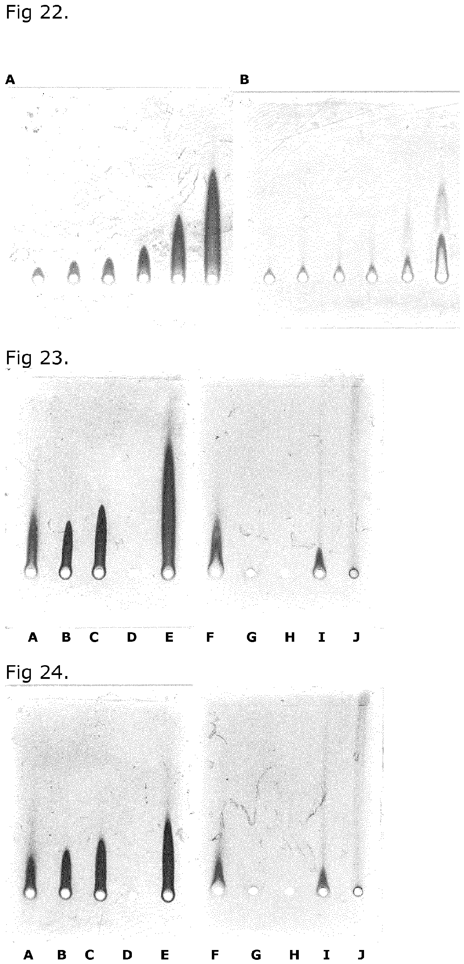

Results concerning extraction of defatted pollen of *Ambrosia Artemisiifolia* are shown in Figure 22 showing RIE of 20 sec, 60 sec, 2 min, 5 min, 10 min and 20 min extracts using polyclonal antibodies against purified Amb a 8 (profilin) (A) and Amb a 1 (major allergen) (B). Results indicate that Amb a 8 is co-extracted with the major allergen Amb a 1 as early as 1 minute after start of extraction.

The ability of various plant profilins to recognize antibodies raised against the profilins Bet v 2 and Phl p 12 was studied by RIE using extracts of pollen of *Ambrosia artemisiifolia, Betula verrucosa, Corylus avellana, Phleum pratense, Artemisia vulgaris, Cryptomeria japonica, Humulus japonicas*, cat dander, mite bodies and freshly made apple juice and polyclonal rabbit antibodies raised against purified Phl p 12 and Bet v 2, respectively.

Results (Figures 23 and 24) indicate that plant profilins of other species than *Phleum pratense* and *Betula Verrucosa* are able to bind to IgG antibodies specific to Phl p 12 and Bet v 2, thus indicating that the treatment of an individual with Phl p 12 or Bet v 2 may suppress a hypersensitivity immune response caused by a non-profilin allergen of a profilin-containing material that does not contain Phl p 12 or Bet v 1, but at least profilin of the genus *Ambrosia, Corylus*, *Cryptomeria*, *Humulus* and *Malus.*

### Example 6

### Recombinant Production of a Polypeptide according to the Invention

In the present example, a variant polypeptide was produced having the variations C13A and C115A compared to SEQ ID NO: 1. Full sequence is given in SEQ ID NO: 44.

Materials: Plasmid: p-RSETB (commercially available from Invitrogen - Life Technologies); *E*. *coli* strain: BL21(DE3), available from NOVAGEN; Lysis buffer: 10 mM phosphate buffer, 30 mM NaCl, 10 mM EDTA, 1 mM benzamidin, 10 µg/ml SBTI (Soy Bean Trypsin Inhibitor), 3 mM PMSF (phenylmethanesulfonylfluoride), and 0.02w/w% sodium azide; LB medium: 1% tryptone, 0.5% yeast extract, 0.5% NaCl, pH 7.0; Column equilibration buffer: PBS pH 7.2; PLP-Sepharose column: The column was prepared following the manufacturer's instructions of the CNBr-activated-Sepharose 4B (GE-Healthcare, ref. 17-0430-01), and using poly-L-Proline (Sigma, ref.P2129) as the ligand; Superdex75 column: prepacked column from GE-Healthcare; Dialysis membrane: 3,500 Da MWCO membrane.

Procedure: A cDNA of SEQ ID NO: 46 encoding a polypeptide of SEQ ID NO: 44 was synthesized and the codon was optimized based on the *E*. *coli* preference (selection of nucleotides corresponding to tRNAs most regularly and abundantly present in *E*. *coli*); the cDNA was introduced into the NdeI/PstI-cut plasmidp-RSETB, which is IPTG (isopropyl-beta-D-thiogalactopyranoside)-inducible; the plasmid was transformed into the BL21 (DE3) *E*. *coli* strain; the freshly transformed cells were grown at 37°C in 1 L of LB medium containing ampicillin in culture flasks shaken at 250 rpm, until an OD600 of 0.6 was reached, and then induced at 18°C; the cell culture was induced by adding IPTG to a concentration of 0.3 mM and left for about 16 hours at 18°C (alternatively, the induction can be performed at higher temperatures, e.g. 37°C, inducing during 3-4 hours, starting at an OD600 of 0.4); the *E.coli* culture was maintained in an ice water bath for at least 5 minutes before harvesting by centrifugation (e.g. 12,000 rpm at 4°C for 20 min); the thus pelleted, induced cells were resuspended in 50 ml lysis buffer and then broken by sonication; the soluble fraction containing the soluble cytoplasmic product was collected by centrifugation (12,000 rpm at 4°C for 20 min) and the supernatant was clarified and then dialysed against PBS (pH 7.2).

The recombinant polypeptide was purified in two chromatographic steps by: affinity chromatography on PLP-Sepharose column equilibrated with column equilibration buffer; elution with 6M urea that must be eliminated immediately by dialysis through dialysis membrane to PBS (the column equilibration buffer), and then size exclusion chromatography on Superdex75 column equilibrated with column equilibration buffer in order to obtain the monomeric recombinant polypeptide product which consists of about 99% protein

### (weight/weight of dry matter)

The identity of the recombinant polypeptide was determined by SDS-PAGE (reduced and non reduced; silver and Coomassie staining), N-terminal sequencing and amino acid analysis as well as MS analyisis (to analyze for impurities and confirm its identity by fingerprinting).

The recombinant polypeptide produced was confirmed to have the amino acid sequence of SEQ ID NO: 44. The N-terminal Methionine appeared to be eliminated in about 95% of the polypeptide product produced. Only about 5% of the polypeptide produced seemed still to carry the N-terminal Methionine.

The polypeptide was then subjected to circular dichroism (CD) analysis in order to explore the folding of the recombinant protein. The method will be able to verify that the majority of the protein is correctly folded. The CD spectra (260 to 184 nm) were determined on an OLIS DSM-10 spectropolariometer using square 0.1 cm light path length cuvettes at 15°C. All spectra were obtained in 10 mM sodium phosphate buffer pH 7.2 and each sample was scanned five times. The raw data spectra was corrected for buffer absorption and all spectra were normalized to CD_signal_λ260 = 0. The raw data (mDeg) was converted to Δε (ellipticity) values using the following equations (mDeg = 32980*ΔA; Δε = ΔA/(c*l)), where "c" is the molar concentrations of the sample and "l" is the light path length in cm.

In addition to the 15°C experiments, a heating experiment was also performed with the sample scanned at different temperatures with 5°C-intervals from 15 to 90°C. This analysis was performed in order to investigate the stability/unfolding of the protein

The polypeptide having the substitutions C13A and C115A surprisingly showed a higher folding stability than the parent peptide (50% of the polypeptide of SEQ ID NO: 44 had unfolded at about 61°C whereas 50% of the polypeptide of SEQ ID NO: 1 had unfolded at about 51°C).

### Example 7

In the present example, a variant polypeptide was produced having the variations C13S and C115S compared to SEQ ID NO: 1. Full sequence is given in SEQ ID NO: 45.

This polypeptide variant was produced as described in example A, except the cDNA used had SEQ ID NO: 47 encoding the polypeptide of SEQ ID NO: 45.

The recombinant polypeptide produced was confirmed to have the amino acid sequence of SEQ ID NO: 45. The N-terminal Methionine appeared to be eliminated in about 95% of the polypeptide product produced. Only about 5% of the polypeptide produced seemed still to carry the N-terminal Methionine.

The polypeptide having the substitutions C13S and C115S showed about the same folding stability as the parent peptide (50% of the polypeptide of SEQ ID NO: 45 had unfolded at about 51°C).

The melting point of nPhl 12, rPhlp 12 and the two variants V1 (C13S and C115S) and V2 (C13A and C115A), respectively, were also determined. It was found that the melting point of the alanine variant (V2) was significantly higher (61°C) than the melting point of nPhl 12 (54°C), rPhlp 12 (58°C) and the variant V1 (51°C), respectively.

### Example 8

Bystander suppression of a hypersensitivity immune response (asthma) caused by Bet v extract (pollen extract of *Betula Verrucosa*) in naïve mice treated by Phl p 12.

Methods and materials are the same as in Example 1, except that Phl p extract is replaced with Bet v extract. Further, the depletion of Phl p 12 is not included in this study.

The results (Figures 25 and 26) indicate that a hypersensitivity immune response caused by birch allergens is suppressed in naïve mice treated sublingually with the profilin Phl p 12.

### Example 9

Bystander suppression of a hypersensitivity immune response (asthma) caused by Bet v extract (pollen extract of *Betula Verrucosa*) in sensitized mice treated by Phl p 12.

Methods and materials are the same as in Examples 3 and 8. Mice are sensitized to Bet v extract (birch allergens) deleped for the profilin Bet v 2 instead of OVA and subsequently treated with Phl p 12.

The results (Figures 27 and 28) indicate that a hypersensitivity immune response caused by birch allergens can be suppressed in sensitized mice treated sublingually with the profilin Phl p 12.

### Example 10

Bystander suppression of a hypersensitivity immune response caused by Phl p extract (pollen extract of *Phleum Pratense*) in naivemice treated by recombinantly produced Ole e 2 (profilin of olive tree pollen)

Methods and materials are similar to Example 2. Mice are treated sublingually with Ole e 2 and mice are subsequently sensitized to grass pollen allergens (Phl p 12 extract) The results (Figures 29 and 30) show that a hypersensitivity immune response caused by grass allergens can be suppressed in naive mice treated sublingually with the profilin Ole e 2.

### Example 11

Bystander suppression of a hypersensitivity immune response caused by OVA (co-exposure with either Phl p 12 or Bet v 2) in naïve mice treated by Phl p 12.

Methods and materials are similar to Example 1. Mice are treated sublingually with Phl p 12 and mice are subsequently sensitized to OVA, OVA under co-exposure to Phl p 12 or to OVA under co-exposure to Bet v 2.

The result (Figure 31) indicates that a hypersensitivity immune response caused by OVA is suppressed in naive mice treated sublingually with the profilin Phl p 12, when the mice are also co-exposed to Phl p 12 or Bet v 2. The result also shows that tolerance to one profilin (Phl p 12) is sufficient to suppress an immune response to an unrelated antigen (OVA), even where co-exposure is provided by another profilin molecule than Phl p 12 (here Bet v 2).

### Example 12

Investigation of T-cell recognition of various profilins in Phl p 12-specific T-cell lines established from PBMC's of grass allergic individuals.

PBMCs were isolated from individuals allergic to allergens of grass pollen of *Phleum pratense* (individuals having IgE reactivity to pollen extracts of *Phleum pratense*)*.*

Phl p 12 reactive T-cell lines were then established by incubation for two weeks followed by re-stimulation of freshly isolated PBMCs with recombinant wild type (wt) Phl p 12 (SEQ ID NO: 1). Established T-cell lines were subsequently stimulated with (a) recombinant wt Phlp 12, (b) natural purified Phl p 12, (c) recombinant Ole e 2 (a profilin homologue of Phl p 12) found in pollen of olive trees (SEQ ID NO: 16), and (d) growth medium as a control. The magnitude of T-cell activation in response to stimulation with the grass and olive profilins was measured by H³ incorporation. Figures A and B represent T-cell lines established from PBMCs from two different individuals, respectively.

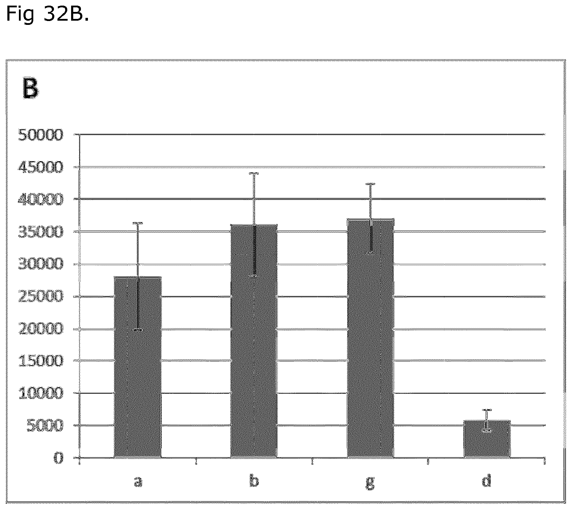

The data shows (Figure 32) that the human T-cells from grass allergic individuals, as expected, become activated following exposure to the cognate antigen (Phl p 12) but also become activated following exposure to the homologous protein Ole e 2. This clearly demonstrates the possibility of extensive immunological cross-reactivity at the T-cell level between homologous but non-identical proteins from phylogenetically distant species. Furthermore, this finding forms the essential basis for the selection of proteins and protein variants with immune-modulatory potential towards various and distant species, which in turn allows for the development of novel therapies for atopic diseases, including, allergy and asthma, with much broader, less species-restricted indications.

## Claims

1. A polypeptide for use in the treatment of a hypersensitivity immune response in an individual, wherein the hypersensitivity immune response is caused by a non-profilin allergen of a profilin-containing plant material, wherein said polypeptide comprises an amino acid sequence having at least 60% identity with SEQ ID NO: 1.

2. The polypeptide for the use according to claim 1, wherein the polypeptide comprises an amino acid sequence having at least 75% identity with SEQ ID NO: 1.

3. The polypeptide for the use according to claim 1, wherein a part of the polypeptide has an amino acid sequence having at least 80% identity with SEQ ID NO: 43.

4. The polypeptide for the use according to any one of the preceding claims, wherein the polypeptide is a profilin having an amino acid sequence selected from the group consisting of SEQ ID NOs: 1-42 or a variant of said profilin, wherein the variant comprises an amino acid selected from A (alanine), G (glycine) or S (serine) in position 13 and/or in position 115 of SEQ ID NO: 1.

5. The polypeptide for the use according to claim 4, wherein the amino acid sequences of SEQ ID NOs: 1-42 is without methionine in the N-terminal end.

6. The polypeptide for the use according to any one of the preceding claims, wherein the profilin-containing plant material is of a plant order selected from the group consisting of *Poales, Asterales, Fagales and Lamiales.*

7. The polypeptide for the use according to any one of the preceding claims, wherein the profilin-containing plant material is pollen.

8. The polypeptide for the use according to claim 7, wherein the profilin-containing plant material is pollen of a plant genus selected from the group consisting of *Anthoxanthum, Cynodon, Dactylis, Festuca, Holcus, Hordeum, Lolium, Oryza, Paspalum, Phalaris, Phleum, Poa, Secale, Sorghum, Triticum* and *Zea.*

9. The polypeptide for the use according to claim 7, wherein the profilin-containing plant material is pollen of a plant genus selected from the group consisting of *Ambrosia* and *Artemisia.*

10. The polypeptide for the use according to claim 7, wherein the profilin-containing plant material is pollen of a plant genus selected from the group consisting of *Betula, Alnus, Carpinus, Corylus, Ostrya, Fagus, Quercus, Castanea, Juglans and Carya.*

11. The polypeptide for the use according to claim 7, wherein the profilin-containing plant material is pollen of the plant genus *Olea.*

12. The polypeptide for the use according to any one of the preceding claims, wherein the individual is not sensitized to the profilin of the profilin-containing plant material before administering the first dose of the polypeptide.

13. The polypeptide for the use according to any one of the preceding claims, wherein the polypeptide is not co-administered with a non-profilin allergen.

14. The polypeptide for the use according to any one of the preceding claims, wherein the polypeptide is administered by the sublingual route.

15. The polypeptide for the use according to any one of the preceding claims, wherein the hypersensitivity immune response is associated with an allergic immune response.

16. A polypeptide having an amino acid sequence of SEQ ID NOs: 1, 2, 3, 4, 5, 6, 7, 8, 9 or 10, wherein 1 or 2 cysteine residue(s) is/are substituted by an amino acid selected from A (alanine), G (glycine) and/or S (serine).

17. The polypeptide according to claim 16, wherein the polypeptide has an amino acid sequence of SEQ ID NOs: 44 or 45.

18. The polypeptide according to any one of claims 16 and 17, wherein the amino acid sequence does not contain methionine in position 1.

19. An isolated nucleic acid encoding the protein of SEQ ID NOs: 44 or 45.

20. The isolated nucleic acid according to claim 19 having SEQ ID NOs: 46 or 47.

## Patentansprüche

1. Polypeptid zur Verwendung bei der Behandlung einer Überempfindlichkeitsimmunreaktion, wobei die Überempfindlichkeitsimmunreaktion durch ein Nicht-Profilin-Allergen eines Profilin enthaltenden Pflanzenmaterials verursacht wird, wobei das Polypeptid eine Aminosäuresequenz umfasst, die mindestens 60 % Identität mit SEQ ID NO: 1 aufweist.

2. Polypeptid zur Verwendung nach Anspruch 1, wobei das Polypeptid eine Aminosäuresequenz umfasst, die mindestens 75 % Identität mit SEQ ID NO: 1 aufweist.

3. Polypeptid zur Verwendung nach Anspruch 1, wobei ein Teil des Polypeptids eine Aminosäuresequenz aufweist, die mindestens 80 % Identität mit SEQ ID NO: 43 aufweist.

4. Polypeptid zur Verwendung nach irgendeinem der vorhergehenden Ansprüche, wobei das Polypeptid ein Profilin, das eine Aminosäuresequenz aufweist ausgewählt aus der Gruppe bestehend aus SEQ ID NO: 1-42 oder eine Variante des Profilins ist, wobei die Variante eine Aminosäure umfasst ausgewählt von A (Alanin), G (Glycin) oder S (Serin) an Position 13 und/oder Position 115 von SEQ ID NO: 1 .

5. Polypeptid zur Verwendung nach Anspruch 4, wobei die Aminosäuresequenzen von SEQ ID NO: 1 - 42 ohne Methionin im N-terminalen Ende sind.

6. Polypeptid zur Verwendung nach irgendeinem der vorhergehenden Ansprüche, wobei das Profilin enthaltende Pflanzenmaterial zu einer Pflanzenordnung gehört ausgewählt aus der Gruppe bestehend aus *Proales, Asterales, Fagales* und *Lamiales.*

7. Polypeptid zur Verwendung nach irgendeinem der vorhergehenden Ansprüche, wobei das Profilin enthaltende Pflanzenmaterial Pollen ist.

8. Polypeptid zur Verwendung nach Anspruch 7, wobei das Profilin enthaltende Pflanzenmaterial Pollen einer Pflanzengattung ist ausgewählt aus der Gruppe bestehend aus *Anthoxanthum, Cynodon, Dactylis, Festuca, Holcus, Hordeum, Lolium, Oryza, Paspalum, Phalaris, Phleum, Poa, Secale, Sorghum, Triticum und Zea.*

9. Polypeptid zur Verwendung nach Anspruch 7, wobei das Profilin enthaltende Pflanzenmaterial Pollen einer Pflanzengattung ist ausgewählt aus der Gruppe bestehend aus *Ambrosia* und *Artemisia.*

10. Polypeptid zur Verwendung nach Anspruch 7, wobei das Profilin enthaltende Pflanzenmaterial Pollen einer Pflanzengattung ist ausgewählt aus der Gruppe bestehend aus *Betula, Alnus, Carpinus, Corylus, Ostrya, Fagus, Quercus, Castanea* und *Carya.*

11. Polypeptid zur Verwendung nach Anspruch 7, wobei das Profilin enthaltende Pflanzenmaterial Pollen der Pflanzengattung *Olea* ist.

12. Polypeptid zur Verwendung nach irgendeinem der vorhergehenden Ansprüche, wobei das Individuum bezüglich des Profilins des Profilin enthaltenden Pflanzenmaterials vor Verabreichen der ersten Dosis des Polypeptids nicht sensibilisiert ist.

13. Polypeptid zur Verwendung nach irgendeinem der vorhergehenden Ansprüche, wobei das Polypeptid nicht gleichzeitig mit einem Nicht-Profilin-Allergen verabreicht wird.

14. Polypeptid zur Verwendung nach irgendeinem der vorhergehenden Ansprüche, wobei das Polypeptid auf sublingualem Weg verabreicht wird.

15. Polypeptid zur Verwendung nach irgendeinem der vorhergehenden Ansprüche, wobei die Überempfindlichkeitsimmunreaktion mit einer allergischen Immunreaktion verbunden ist.

16. Polypeptid, das eine Aminosäuresequenz von SEQ ID NO: 1, 2, 3, 4, 5, 6, 7, 8, 9 oder 10 aufweist, wobei 1 oder 2 Cysteinrest(e) durch eine Aminosäure substituiert ist/sind, ausgewählt unter A (Alanin), G (Glycin) und/oder S (Serin).

17. Polypeptid nach Anspruch 16, wobei das Polypeptid eine Aminosäuresequenz von SEQ ID NO: 44 oder 45 aufweist.

18. Polypeptid nach irgendeinem der Ansprüche 16 und 17, wobei die Aminosäuresequenz kein Methionin in Position 1 enthält.

19. Isolierte Nukleinsäure, die für das Protein von SEQ ID NO: 44 oder 45 codiert.

20. Isolierte Nukleinsäure nach Anspruch 19, die SEQ ID NO: 46 oder 47 aufweist.

## Revendications

1. Polypeptide pour son utilisation dans le traitement d'une réponse immunitaire d'hypersensibilité chez un individu, dans lequel la réponse immunitaire d'hypersensibilité est provoquée par un allergène différent de la profiline d'une substance végétale contenant de la profiline, dans lequel ledit polypeptide comprend une séquence d'acides aminés ayant une identité d'au moins 60 % avec SEQ ID NO : 1.

2. Polypeptide pour son utilisation selon la revendication 1, dans lequel le polypeptide comprend une séquence d'acides aminés ayant une identité d'au moins 75 % avec SEQ ID NO : 1.

3. Polypeptide pour son utilisation selon la revendication 1, dans lequel une partie du polypeptide a une séquence d'acides aminés ayant une identité d'au moins 80 % avec SEQ ID NO : 43.

4. Polypeptide pour son utilisation selon l'une quelconque des revendications précédentes, dans lequel le polypeptide est une profiline ayant une séquence d'acides aminés sélectionnée dans le groupe constitué de SEQ ID NO : 1 à 42 ou un variant de ladite profiline, dans lequel le variant comprend un acide aminé sélectionné parmi A (alanine), G (glycine) ou S (sérine) en position 13 et/ou en position 115 de SEQ ID NO : 1.

5. Polypeptide pour son utilisation selon la revendication 4, dans lequel les séquences d'acides aminés de SEQ ID NO : 1 à 42 n'ont pas de méthionine dans l'extrémité N-terminale.

6. Polypeptide pour son utilisation selon l'une quelconque des revendications précédentes, dans lequel la substance végétale contenant de la profiline est d'un ordre de plantes sélectionné dans le groupe constitué des *Poales, Asterales, Fagales* et *Lamiales.*

7. Polypeptide pour son utilisation selon l'une quelconque des revendications précédentes, dans lequel la substance végétale contenant de la profiline est le pollen.

8. Polypeptide pour son utilisation selon la revendication 7, dans lequel la substance végétale contenant de la profiline est le pollen d'un genre de plantes sélectionné dans le groupe constitué des genres *Anthoxanthum, Cynodon, Dactylis, Festuca, Holcus, Hordeum, Lolium, Oryza, Paspalum, Phalaris, Phleum, Poa, Secale, Sorghum, Triticum* et Zea.

9. Polypeptide pour son utilisation selon la revendication 7, dans lequel la substance végétale contenant de la profiline est le pollen d'un genre de plantes sélectionné dans le groupe constitué des genres *Ambrosia* et *Artemisia.*

10. Polypeptide pour son utilisation selon la revendication 7, dans lequel la substance végétale contenant de la profiline est le pollen d'un genre de plantes sélectionné dans le groupe constitué des genres *Betula, Alnus, Carpinus, Corylus, Ostrya, Fagus, Quercus, Castanea, Juglans* et *Carya.*

11. Polypeptide pour son utilisation selon la revendication 7, dans lequel la substance végétale contenant de la profiline est le pollen du genre de plantes *Olea.*

12. Polypeptide pour son utilisation selon l'une quelconque des revendications précédentes, dans lequel l'individu n'est pas sensibilisé à la profiline de la substance végétale contenant de la profiline avant l'administration de la première dose du polypeptide.

13. Polypeptide pour son utilisation selon l'une quelconque des revendications précédentes, dans lequel le polypeptide n'est pas co-administré avec un allergène différent de la profiline.

14. Polypeptide pour son utilisation selon l'une quelconque des revendications précédentes, dans lequel le polypeptide est administré par la voie sublinguale.

15. Polypeptide pour son utilisation selon l'une quelconque des revendications précédentes, dans lequel la réponse immunitaire d'hypersensibilité est associée à une réponse immunitaire allergique.

16. Polypeptide ayant une séquence d'acides aminés de SEQ ID NO : 1, 2, 3, 4, 5, 6, 7, 8, 9 ou 10, dans lequel 1 ou 2 résidu(s) cystéine est/sont substitué(s) par un acide aminé sélectionné parmi A (alanine), G (glycine) et/ou S (sérine).

17. Polypeptide selon la revendication 16, dans lequel le polypeptide a une séquence d'acides aminés de SEQ ID NO : 44 ou 45.

18. Polypeptide selon l'une quelconque des revendications 16 et 17, dans lequel la séquence d'acides aminés ne contient pas de méthionine en position 1.

19. Acide nucléique isolé codant pour la protéine de SEQ ID NO : 44 ou 45.

20. Acide nucléique isolé selon la revendication 19 ayant SEQ ID NO : 46 ou 47.
